(19) **Europäisches Patentamt**
**European Patent Office**
**Office européen des brevets**

(11) **EP 3 515 470 B1**

(12) **EUROPEAN PATENT SPECIFICATION**

(45) Date of publication and mention
of the grant of the patent:
**14.06.2023 Bulletin 2023/24**

(21) Application number: **17853553.0**

(22) Date of filing: **21.09.2017**

(51) International Patent Classification (IPC):
**G01N 33/68** (2006.01)  **C12Q 1/6883** (2018.01)

(52) Cooperative Patent Classification (CPC):
**A61P 3/00; A61K 31/69; A61P 35/00; A61P 37/00;**
**C12Q 1/6883; G01N 33/6872;** C12Q 2600/156;
G01N 2800/20

(86) International application number:
**PCT/SG2017/050478**

(87) International publication number:
**WO 2018/056907 (29.03.2018 Gazette 2018/13)**

(54) **METHODS FOR PREDICTING SKIN INFLAMMATION AND DETERMINING CANCER SUSCEPTIBILITY**

VERFAHREN ZUR VORHERSAGE EINER HAUTENTZÜNDUNG UND ZUR BESTIMMUNG DER KREBSANFÄLLIGKEIT

PROCÉDÉS POUR PRÉDIRE UNE INFLAMMATION CUTANÉE ET DÉTERMINER UNE SUSCEPTIBILITÉ AU CANCER

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **21.09.2016 SG 10201607886X**

(43) Date of publication of application:
**31.07.2019 Bulletin 2019/31**

(73) Proprietors:
• **Agency for Science, Technology and Research**
**Singapore 138632 (SG)**
• **The Laboratory of Cytogenetics, Molecular Genetics**
**and Human Reproduction**
**Sousse 4000 (TN)**

(72) Inventors:
• **REVERSADE, Bruno**
**Singapore 138648 (SG)**
• **ZHONG, Lei**
**Singapore 138648 (SG)**
• **MAMAI, Ons**
**Singapore 138648 (SG)**

(74) Representative: **Regimbeau**
**20, rue de Chazelles**
**75847 Paris Cedex 17 (FR)**

(56) References cited:
**WO-A1-00/71135**      **WO-A1-2015/084875**
**WO-A2-2007/106790**   **WO-A2-2007/106790**
**WO-A2-2007/117419**   **WO-A2-2011/109459**
**WO-A2-2012/027652**   **US-A1- 2004 248 775**
**US-A1- 2009 104 200**

• **LEVANDOWSKI C. B. ET AL.: "NLRP1 haplotypes associated with vitiligo and autoimmunity increase interleukin-1 processing via the NLRP1 inflammasome", PROC. NATL. ACAD. SCI., vol. 110, no. 8, 19 February 2013 (2013-02-19), pages 2952-2956, XP055501045,**
• **ZHONG F.L. ET AL.: "Germline NLRP1 mutations cause skin inflammatory and cancer susceptibility syndromes via inflammasome activation", CELL, vol. 167, no. 1, 22 September 2016 (2016-09-22), pages 187-202, XP029748863,**
• **JIN, Y. ET AL.: 'NALP1 in Vitiligo-Associated Multiple Autoimmune Disease' N ENGL J MED vol. 356, no. 12, 22 March 2007, pages 1216 - 1225, XP002544855**

- LEVANDOWSKI, C.B. ET AL.: 'NLRP1 haplotypes associated with vitiligo and autoimmunity increase interleukin-1 beta processing via the NLRP1 inflammasome' PROC NATL ACAD SCI USA. vol. 110, no. 8, 04 February 2013, pages 2952 - 2956, XP055501045
- OKAMOTO, M. ET AL.: 'Constitutively Active Inflammasome in Human Melanoma Cells Mediating Autoinflammation via Caspase-1 Processing and Secretion of Interleukin-1beta' J BIOL CHEM vol. 285, no. 9, 28 December 2009, pages 6477 - 6488, XP055210236
- BOBE, P. ET AL.: 'Arsenic trioxide: a promising novel therapeutic agent for lymphoproliferative and autoimmune syndromes in MRL/lpr mice' BLOOD vol. 108, no. 13, 22 August 2006, pages 3967 - 3975, XP055452567
- KOHNO, S. ET AL.: 'Inhibition of skin sclerosis by 15deoxy delta12, 14- prostaglandin J2 and retrovirally transfected prostaglandin D synthase in a mouse model of bleomycin-induced scleroderma' BIOMED PHARMACOTHER vol. 60, no. 1, 25 October 2005, pages 18 - 25, XP029609681
- ELLIS, L.Z. ET AL.: 'Green tea polyphenol epigallocatechin-3-gallate suppresses melanoma growth by inhibiting inflammasome and IL -1 beta secretion' BIOCHEM BIOPHYS RES COMMUN, [Online] vol. 414, no. 3, 28 October 2011, pages 551 - 556, XP028328933 Retrieved from the Internet: <URL:https://www.ncbi.nlm.nih.gov/pubmed/21982776> [retrieved on 2017-12-28]
- WILLIAMS, T.M. ET AL.: 'The NLRP1 Inflammasome Attenuates Colitis and Colitis-Associated Tumorigenesis' J IMMUNOL vol. 194, 27 February 2015, pages 3369 - 3380, XP055501054
- MASTERS, S.L. ET AL.: 'NLRP1 Inflammasome Activation Induces Pyroptosis of Hematopoietic Progenitor Cells' IMMUNITY vol. 37, no. 6, 06 December 2012, pages 1009 - 1023, XP055327994
- MURPHY, A.J. ET AL.: 'IL -18 Production from the NLRP1 Inflammasome Prevents Obesity and Metabolic Syndrome' CELL METAB vol. 23, no. 1, 22 October 2015, pages 155 - 164, XP029385086
- NARRA, K. ET AL.: 'Phase II trial of single agent Val-boroPro (talabostat) inhibiting fibroblast activation protein in patients with metastatic colorectal cancer' CANCER BIOLOGY & THERAPY vol. 6, no. 11, 01 November 2007, pages 1691 - 1699, XP055324844
- MAIER, N.K. ET AL.: 'Arsenic trioxides and other arsenical compounds inhibit the NLRP1, NLRP3, and NAIP5/NLRC4 inflammasomes' J IMMUNOL. vol. 192, no. 2, 03 January 2014, pages 763 - 770, XP055501088
- MAIER, N.K.: 'The Cyclopentenone Prostaglandin 15d-PGJ2 Inhibits the NLRP1 and NLRP3 Inflammasomes' J IMMUNOL. vol. 194, no. 6, 13 February 2015, pages 2776 - 2785, XP055501094
- FINGER, J.N. ET AL.: 'Autolytic Proteolysis within the Function to Find Domain (FIIND) Is Required for NLRP1 Inflammasome Activity' J BIOL CHEM vol. 287, no. 30, 04 June 2012, pages 25030 - 25037, XP055501204
- ZHONG, F.L. ET AL.: 'Germline NLRP1 Mutations Cause Skin Inflammatory and Cancer Susceptibility Syndromes via Inflammasome Activation' CELL vol. 167, no. 1, 22 September 2016, pages 187 - 202, XP029748863

**Description**

**FIELD OF THE INVENTION**

The present invention relates generally to the field of molecular biology. In particular, the present invention relates to the use of biomarkers for the detection and diagnosis of cancer.

**BACKGROUND OF THE INVENTION**

The timely activation and resolution of the innate immune response is essential for host defence, tissue homeostasis, and tumour immunosurveillance. One key innate immune pathway relies on the inflammasome complexes, which consist of an array of, for example, ligand-sensing nucleotide-binding domain, leucine-rich repeat containing (NLR) proteins, the adaptor protein ASC, and caspase-1. NLR proteins patrol the cytosol and initiate inflammasome assembly, pyroptotic cell death, and pro-inflammatory cytokine release upon ligand binding. While the inflammasome complexes are essential for pathogen clearance under physiological conditions, their aberrant activation can be detrimental. This is evident, for example, in a group of auto-inflammatory disorders caused by germline-activating mutations in inflammasome sensor proteins, including cryopyrin-associated periodic syndromes (CAPSs), familial Mediterranean fever syndromes (FMFs), and certain forms of macrophage activation syndromes (MASs). These patients experience diagnostic symptoms such as periodic fever and sterile inflammation as a result of spontaneous macrophage activation and release of pyrogenic cytokines.

Inflammasome complexes function as key innate immune effectors that trigger inflammation in response to pathogen- and danger-associated signals. It has been shown that, for example, the inflammasome sensor NLRP1 is the most prominent inflammasome sensor in human skin, and all pathogenic NLRP1 mutations are predispose to inflammasome activation and predispose to cancer and. Mechanistically, NLRP1 mutations have been shown to lead to increased self-oligomerisation by disrupting the PYD and LRR domains, which are essential in maintaining NLRP1 as an inactive monomer. Primary keratinocytes from patients were shown to experience spontaneous inflammasome activation and paracrine IL-1 signalling, which is sufficient to cause symptoms, such as skin inflammation and epidermal hyperplasia. WO 2007/106790 A2 relates to the treatment, amelioration or prevention of vitiligo characterized by at least one gene mutation in NLRP1. The genetic variations in the NALP1 (=NLRP1) gene are used to diagnose or predict the risk of vitiligo. WO 2015/084875 A1 discloses methods of preventing or treating diseases such as vitiligo, psoriasis and Addison's disease using aromatic-cationic peptides, wherein the subject is suffering from or is at increased risk for a disease or condition characterized by a mutation in NLRP1.

Although the function of NLR proteins in systemic inflammation is well established, less is known about their roles in organ-specific immune response and tissue homeostasis, particularly in epithelial tissues such as, for example, the skin. Apart from forming a structural barrier, human skin actively interacts with the immune system in guarding against invading pathogens and tissue damage. On the other hand, chronic unresolved skin inflammation can result in a variety of dermatological diseases and promote the development of both benign and malignant epithelial skin lesions.

Thus, there is a need for methods and agents for treating or preventing skin disorders and determining cancer susceptibility.

**SUMMARY OF THE INVENTION**

In one aspect, the present invention refers to a method of determining the likelihood or predisposition of a subject in developing MSPC (multiple self-healing palmoplantar carcinoma) or FKLC (familial keratosis lichenoides chronica), comprising detecting NLR family, pyrin domain containing protein 1 (NLRP1) mutation in a sample obtained from the subject, wherein the NLRP1 mutation is located at PYD (pyrin domain) and/or LRR (leucine-rich repeats) domain.

In another aspect, the present invention refers to a method of determining whether a skin inflammation in a subject is MSPC (multiple self-healing palmoplantar carcinoma) or FKLC (familial keratosis lichenoides chronica), comprising detecting NLRP1 mutation in a sample obtained from the subject, wherein the NLRP1 mutation is located at PYD (pyrin domain) and/or LRR (leucine-rich repeats) domain.

The invention is defined by the appended claims.

**BRIEF DESCRIPTION OF THE DRAWINGS**

The invention will be better understood with reference to the detailed description when considered in conjunction with the non-limiting examples and the accompanying drawings, in which:

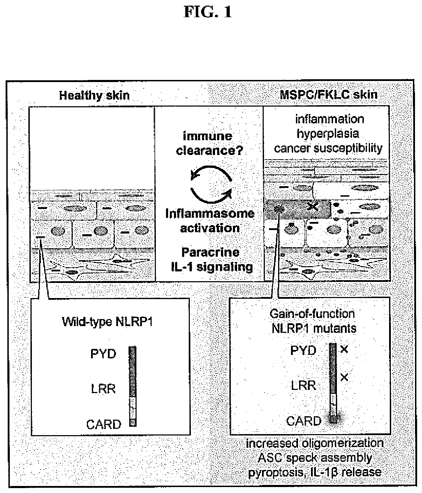

    **Fig. 1** shows a graphical representation of how an inflammasome sensor leads to increased susceptibility to a skin cancer and the involved regulatory auto-inhibition in the inflammasome.

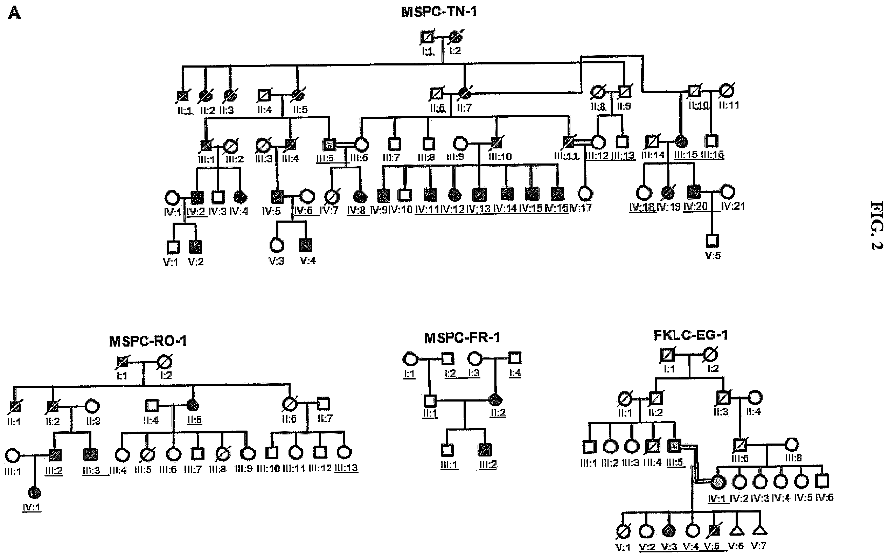

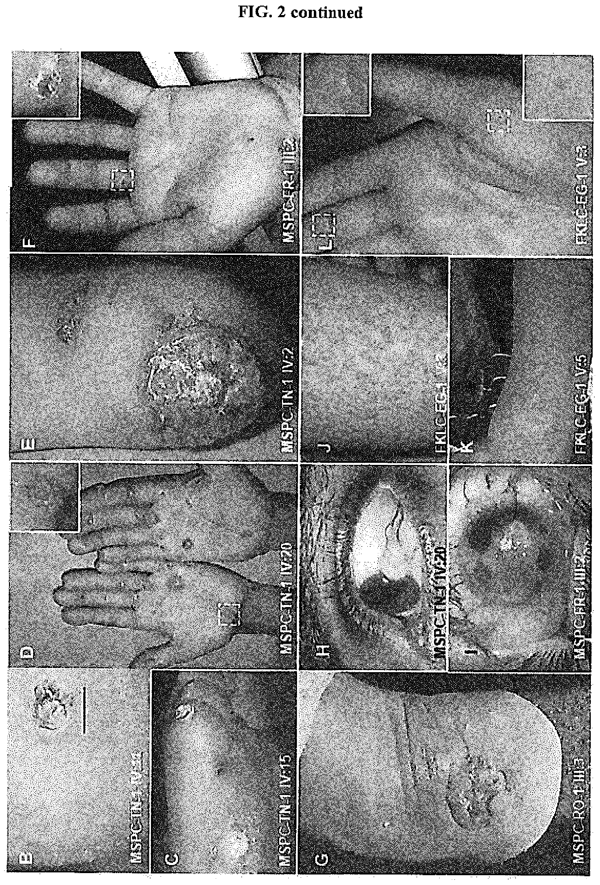

**Fig. 2** shows a genogram showing the pedigrees and Clinical features of MSPC and FKLC families. **(A)** Pedigrees of MSPC (MSPC-TN-1, MSPC-RO-1, and MSPC-FR-1) and FKLC families (FKLC-EG-1). The NLRP1 genotypes of all underlined individuals were verified by Sanger sequencing. **(B-L)** Major clinical symptoms of MSPC and FKLC. **(B)** Indurated ulcer with overlying crust and surrounding hyperkeratosis on the heel. **(C)** Firm, scaly nodule on the outer part of the right foot and subungual lesion on the little toe. **(D)** Multiple discrete, self-healing, warty keratoacanthomas on both palms and fingers (one lesion detailed in inset). **(E)** "Ulcerated squamo-proliferative" plaque on the heel with a further smaller lesion on the sole. **(F)** Multiple discrete warty papules and nodules on the left palm and fingers (one lesion detailed in inset), consistent with keratoacanthoma. **(G)** Squamo-proliferative keratoacanthoma on the heel with surface ulceration. **(H)** Left eye showing conjunctival and corneal scarring with hyperemia. **(I)** Obliterative corneal scarring and surrounding conjunctival inflammation. **(J)** Multiple discrete and semi-confluent lichenoid papules on the upper arm. **(K)** Multiple discrete and semi-confluent lichenoid papules on the forearm. **(L)** Hyperkeratotic papules on the thenar eminence and palmar surface of fingers (shown in more detail in insets).

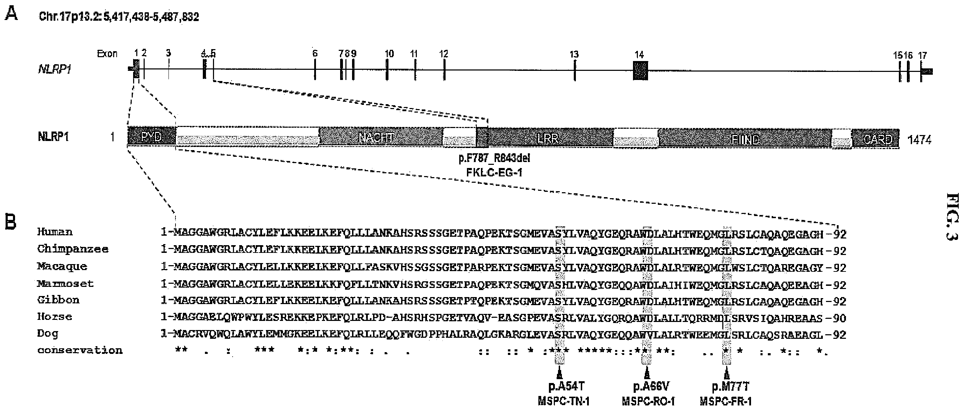

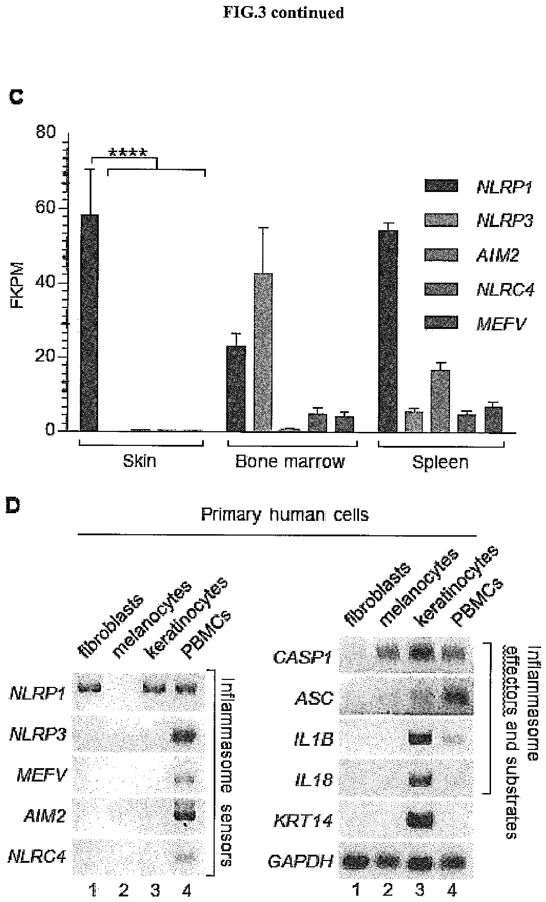

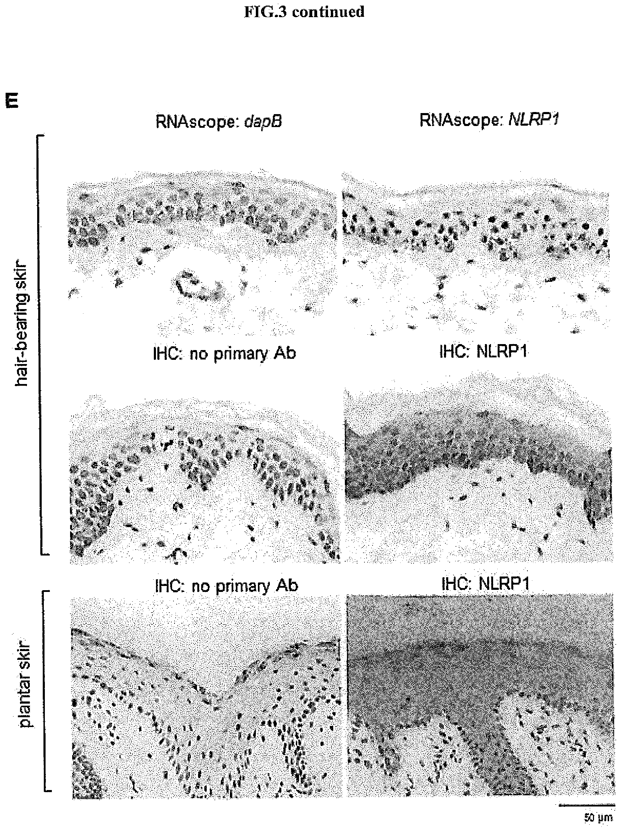

**Fig. 3** presents data showing that MSPC and FKLC Are Caused by Germline Mutations in NLRP1, the Predominant Inflammasome Sensor in Human Skin. **(A)** NLRP1 genomic locus (top) and NLRP1 protein domain structure (bottom). All MSPC cases harbor germline heterozygous missense mutations in NLRP1 exon 1, which encodes the pyrin domain (PYD). FKLC-EG-1 V:3 and V:5 are homozygous for an in-frame deletion removing exon 5 (p. F787_R843del). **(B)** Conservation of NLRP1 PYD in mammals. Missense mutations in MSPC are highlighted in gray. **(C)** Transcript levels of NLRP1 and other inflammasome sensors in primary human skin, bone marrow, and spleen tissues. **(D)** RT-PCR expression levels of known inflammasome sensors, effectors, and substrates in primary skin fibroblasts, melanocytes, keratinocytes, and peripheral blood mononuclear cells (PBMCs). All cells were isolated from healthy donors. KRT14, keratinocyte-specific marker. **(E)** Detection of NLRP1 mRNA and NLRP1 protein in primary FFPE skin sections. dapB, negative control for RNAscope in situ staining.

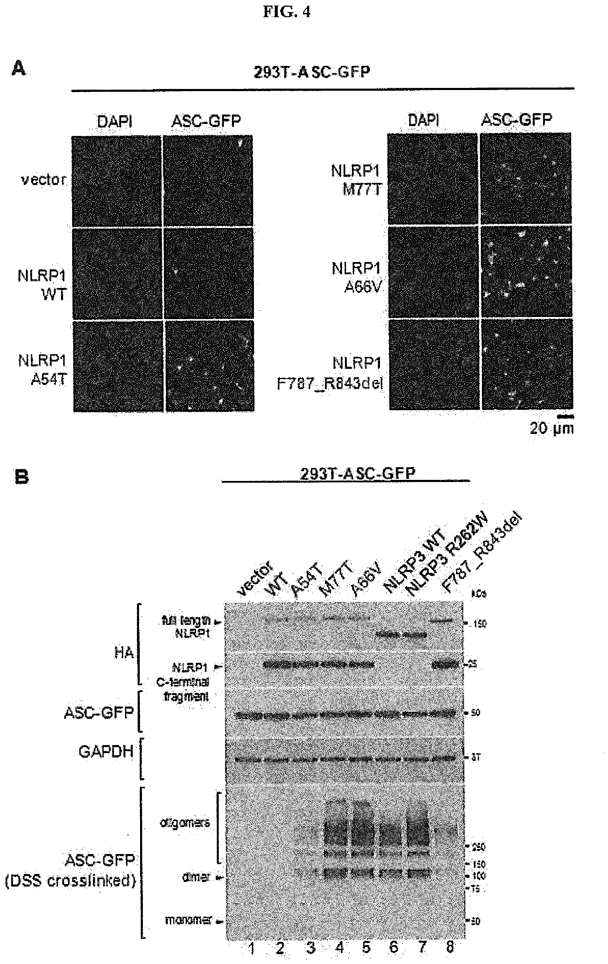

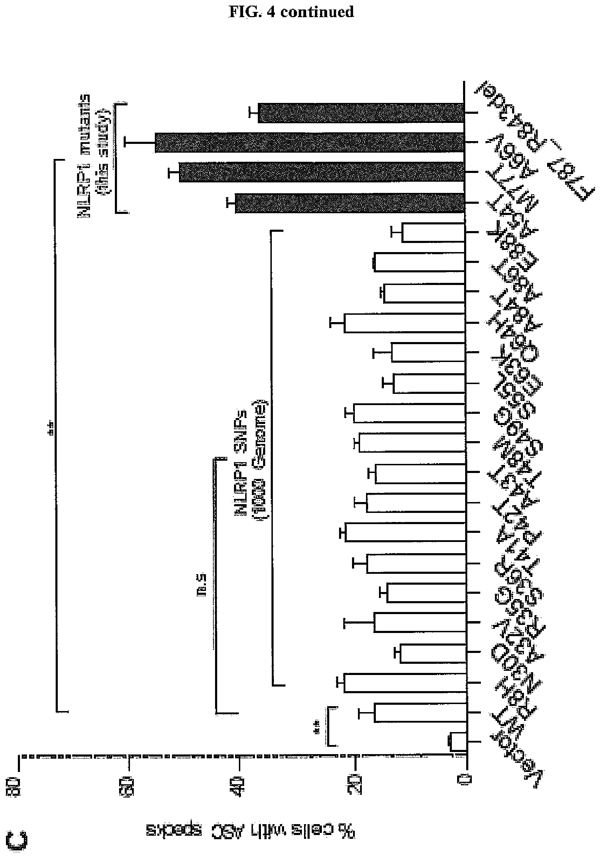

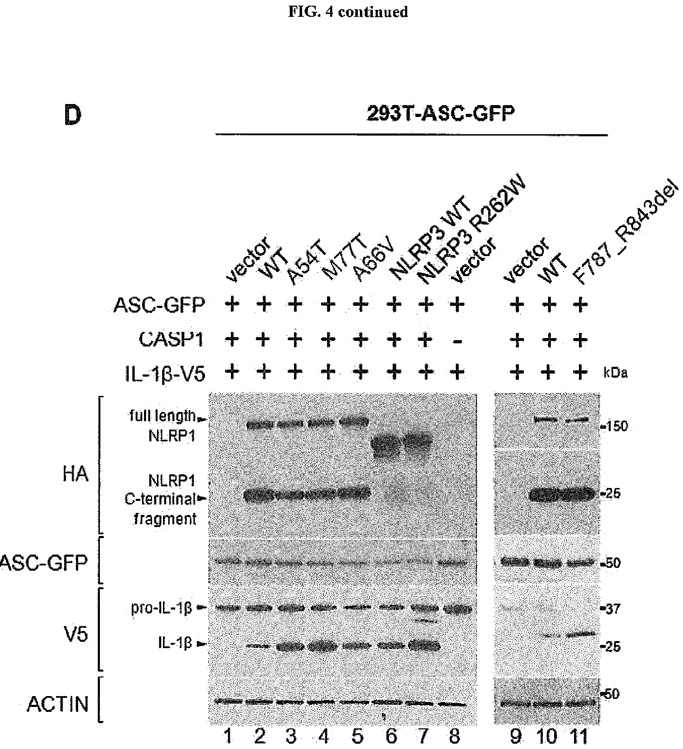

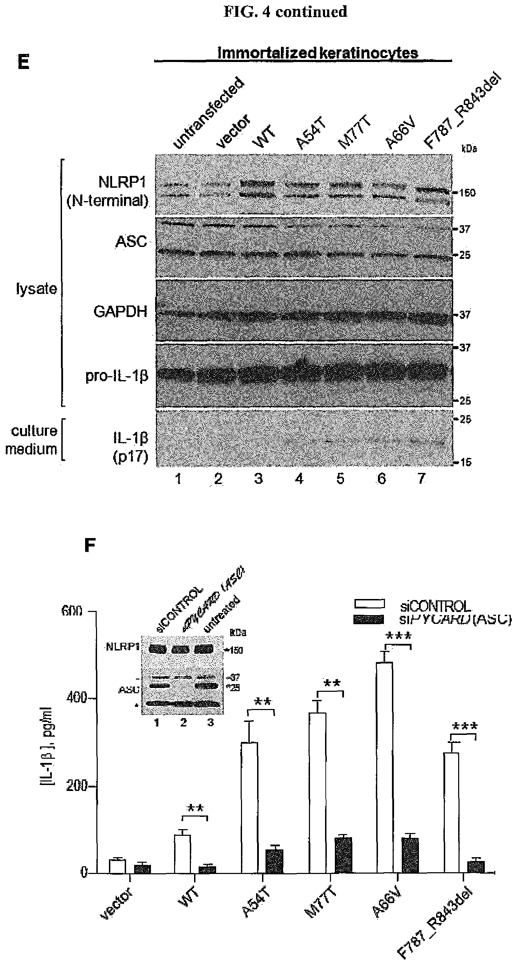

**Fig. 4** presents data showing that all MSPC and FKLC mutations cause increased NLRP1 inflammasome activation. **(A)** Increased ASC-GFP speck formation by NLRP1 mutants. 293T-ASC-GFP cells were transfected with empty vector, wild-type NLRP1 and NLRP1 mutants and fixed 16 hour post-transfection. **(B)** NLRP1 mutants increase ASC-GFP oligomerization. ASC-GFP cells were transfected as in **(A).** Cell pellets were subjected to crosslinking with 1 mM DSS for 15 min at 37° C. **(C)** Quantitative comparison of ASC-GFP speck formation in 293T-ASC-GFP cells expressing disease-causing mutants or common NLRP1 SNPs in the PYD domain. **(D)** Increased pro-caspase-1 activation and pro-IL-1b cleavage by NLRP1 mutants in inflammasome-reconstituted 293T cells. 293T-ASC-GFP cells were transfected with wild-type NLRP1 and NLRP1 mutants with pro-caspase-1 and V5-tagged pro-IL-1b. Cells were harvested 16 hour post-transfection. **(E)** Increased caspase-1 activation, pro-IL-1b cleavage, and IL-lb secretion by NLRP1 mutants in immortalized human keratinocytes. N/TERT-immortalized keratinocytes were transfected with the indicated NLRP1 variants and harvested 16 hour post-transfection. Levels of endogenous and overexpressed NLRP1 were measured with an antibody against NLRP1-PYD. Conditioned media was concentrated 103 before SDS-PAGE. **(F)** Inflammasome activation by NLRP1 mutants requires ASC, N/TERT-immortalized keratinocytes were transfected with control siRNAs and siRNAs against PYCARD (ASC). Cells were reseeded 3 days post-transfection and re-transfected with wild-type NLRP1 or NLRP1 mutants. The level of secreted IL-lb was measured by ELISA 16 hour post cDNA transfection. Asterisks indicate non-specific bands.

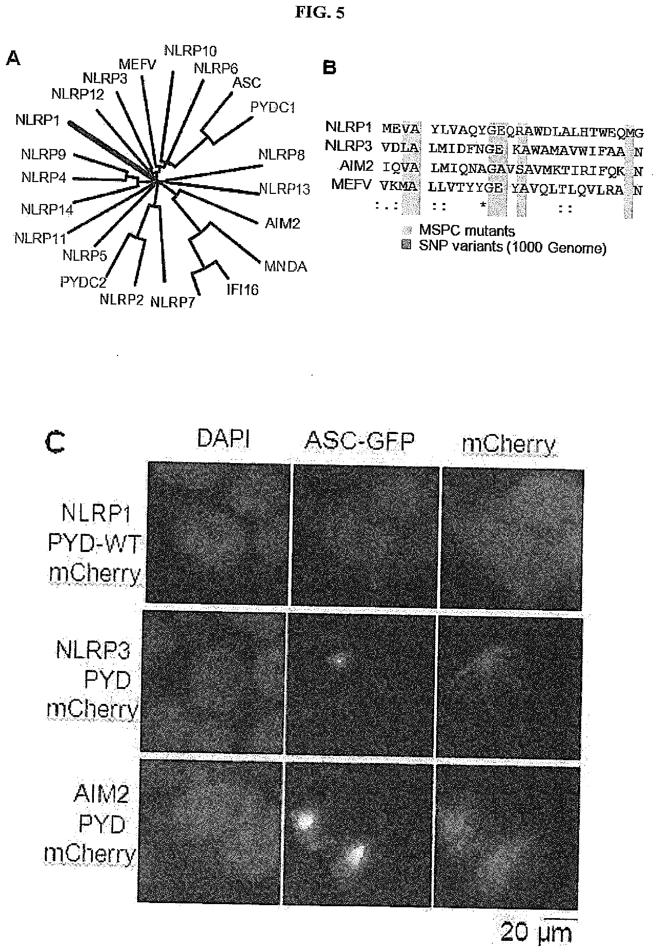

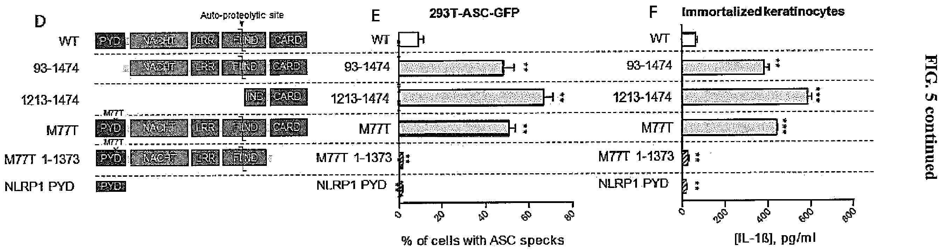

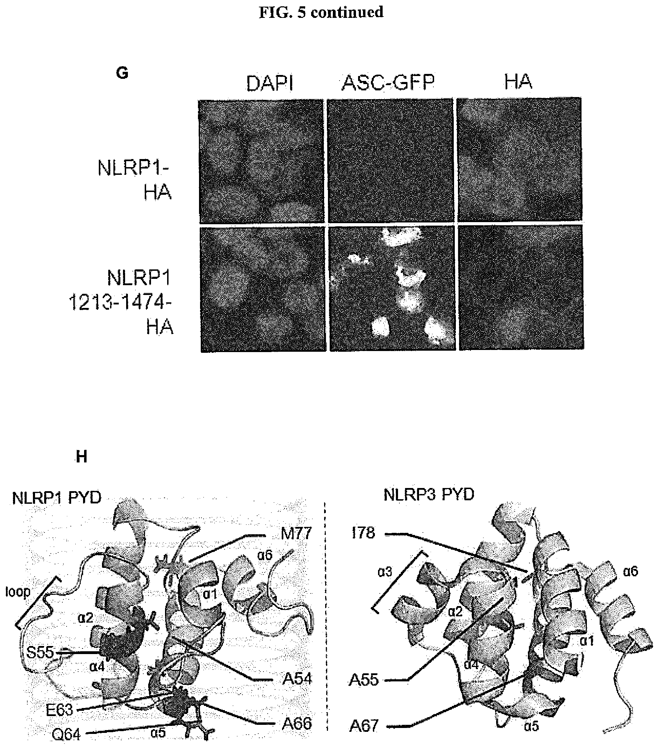

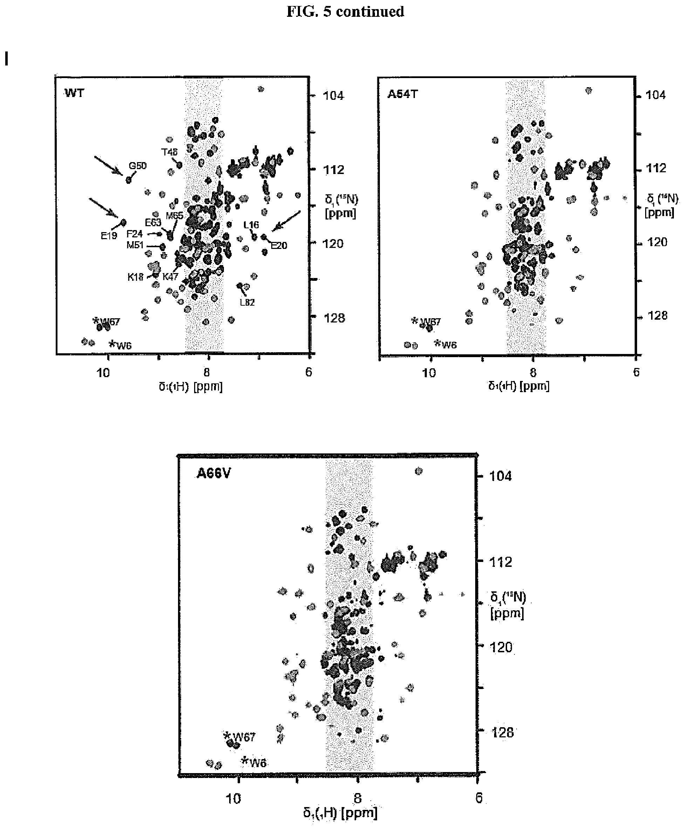

**Fig. 5** presents data showing that the NLRP1 pyrin domain is auto-inhibitory and MSPC mutations disrupt its folding. **(A)** Sequence comparison of all annotated human pyrin domains (PYDs). NLRP1 PYD is shown in gray. PYDs from other known inflammasome components are shown in blue. **(B)** Conservation of residues mutated in MSPC among known inflammasome sensors. Amino acid residues mutated in MSPC are shown in red. Adjacent common polymorphic residues are shown in blue. **(C)** NLRP1 PYD does not nucleate ASC-GFP specks. 293T-ASC-GFP cells were transfected with mCherry-tagged NLRP1, NLRP3, and AIM PYD constructs, and fixed 24 hours post-transfection. **(D)** Truncation mutants of NLRP1. NLRP1 undergoes auto-proteolytic cleavage in the FIIND domain. **(E)** Comparison of NLRP1 truncation mutants in ASC-GFP speck formation. Full-length NLRP1 and truncation mutants were overexpressed in 293T-GFP cells by transient transfection. Cells were fixed 24 hours post-transfection. **(F)** Comparison of NLRP1 truncation mutants in keratinocyte IL-lb secretion. Full-length NLRP1 and truncation mutants were overexpressed in N/TERT- immortalized keratinocytes. Conditioned media were collected 16 hours post-transfection and analysed by IL-1b ELISA. **(G)** The C-terminal auto-proteolytic fragment of NLRP1 is sufficient to nucleate ASC-GFP specks. HA-tagged NLRP1 (amino acids 1213 to 1414) was overexpressed in 293T-ASC-GFP cells. Cells were fixed 24 hours post-transfection and stained with an anti-HA antibody. **(H)** Structural comparison of NLRP1 PYD (PDB: 1PN5) and NLRP3 PYD (PDB: 3QF2). The characteristic $\alpha$ helices that are common to the death domain (DD) super family are labelled as a1 to a6. The mutated residues in MSPC, A54, A66, and M77 are highlighted in gray in NLRP1 PYD while adjacent polymorphic residues are shown in dark gray. The NLRP3 residues that are structurally analogous to MSPC NLRP1 mutations are shown in black. **(I)** Difference in folding state between recombinant wild-type NLRP1 PYD and MSPC mutant PYDs analysed by 2D [$^{15}$N,$^1$H]-HSQC NMR. Cross peaks from the GB1 solubility tag are coloured gray. The dark gray arrows highlight individual NMR signals characteristic of the natively folded PYD domain. The shaded area, 7.9 ppm $< d_1(^1H) <$ 8.5 ppm, indicates the random-coil chemical shift region. Asterisks denote the indole side chain resonances of Trp6 and Trp67.

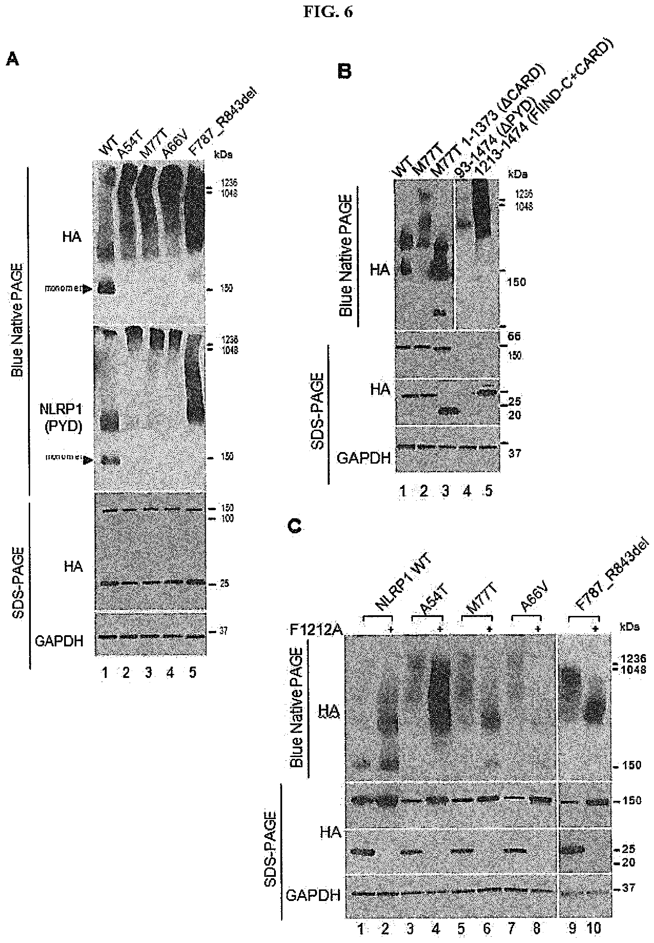

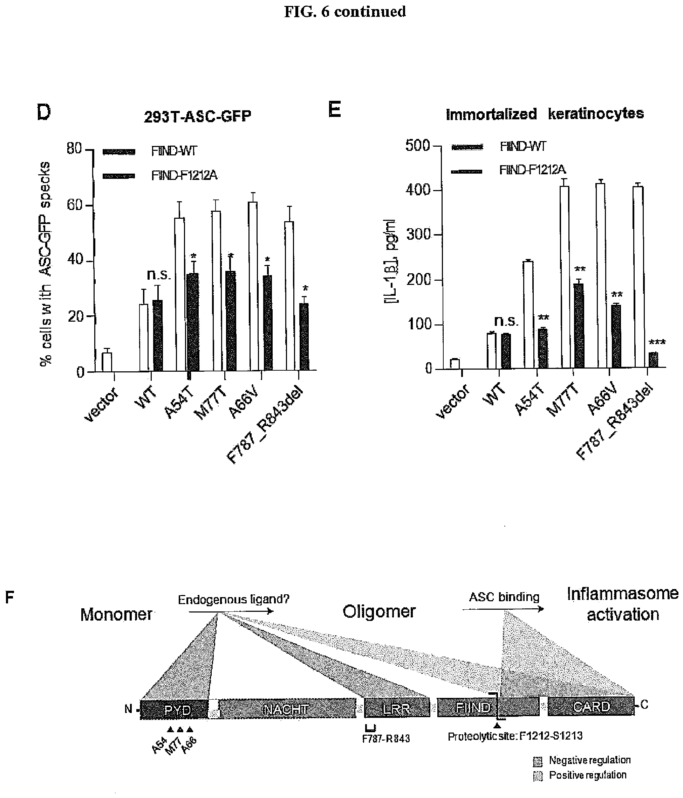

**Fig. 6** presents data showing that PYD and the LRR domain repress NLRP1 self-oligomerisation. **(A)** Distinct conformations of wild-type NLRP1 and NLRP1 mutants. Wildtype NLRP1 and NLRP1 mutants were transiently overexpressed in 293T cells. Cell pellets were harvested 48 hour post-transfection. 20 mg of lysate was analysed in parallel either in the native state by blue native PAGE (top) or in the denatured state by SDS-PAGE (bottom). Arrowheads indicate the putative NLRP1 monomer (~150 kDa). **(B)** Domain requirement for NLRP1 oligomerisation. The NLRP1 DPYD mutant had reduced level of accumulation. **(C)** NLRP1 oligomerisation requires auto-proteolytic cleavage within the FIIND domain. A cleavage site mutation, F1212A, was introduced in wild-type NLRP1 and NLRP1 mutants. All resulting mutants were expressed in 293T cells and analysed as (A) and (B). **(D)** Cleavage mutation F1212A reduces ASC-GFP speck formation by NLRP1 mutants in 293T-ASC-GFP cells. **(E)** Cleavage mutation F1212A in the FIIND domain reduces inflammasome activation in keratinocytes. Wild-type and the indicated NLRP1 mutants were overexpressed in N/TERT keratinocytes. Conditioned media was analysed 16 hour post-transfection by IL-1b ELISA. **(F)** shows a proposed model of NLRP 1 activation.

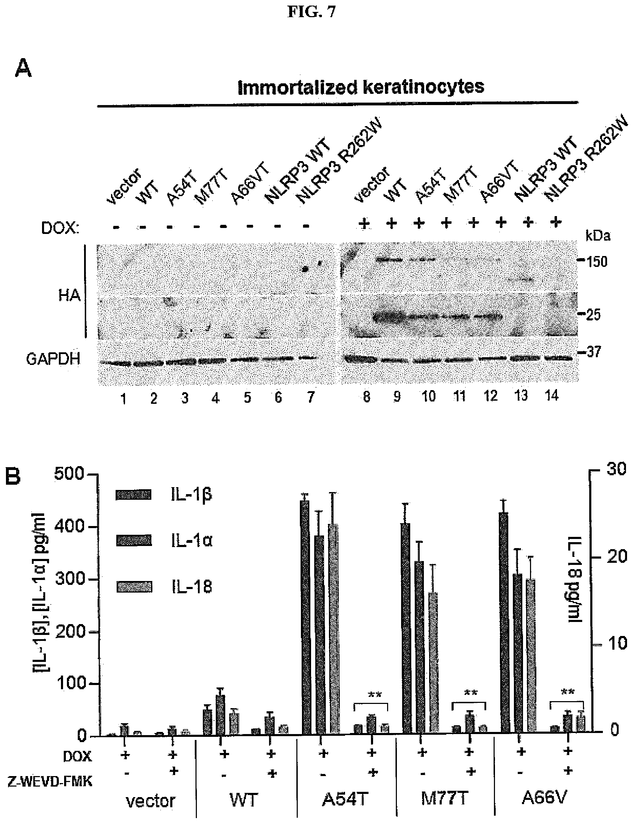

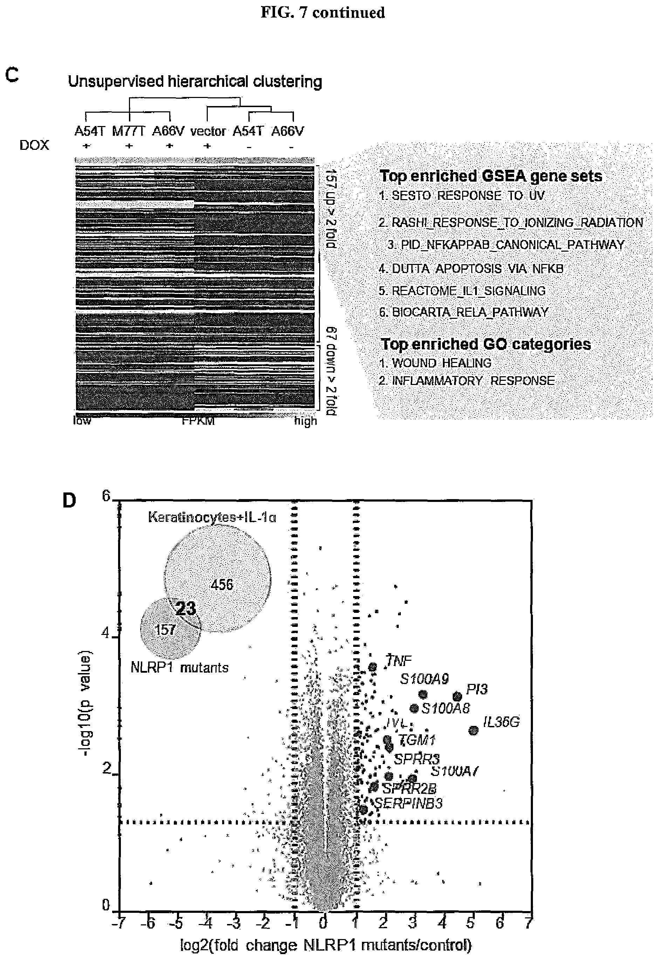

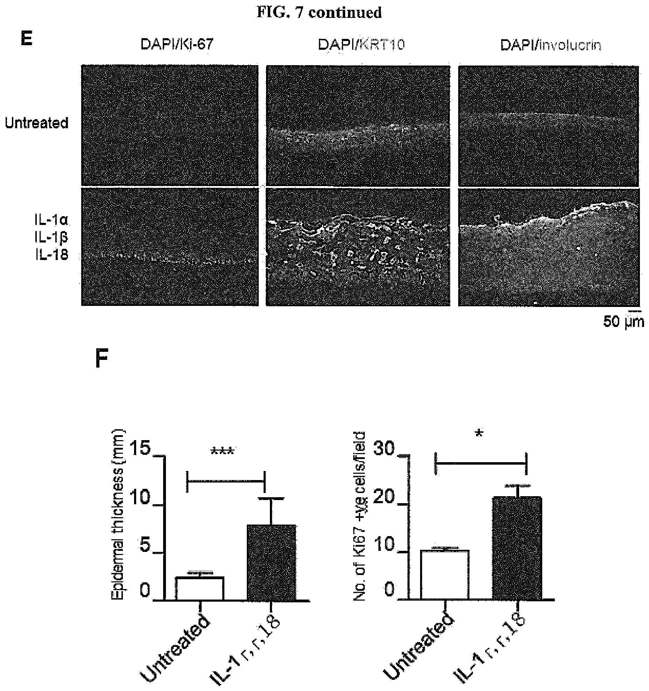

**Fig. 7** presents data showing that NLRP1 mutants cause inflammatory cytokine release and epidermal hyperplasia via paracrine IL-1 signalling **(A)** Induction of NLRP1 expression in immortalized keratinocytes. **(B)** Secretion of inflammasome-dependent cytokines IL-1a, IL-lb, and IL-18 following doxycycline induction of NLRP1 mutants. Conditioned media was harvested 16 hours after doxycycline addition and analysed by ELISA. For caspase-1 inhibition, cells were pre-treated with Z-WEVD-FMK for 1 hour before doxycycline addition. **(C)** RNA sequencing (RNA-seq) heat map and GSEA analysis of differentially expressed transcripts between control and NLRP1 mutant expressing keratinocytes. **(D)** Top upregulated genes in NLRP1 mutant expressing keratinocytes include stress-, inflammation-, and differentiation-related markers. Inset indicates overlap with a published dataset of IL-1a-induced transcripts in primary keratinocytes. **(E)** IL-1 cytokines induce epidermal hyperplasia in 3D skin organotypic culture. Reconstructed skin culture was either left untreated or treated with 10 ng/ml of IL-1a, IL-lb, and IL-18 for 7 days post-"air-lift". **(F)** Quantification of epidermal thickness and the number of Ki-67-positive cells in IL-1a, IL-lb, and IL-18 treated 3D skin organotypic cultures shown in (E).

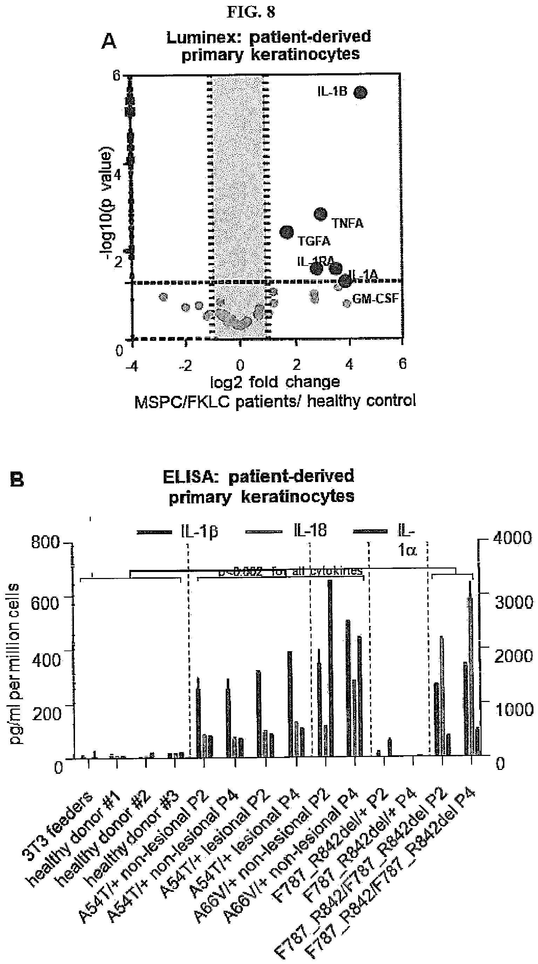

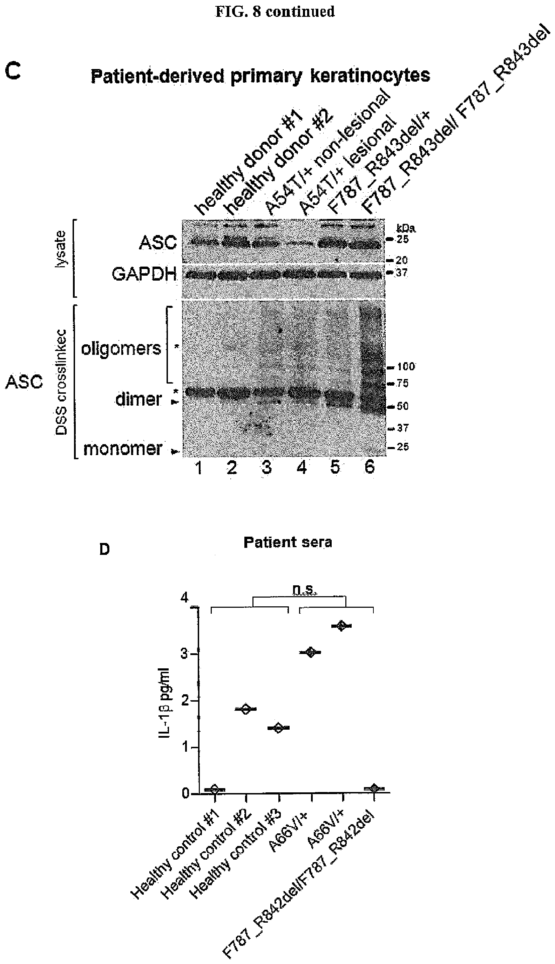

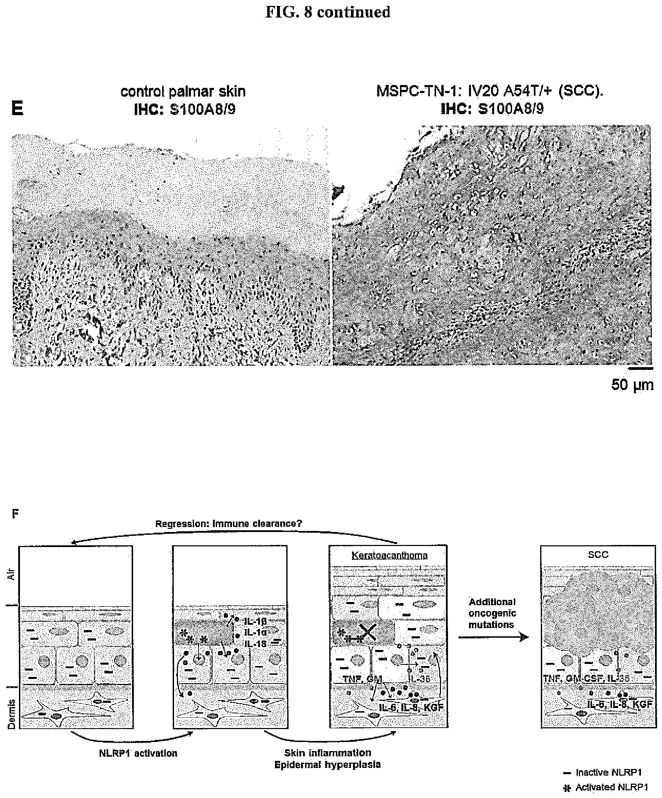

**Fig. 8** presents data showing *in vivo* evidence for keratinocyte-specific inflammasome activation in MSPC and FKLC patients. **(A)** Luminex cytokine/chemokine analysis of primary keratinocyte cultures derived from healthy donors and MSPC and FKLC patients. Top upregulated cytokines in patients are highlighted in red. The p value was calculated using one-tailed Student's t test. **(B)** High levels of IL-1 cytokines in early passage, patient-derived primary keratinocyte cultures. Cytokine levels were normalized to cell numbers. **(C)** Increased endogenous ASC oligomer formation in patient-derived primary keratinocytes. All keratinocytes were harvested at passage 4. Arrowheads indicate ASC monomers and dimers. Asterisks indicate non-specific bands. **(D)** No consistent increase in serum IL-1b levels in MSPC or FKLC patients as compared to healthy individuals. **(E)** Induction of inflammatory marker, S100A8 and S100A9 in an MSPC skin lesion biopsy. **(F)** Proposed model of pathogenesis for MSPC and FKLC.

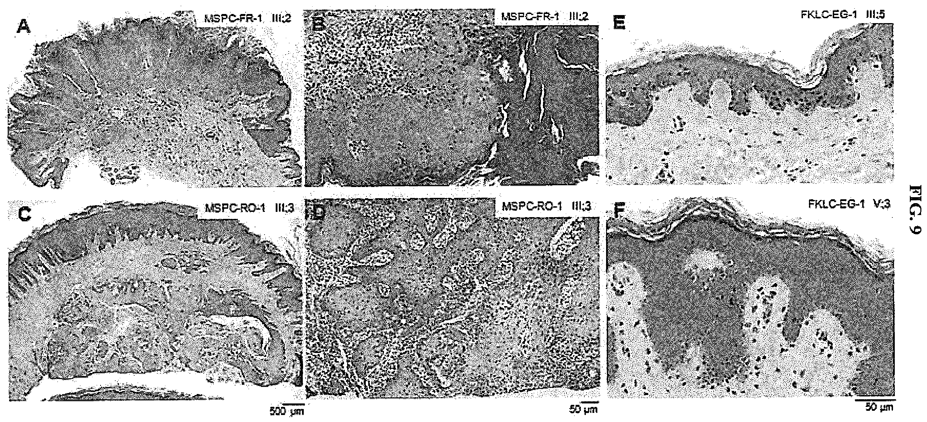

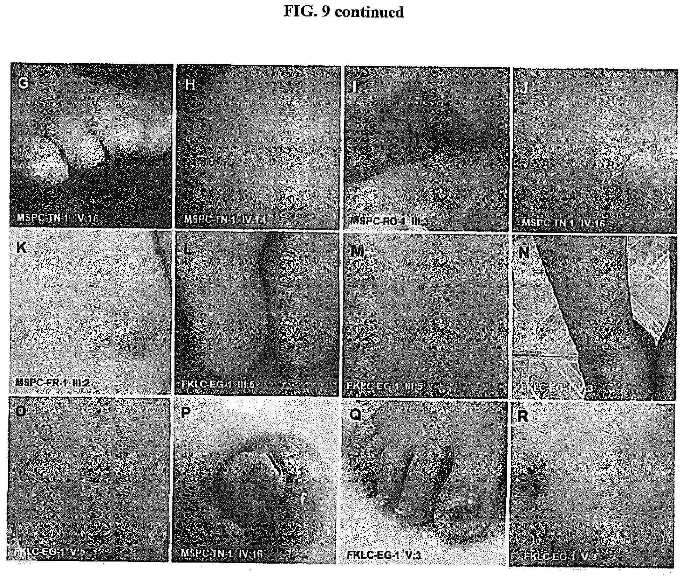

**Fig. 9** shows images of histology of characteristic lesions for MSPC and FKLC and additional clinical symptoms, in relation to Fig. 2 above. **(A)** Circumscribed acanthosis, hyperkeratosis, and papillomatosis with focal lichenoid infiltrate. **(B)** Hyperkeratosis, acanthosis and dyskeratosis with lichenoid infiltrate and pigmentary incontinence. High-magnification view of the lesion shown in A. **(C)** Well differentiated squamous cell carcinoma (SCC) with lichenoid inflammation. **(D)** SCC with evident keratinization and lichenoid inflammation. High-magnification view of the lesion shown in C. **(E)** Mild acanthosis and hyperkeratosis. Colloid bodies (apoptotic keratinocytes) present in papillary dermis. **(F)** Acanthosis and hyperkeratosis. Colloid bodies within papillary dermis are consistent with burnt-out lichenoid inflammation. **(G)** Crusted scaly papule abutting the right little toe nail. **(H)** Multiple pigmented scaly papules on the trunk. **(I)** Ulcerated scaly papule on the lower lip. **(J)** Variably sized small keratotic papules on the thigh. **(K)** Grouped and focally confluent scaly erythematous papules. **(L)** Focal keratoderma on the heels and soles. **(M)** Multiple dusky erythematous papules on the trunk. **(N)** Multiple aggregated hyperkeratotic violaceous papules. **(O)** Sheeted erythematous macules and papules on the trunk. **(P)** Thickened fragmented nail plate with surrounding inflammation involving the nail folds and distal margin. **(Q)** Thickening and irregular growth of the toe nails of the right foot. **(R)** Violaceous inflammatory plaques and crusting on the right cheek.

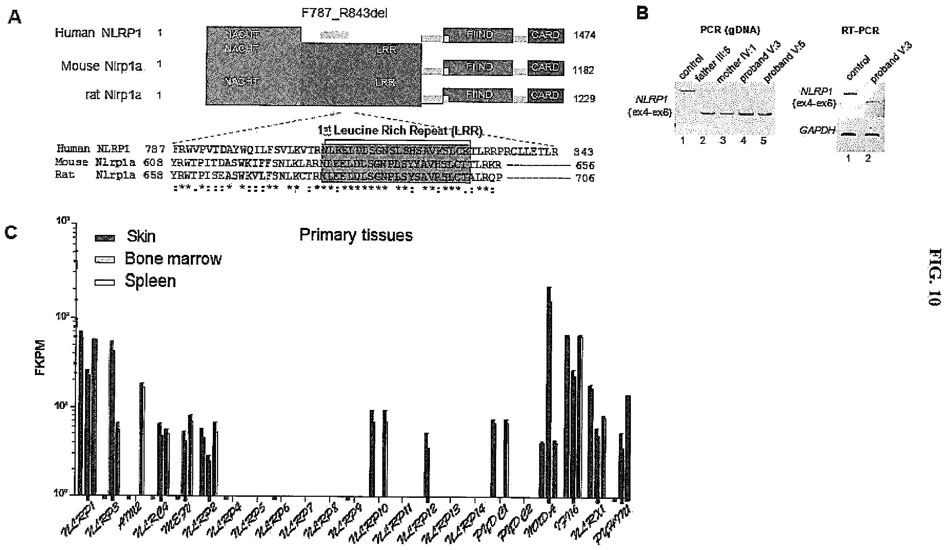

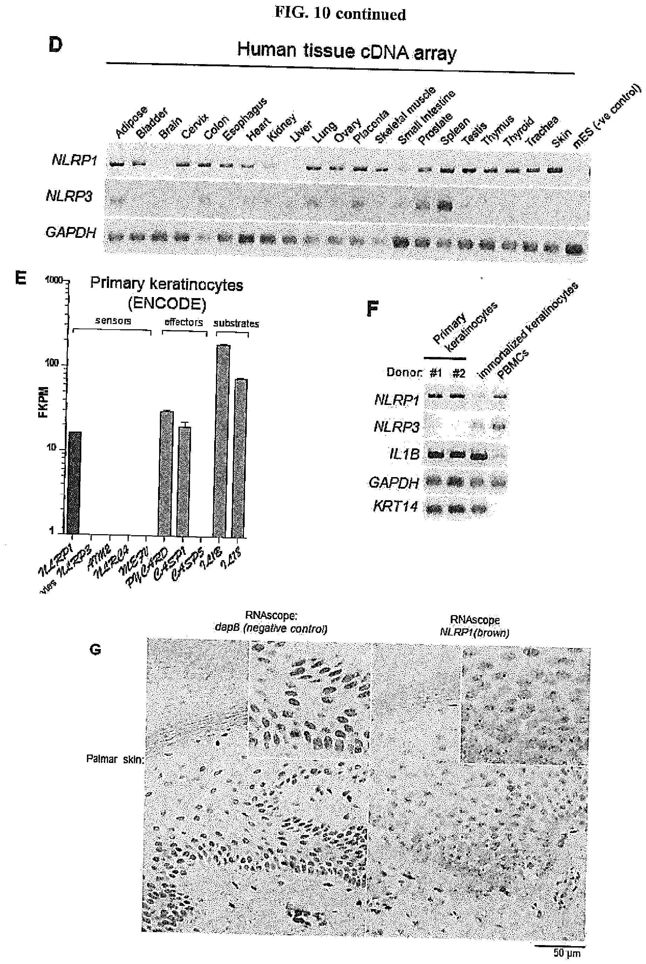

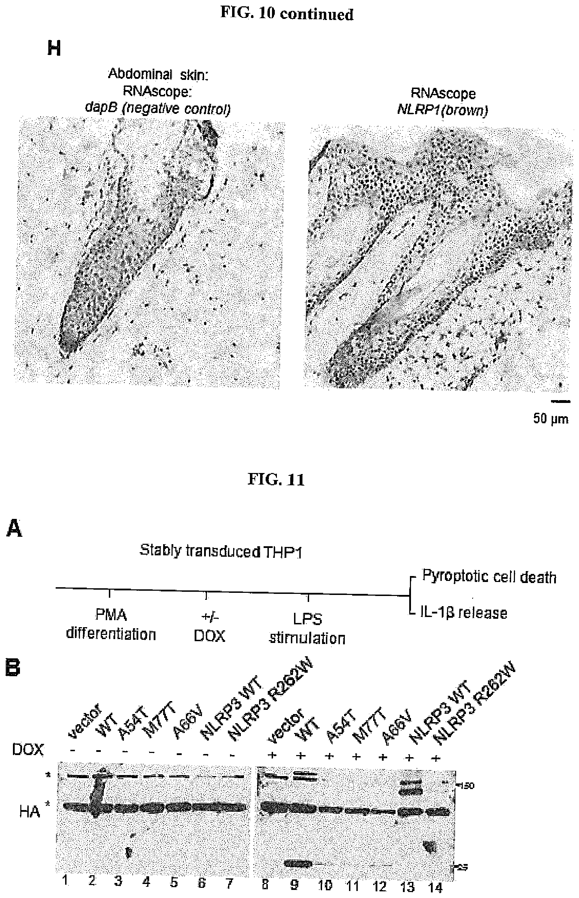

**Fig. 10** presents data showing the homozygous in-frame deletion of NLRP1 exon 5 in FKLC probands and prominent expression of NLRP1 in keratinocytes, in relation to Fig. 3. (A) Comparison of domain structure of human NLRP1 and rodent homologs. Top panel: the N-terminal PYD is only found in human NLRP1, while other domains, including the LRR, are conserved. Bottom panel: conservation of amino acid resides (F787-R842) that are deleted in FKLC-EG-1. **(B)** Germline deletion of NLRP1 exon 5 in FKLC-EG-1. Left panel: PCR validation of NLRP1 exon 5 deletion in FKLC-EG-1 genomic DNA, with the forward PCR primer in exon 4 and reverse primer in exon 6. Right panel: RT-PCR detection of exon 5-deleted NLRP1 transcript in mRNAs from FKLC-EG-1 patients. mRNA samples were extracted from buccal cells using the Oragene Saliva RNA collection kit. **(C)** Transcript levels of all PYD-containing genes in primary skin, bone marrow, and spleen tissues. RNA-seq FKPM values were obtained from Human Protein Atlas. **(D)** Relative expression levels of NLRP1 and NLRP3 in various human tissues. Mouse embryonic stem cells (mES) as negative control. Note that the GAPDH primers were designed to detect both human and mouse GAPDH. **(E)** Relative expression levels of known inflammasome sensors, effectors, and substrates in primary keratinocytes measured by RT-PCR. RNA-seq FKPM values were downloaded from the ENCODE project (Consortium, 2012). **(F)** Relative expression levels of NLRP1, NLRP3 and IL1B in primary human keratinocytes, N/TERT immortalized keratinocytes and PBMCs. **(G)** RNAscope in situ staining of NLRP1 in palmar skin. dapB, negative control. **(H)** RNAscope in situ staining of NLRP1 in hair follicles. dapB, negative control.

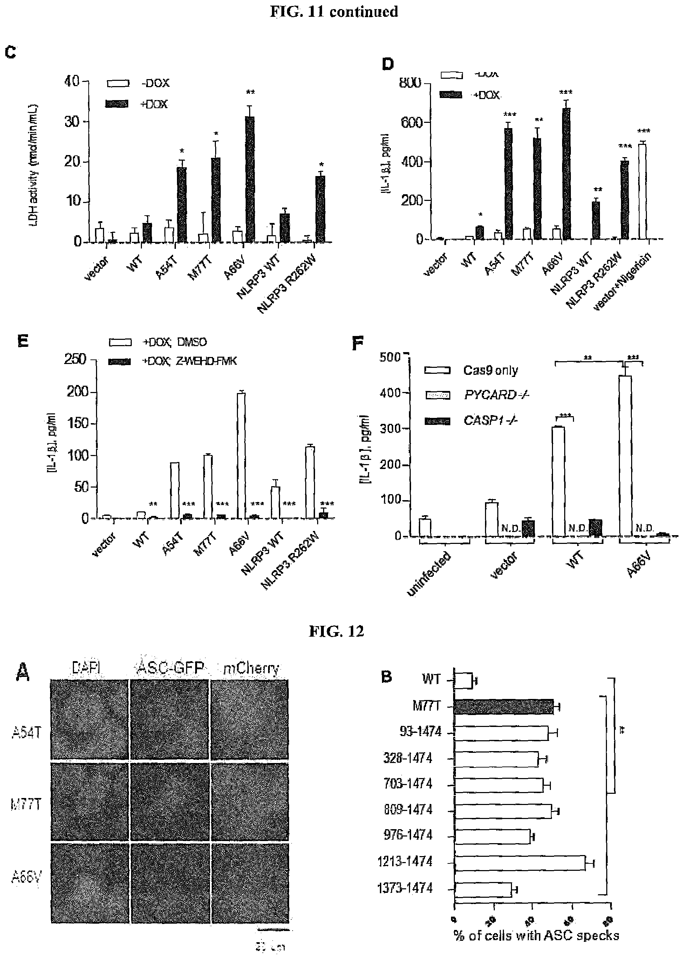

**Fig. 11** presents data showing that NLRP1 mutants in MSPC lead to ASC-dependent inflammasome hyper-activation in THP-1 derived macrophages, in relation to Fig. 4. **(A)** Experimental design to measure inflammasome activation in THP-1 derived macrophages. **(B)** Level of NLRP1 induction 24 hour post-doxycycline (1 mg/ml) addition. **(C)** Increased cell death due to NLRP1 mutant expression measured by lactase dehydrogenase activity. **(D)** Increased IL-lb secretion due to NLRP1 mutant expression. **(E)** IL-1b secretion caused by NLRP1 mutant induction is blocked by a caspase-1/4/5 inhibitor, Z-WEHD-FMK. **(F)** Increased IL-1b secretion by NLRP1 A66V depends on caspase-1 and ASC. THP-1 CRISPR knockout cell lines, CASP1$^{-/-}$ and PYCARD (ASC)$^{-/-}$ were reported previously.

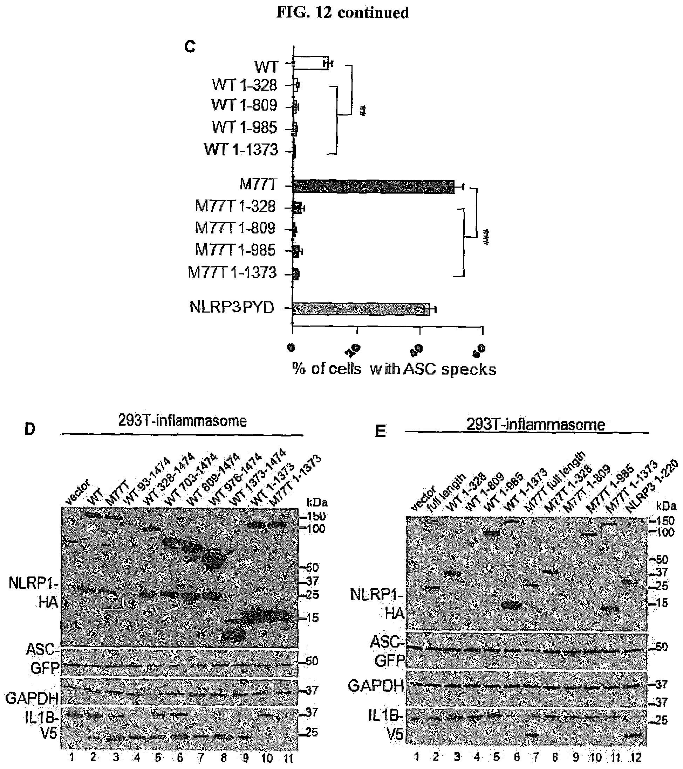

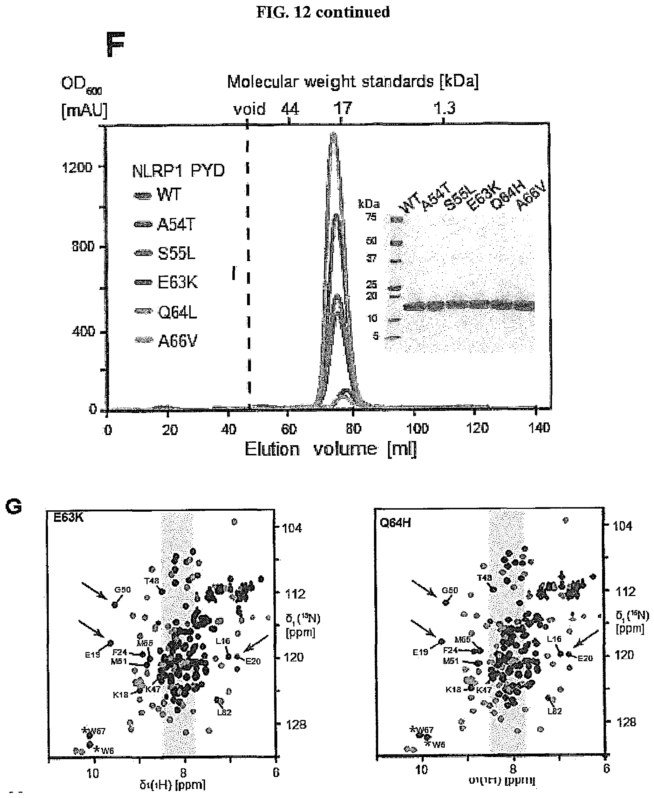

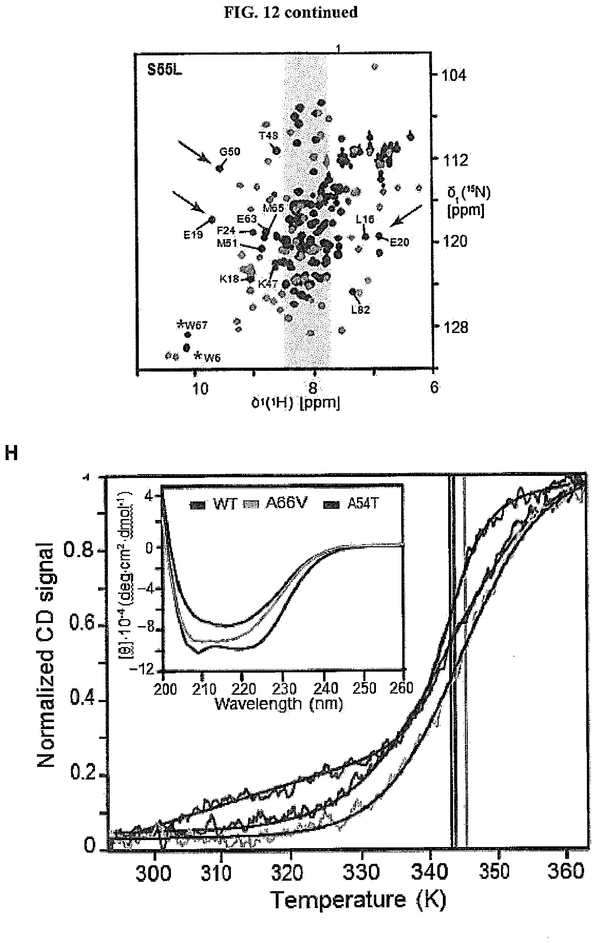

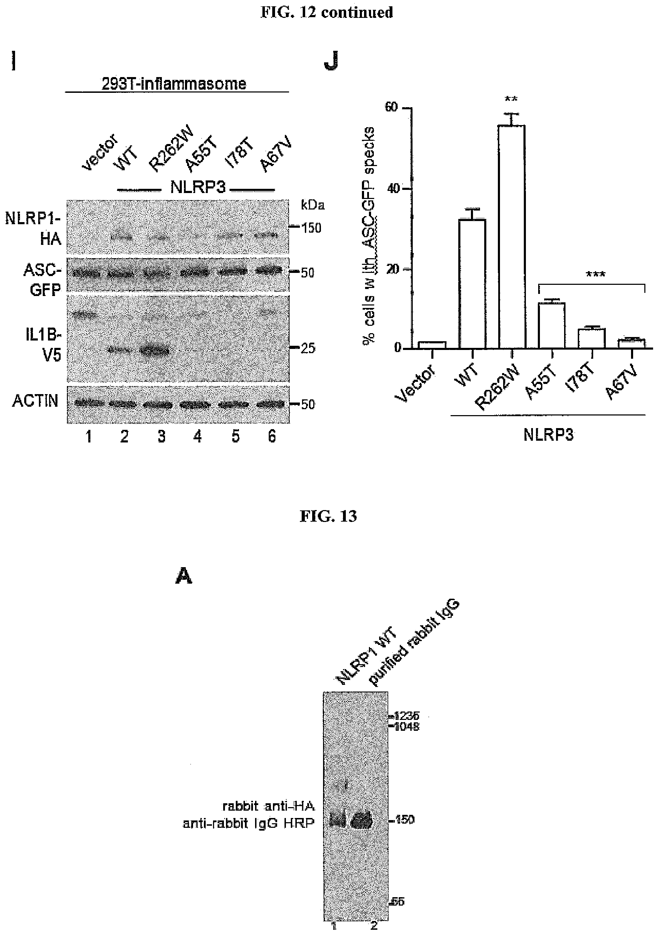

**Fig. 12** presents additional data showing the auto-inhibitory role of NLRP1 PYD and the misfolding of pathogenic PYD mutants, in relation to Fig. 5. **(A)** MSPC NLRP1 PYD mutants are unable to nucleate ASC-GFP specks. All NLRP1 PYD mutants were expressed as mCherry-tagged fusions and transfected into 293T-ASC-GFP cells. **(B)** Comparison of NLRP1 N-terminal truncation mutants in ASC-GFP speck formation in 293T-ASC-GFP cells. (C) Comparison of NLRP1 C-terminal truncation mutants in ASC-GFP speck formation. NLRP3 PYD was included as a positive control. All mutants were ex- pressed in 293T-ASC-GFP cells and assayed 24 hours post transfection. **(D)** Pro-caspase-1 activation and pro-IL-1b processing by NLRP1 N-terminal truncations mutants. All NLRP1 mutants were co-transfected with pro-caspase-1 and pro-IL-1b-V5 in 293T-ASC-GFP cells. Note that the NLRP1 DPYD mutant (amino acids 93 to 1474) shows consistently low expression levels and is only very weakly detectable 16 hours post-transfection. **(E)** Pro-caspase-1 activation and pro-IL-1b processing by NLRP1 C-terminal truncation mutants. **(F)** Gel filtration profiles of all purified NLRP1 PYDs. Recombinant wild-type and mutant NLRP1 PYDs eluted as monomeric fractions. Inset: Coomassie stain of SDS-PAGE gel with all recombinant NLRP1 PYDs. **(G)** 2D [$^{15}$N,$^{1}$H]-HSQC NMR spectra of NLRP1 SNP variants. **(H)** Comparison of thermal unfolding curves for wildtype PYD, PYD A66V and PYD A54T. The black line represents the fit of the data to a two-state equilibrium folding model. Vertical lines reports individual melting temperature (Tm) for wildtype PYD and its mutants as obtained from fitting procedure. Compared to the wild-type spectrum, the spectra of the NLRP1-PYD mutants A66V and A54T feature a reduced amplitude of helical content and a single dominant b sheet peak at 215 nm, indicating that the $\alpha$-helical content of the PYD is substantially reduced as a result of the mutations. Thermal melting experiments monitored by CD spectroscopy are dominated by the unfolding of the GB1 tag, which has a strong unfolding transition around 343K for all constructs. Thermal unfolding of the wild-type PYD progresses through a broadened transition in the range 305-335K, indicative of non-cooperative unfolding. The spectral profiles for both mutants A66V and A54T lack this broad transition, in agreement with the absence of a stable PYD fold. **(I)** Structurally analogous MSPC mutations in NLRP3 abrogate caspase-1 activation. All mutants were co-transfected with pro-caspase-1 and pro-IL-1b-V5 in 293T-ASC-GFP cells. **(J)** Analogous MSPC mutations in NLRP3 abrogate ASC-GFP speck formation

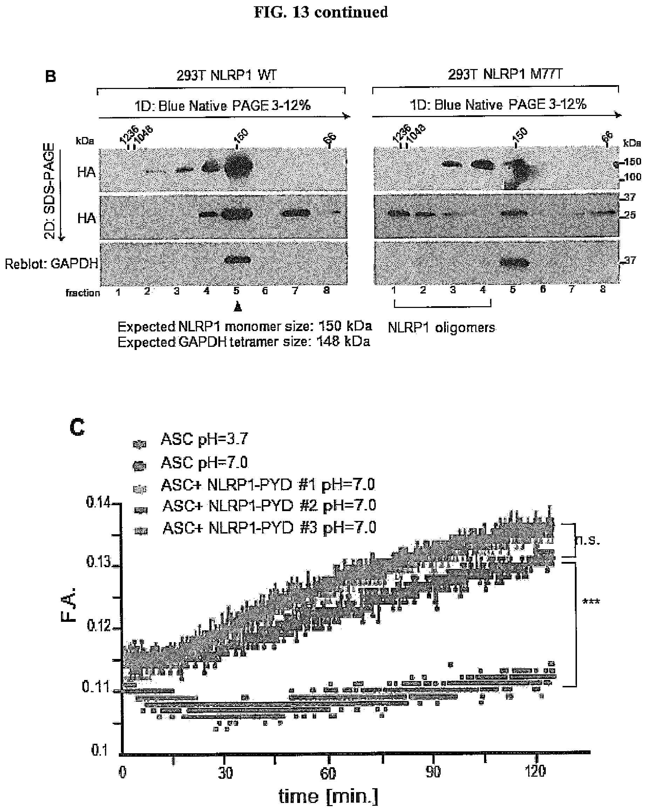

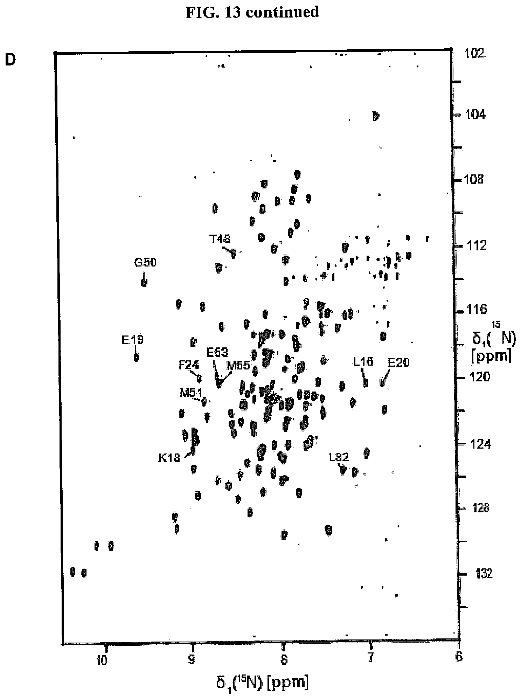

**Fig. 13** presents supporting data for the NLRP1 activation mechanism, in relation to Fig. 6. **(A)** Wild-type NLRP1 is predominantly a monomer. 293T cells transiently transfected with HA-tagged wild-type NLRP1 were lysed in BN-PAGE lysis buffer containing 1% digitonin. 20 mg of lysate was analysed on Blue Native PAGE along with 1ng of purified rabbit IgG, followed by western blot with a rabbit anti-HA antibody. An HRP-conjugated secondary antibody against all rabbit IgG subtypes was used to visualize NLRP1 and the purified rabbit IgG in the native form. Rabbit IgGs are ~150kDa in molecular weight. **(B)** NLRP1 M77T mutation leads to increased oligomerisation by the C-terminal cleavage fragment. 20 $\mu$G of 293T lysate expressing wild-type NLRP1 and NLRP1 M77T were fractionated using Blue Native PAGE. After electrophoresis, entire lanes were excised from the gel and cut into 8 equal pieces. Proteins were eluted from each gel fraction in 50 mM Tris-HCl, 0.1% SDS, 150 mM NaCl at pH 7 at room temperature overnight and concentrated 50-fold. The concentrated proteins were further analysed along the second dimension by SDS-PAGE. **(C)** Fluorescence anisotropy measurement of the effect of NLRP1 PYD on ASC filament formation. ASC filament formation was initiated by a pH switch from 3.7 to 7.0. No significant difference was detected in the presence of NLRP1 PYD. **(D)** Overlay of 2D [$^{15}$N,$^{1}$H]-HSQC NMR spectra of recombinant NLRP1 PYD and after addiction of CARD. No significant chemical shift perturbations were detected.

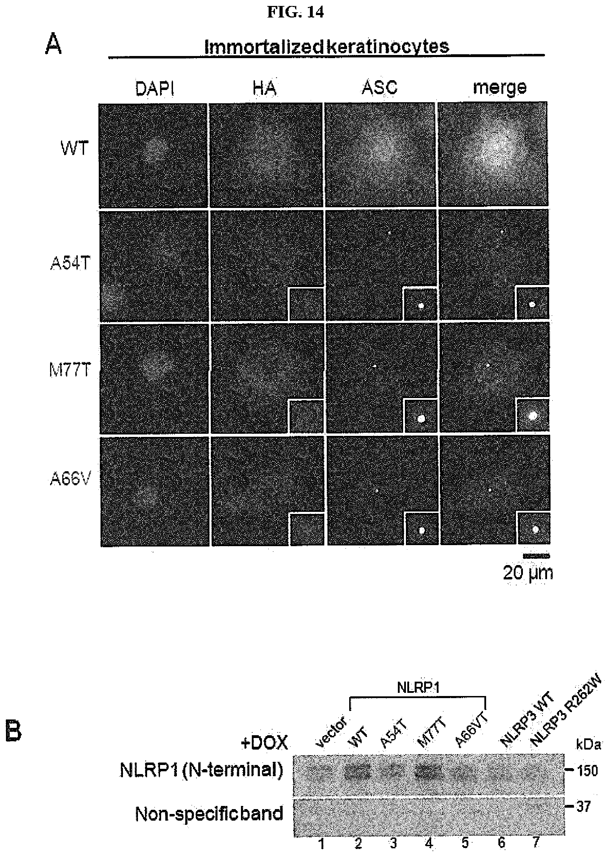

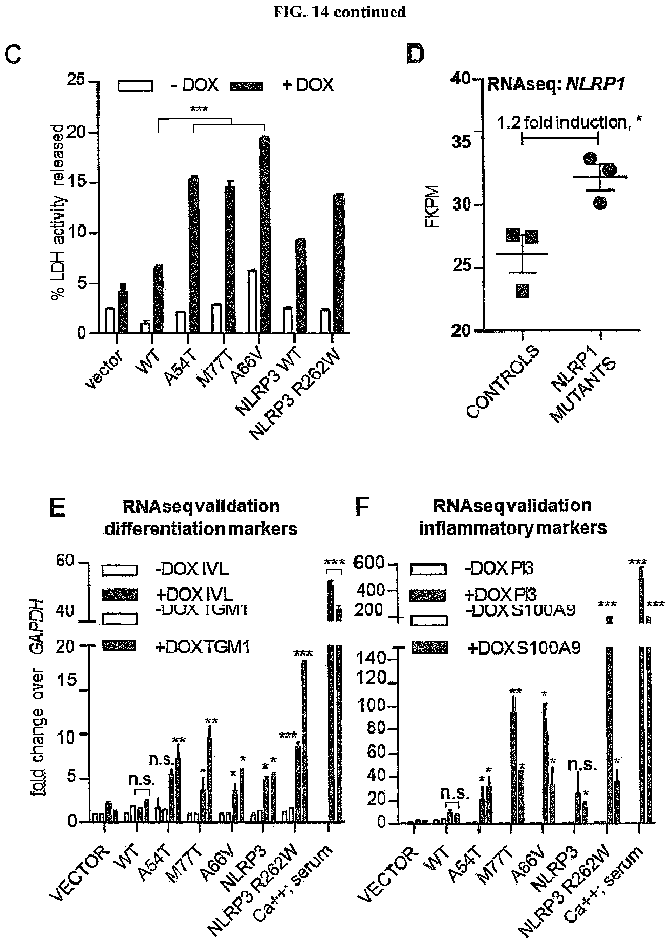

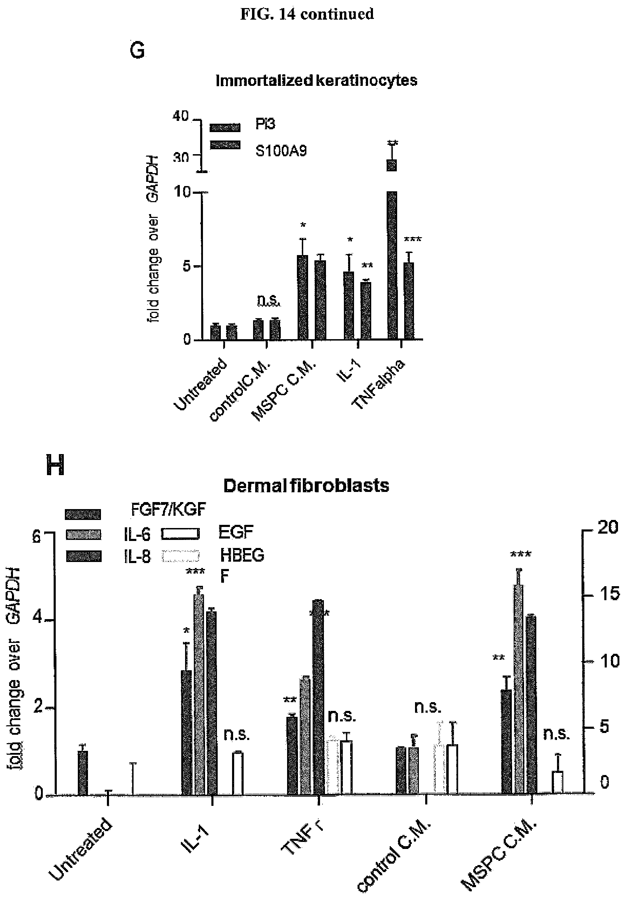

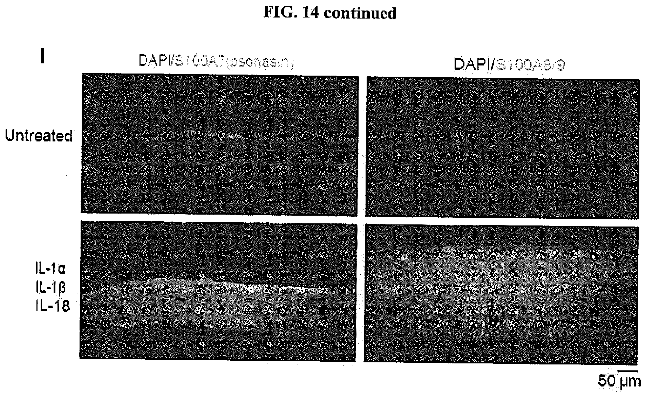

**Fig. 14** presents data showing the additional characterization of paracrine IL-1 signalling following NLRP1-dependent inflammasome activation in keratinocytes, in relation to Fig. 7. **(A)** Immunostaining of ASC specks in immortalized keratinocytes after doxycycline induction of NLRP1 expression. Wild-type NLRP1 and mutant NLRP1 were detected by immunofluorescence staining against the HA tag. **(B)** Level of NLRP1 induction relative to the endogenous protein (lane 1) in keratinocytes. A non-specific band from the same NLRP1 western blot was used as a loading control.

**(C)** Induction of cell deaths following NLRP1 induction in immortalized keratinocytes measured by lactase dehydrogenase activity. **(D)** Level of NLRP1 transcript induction in RNA-seq samples shown in Fig. 6C and 6D. **(E)** Q-PCR validation of RNA-seq results: keratinocyte differentiation markers, IVL and TGM1. **(F)** Q-PCR validation of RNA-seq results: skin inflammation markers, PI3 and S100A9. **(G)** Recombinant IL-1 and TNFa and MSPC primary keratinocyte-conditioned media induce skin inflammation markers in keratinocytes. **(H)** Recombinant IL-1 and TNFa and MSPC primary keratinocyte-conditioned media induce inflammatory cytokines IL-6, IL-8 and KGF in fibroblasts. Other growth factors such as EGF and HB-EGF were not responsive to IL-1 and TNFa stimulation and served as negative controls. **(I)** Recombinant IL-1 treatment upregulates inflammatory markers S100A7, S100A8 and S100A9 in stratified epidermis in *ex vivo* organotypic cultures. IL-1a, IL-Ib and IL-18 were added to the culture medium a final concentration of 10 ng/ml each. All cultures were fixed and stained 7 days after 'air-lift'.

**Fig. 15** presents additional *in vivo* data for skin-specific inflammasome activation in MSPC and FKLC patients, in relation to Fig. 8. (A) Luminex cytokine and chemokine analysis of patient sera. No significant change was detected between healthy controls and probands. **(B)** Immunostaining of inflammatory marker, S100A7/psoriasin in a MSPC SCC biopsy.

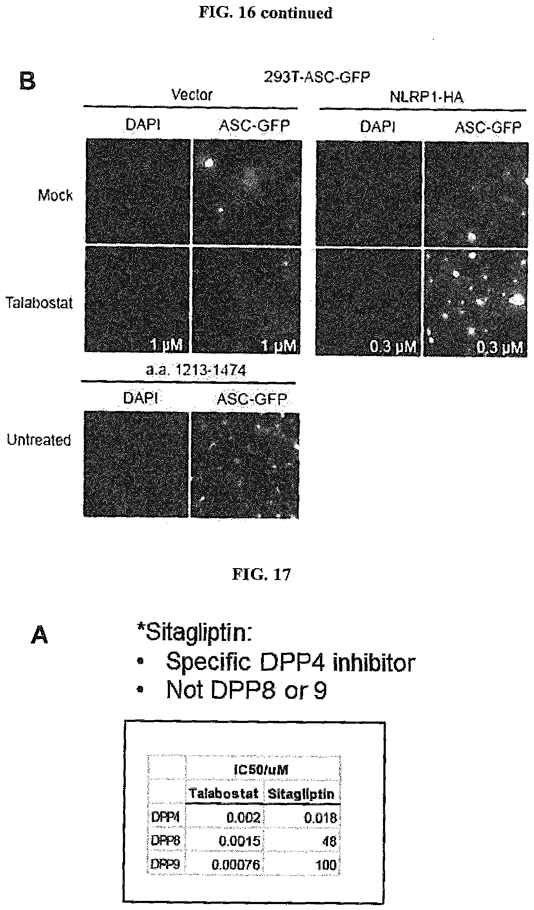

Fig. 16 presents data showing that Talabostat induces ASC-GFP specks in the presence of NLRP1.

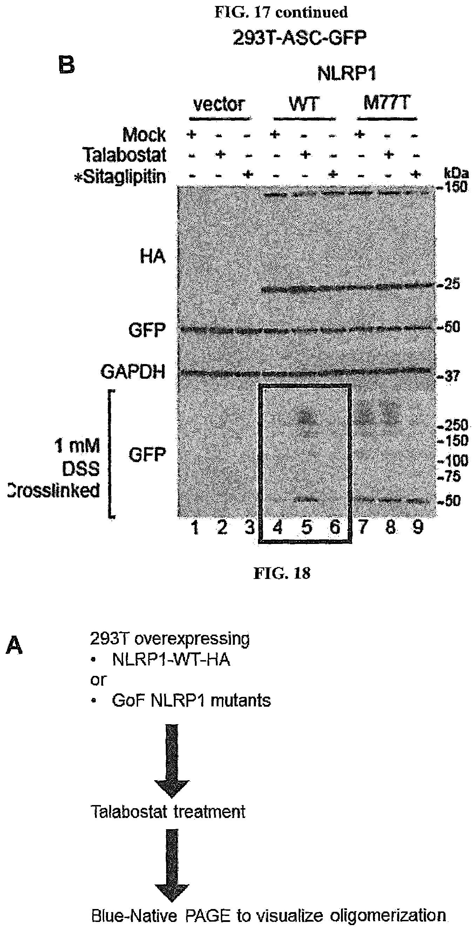

Fig. 17 presents data showing that talabostat induces ASC-GFP oligomers. It is shown that cross-link ASC-GFP oligomers and the Western Blot data shows preserved oligomers.

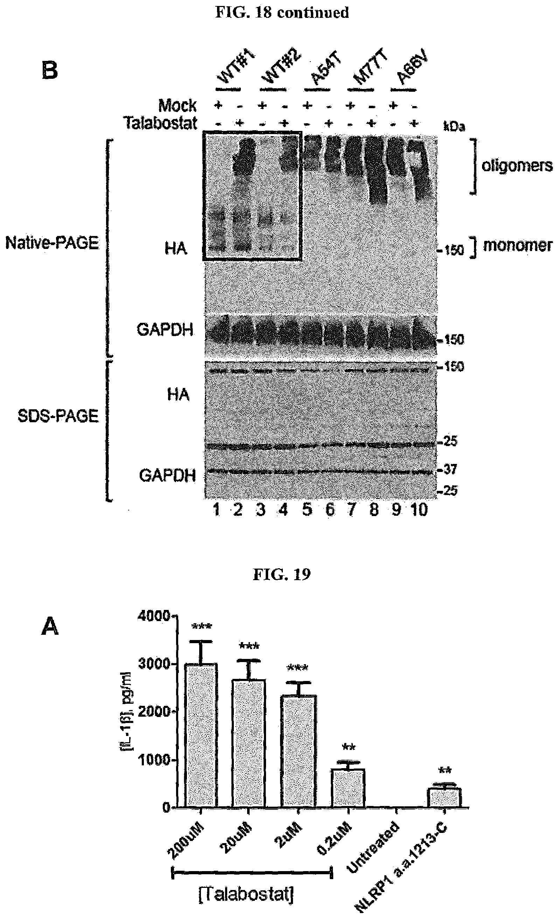

Fig. 18 presents data showing that Talabostat induces NLRP1 oligomerization.

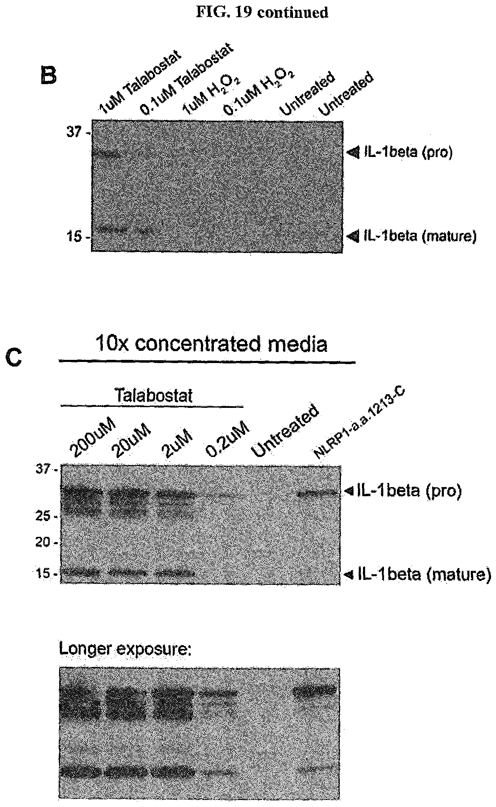

Fig. 19 presents data showing that Talabostat induces conventional pyroptosis in keratinocytes (IL-1beta).

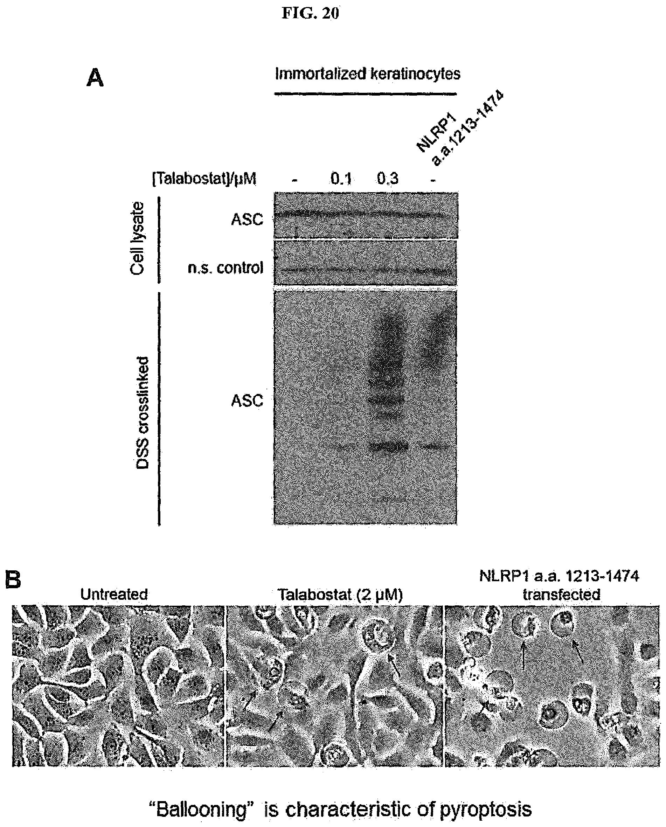

Fig. 20 presents data showing that Talabostat induces conventional pyroptosis in keratinocytes (ASC, morphology).

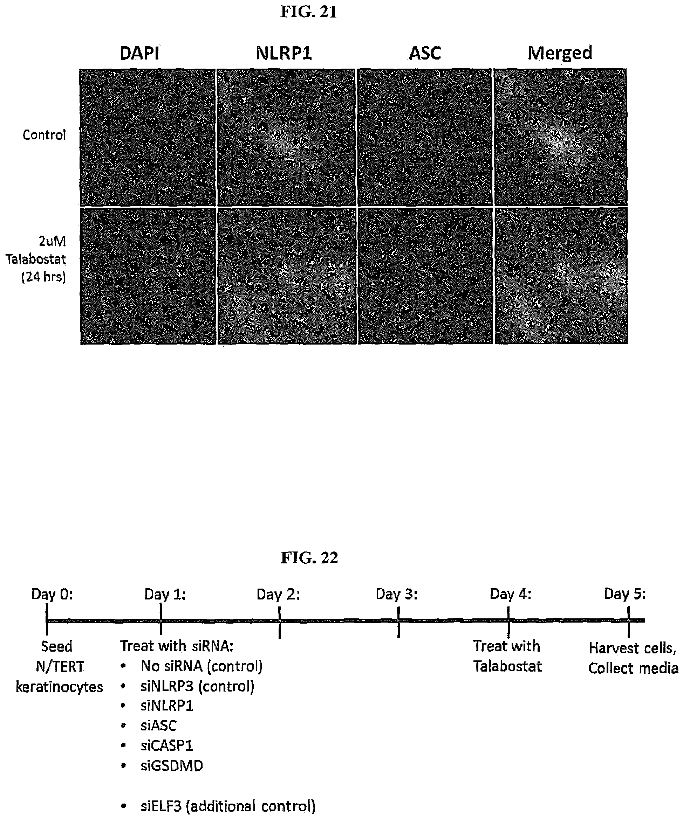

Fig. 21 presents data showing that Talabostat induces conventional pyroptosis in keratinocytes (IF).

Fig. 22 presents data showing the requirement of inflammasome components in Talabostat-induced pyroptosis.

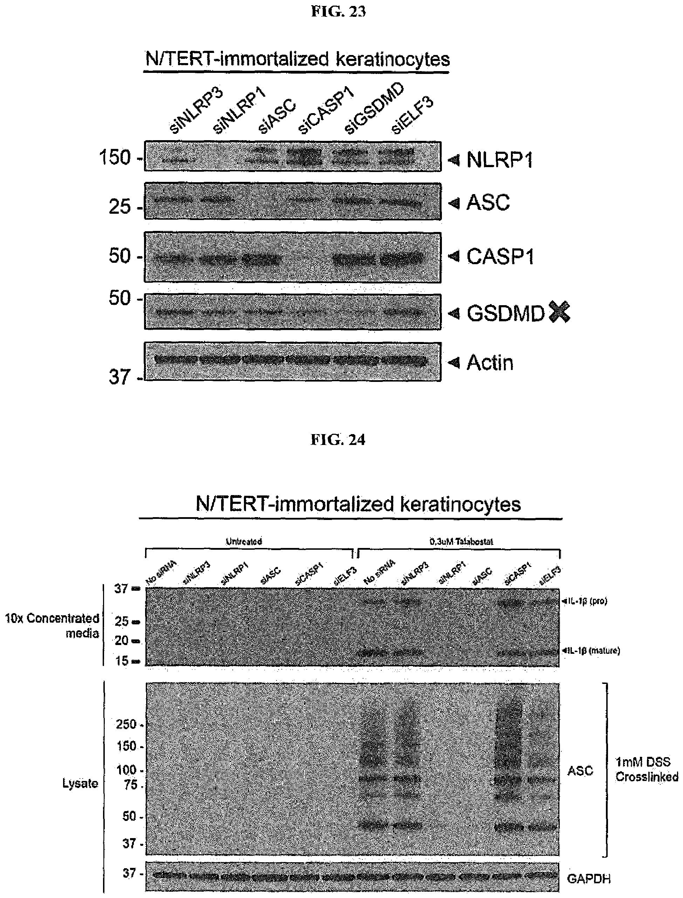

Fig. 23 presents data showing the siRNA knockdown of inflammasome components.

Fig. 24 presents data showing that Talabostat-induced pyroptosis is NLRP1 and ASC-dependent

**DEFINITIONS**

Substitutions are herein indicated by providing the wild-type amino acid residue, followed by the position number, followed by the substituted amino acid residue to be substituted. As would be apparent to the person skilled in the art, the amino acid residue position number is with reference to the amino acid sequence of wildtype human NLRP1 (as provided in Fig. 3B).

As would be apparent to the person skilled in the art, the amino acid residue position number is with reference to the amino acid sequence of wildtype human NLRP1 (as provided in Fig.10A).

**DETAILED DESCRIPTION OF THE PRESENT INVENTION**

A key pathway in the human innate immune system is reliant upon inflammasome complexes, which consist of an array of, for example, ligand-sensing nucleotide-binding domain, leucine-rich repeat containing (NLR) proteins, the adaptor protein ASC, and caspase-1, for activation. As used herein, the term "inflammasomes" refer to a multi-protein, intracellular complex that detects pathogenic microorganisms and sterile stressors, and that activates the highly pro-inflammatory cytokines interleukin-1b (IL-1b) and IL-18. Inflammasomes also known to induce a form of cell death termed pyroptosis. Dysregulation of inflammasomes is associated with a number of auto inflammatory syndromes and autoimmune diseases.

For example, ligand-sensing nucleotide-binding domain, leucine-rich repeat containing (NLR) proteins patrol the cytosol and initiate inflammasome assembly, pyroptotic cell death, and pro-inflammatory cytokine release upon ligand binding. While the inflammasome complexes are essential for pathogen clearance under physiological conditions, their aberrant activation can be detrimental to their host. Here, it is shown that germline mutations in the inflammasome sensor NLRP1 cause at least two overlapping skin disorders: multiple self-healing palmoplantar carcinoma (MSPC) and familial keratosis lichenoides chronica (FKLC). A group of non-fever inflammasome disorders has been established, thereby uncovering previously unknown auto-inhibitory function for the pyrin domain, and providing a genetic link between NLRP1 and skin inflammatory syndromes and skin cancer predisposition.

Herein described, but not forming part of the invention, is a method of treating or preventing an autoimmune skin disorder and/or inflammatory skin disorder in a subject in need thereof. In one example, the method comprises administering a therapeutically effective amount of an inhibitor capable of inhibiting the activation of inflammasome sensor NLRP1 (Nucleotide-binding domain, leucine-rich repeat containing (NLR) family, pyrin domain containing protein 1 - NLPR1).

As used here, the term "inhibiting" or "inhibition" refers to the ability of a given compound to limit, prevent or block the action or function of the target compound. This can be cause by, for example, the binding of the inhibitor resulting in a conformational change in the target compound, thus rendering the target compound unable to further function in a normal fashion compared to an uninhibited target compound. The binding of the inhibitor can be, for example, reversible or irreversible, depending on the principles underlying the binding of the inhibitor to the target molecule. In terms of inhibition mechanisms, this can take place using different biological or chemical principles. For example, in terms of enzyme inhibition, an inhibitor can inhibit an enzyme via inhibition that is competitive, uncompetitive, noncompetitive or mixed. An enzyme can also be inhibited via covalent inactivation, which is an example of irreversible inhibition.

As used herein, the term "inhibitor capable of inhibiting the activation of inflammasome sensor NLRP1" or "NLRP1 inhibitor" refers to an agent or a compound that is capable of inhibiting the activation of NLRP1. For example, a compound capable of preventing oligomerisation of NLRP1, a mechanism by which the NLRP1 protein initialises downstream pathways, is considered to fall within the ambit of the term "NLRP1 inhibitor".

The term "oligomerisation" refers to a chemical process that links monomeric (that is single unit) compounds, for example, but not limited to, amino acids, nucleotides, monosaccharides, or chemical monomers, to form dimers, trimers, tetramers, or longer chain molecules (also known as multimers or oligomers).

Examples of oligomerisation include, but are not limited to, self-oligomerisation, which is the oligomerisation of one peptide unit with one or more multiple units of itself (that is being identical in structure or sequence), and examples of oligomerisation of peptides to other peptides that are not identical in structure or sequence.

Thus, in one example, the inhibitor inhibits the activation of NLRP1 by preventing an oligomerisation of NLRP1. In another example, the inhibitor inhibits the activation of NLRP1 by preventing self-oligomerisation of NLRP1. In another example, the inhibitor inhibits the activation of NLRP1 by preventing oligomerisation between the NLRP1, or fragments thereof, and inflammasome adaptor protein ASC (apoptotic speck protein).

Other methods of inhibition include, for example, inhibiting the binding of the intended binding partner of a protein, for example a receptor or another protein, by displaying the same binding site as the receptor, for example, but not acting in the same manner as the intended binding partner once the peptide is bound. Another example of protein inhibition is mimicking the function of a natural inhibitor and thereby inhibiting the function of the target protein. Thus, in one example, the inhibitor inhibitis the activation of NLRP1 by mimicking the function of wild type PYD domain (pyrin domain) and wild type LRR (leucine-rich repeats) domain.

Compounds or agents capable of such inhibition may not belong to the same chemical group or have structural similarities, but are grouped together by their ability to function as NLRP1 inhibitor. Examples of such inhibitors as disclosed above are, but are not limited to, a small molecule, an antibody, a polypeptide, and a nucleic acid. In one example, the inhibitor of the oligomerisation between NLRP1, or fragments thereof, and the inflammasome adaptor protein ASC is, but is not limited to, a small molecule, an antibody, a polypeptide, and a nucleic acid.

Another method by which the self- activating properties of NLRP1 can be prevented is, for example, by preventing proteolytic cleavage of NLRP1. Proteolytic cleavage is a process by which peptides are usually degraded, in other words, cut into shorter fragments. In some example, only specific sections of the peptide are cleaved, for example the N- or the C-terminus of a peptide, thereby conferring the cleaved peptide with new or previously dormant capabilities. One example of such peptides is a zymogen, which is an inactive precursor of an enzyme. Zymogens require a biochemical change (for example, a hydrolysis reaction revealing the active site, cleavage to an active form, or changing the configuration to reveal the active site) in order for it to become an active enzyme.

Thus, in one example, the inhibitor prevents the proteolytic cleavage of NLRP1. In another example, the location of the proteolytic cleavage is within the FIIND domain of NLRP1. In another example, wherein the location of the proteolytic cleavage is between phenylalanine at position 1212 and serine at position 1213 (also written as F1212-S1213).

In another example, the inhibitor of the proteolytic cleavage is selected from the group consisting of a small molecule, an antibody, a polypeptide, and a nucleic acid.

Herein described, but not forming part of the invention, are also methods of treating or preventing an autoimmune skin disorder and/or inflammatory skin disorder in a subject. In one example, the method comprises administering a therapeutically effective amount of an agent that prevents the secretion of stress responsive secreted factors (also known as pro-inflammatory cytokines), keratinocytes differentiation markers, inflammasome-dependent cytokines, and growth factors in the skin.

In one example, the inflammasome-dependent cytokine is, but is not limited to, IL-1$\alpha$ (interleukin-1alpha), IL-1$\beta$ (interleukin-lbeta), and IL-18 (interleukin-18). In another example, the inflammasome-dependent cytokine is at least one, at least two, or more inflammasome-dependent cytokines. In yet another example, the inflammasome-dependent cytokine is one, two, three or four of the inflammasome-dependent cytokines disclosed herein.

Also disclosed herein is a method of determining whether a skin inflammation in a subject is an inflammatory skin disorder and/or an autoimmune skin disorder selected from MSPC (multiple self-healing palmoplantar carcinoma), and FKLC (familial keratosis lichenoides chronica). In one example, the inflammatory skin disorder and/or an autoimmune skin disorder is MSPC. In another example, the MSPC is, but is not limited to, MSPC-TN-1, MSPC-RO-1, MSPC-FR-1, and MSPC SCC. In yet another example, the inflammatory skin disorder and/or an autoimmune skin disorder is FKLC. In a further example, the FKLC is FKLC-EG-1.

Herein described, but not forming part of the invention, is a method of treating or preventing cancer in a subject in need thereof. In one example, the method comprises administering a therapeutically effective amount of an activator capable of activating inflammasome sensor NLRP1.

Examples of such activators capable of activating inflammasome sensor NLRP1 are, but are not limited to, compounds that are known to have antineoplastic and/or hematopoiesisstimulating activities. In another example, the activators are small molecules. Examples of such activators as described herein are, but are not limited to talabostat, CHEMBL305170, CHEMBL195189, CHEMBL383705, CHEMBL16709, CHEMBL66032, CHEMBL63406, CHEMBL1790483, CHEMBL63698, CHEMBL1813248, CHEMBL460984, and CHEMBL65406. In one example, the activator is talabostat, also known as Val-boro-Pro (PubChemCID: 6918572).

Also herein described, but not forming part of the invention, is a method of treating obesity, metabolic syndrome, and/or metabolic disorder in a subject in need thereof, comprising administering a therapeutically effective amount of an inflammasome sensor NLRP1 mutant into the subject in need thereof.

As used herein, the term "obesity" refers to a medical condition in which excess body fat has accumulated to the extent that it can have a negative effect on health. In general, subjects are considered to be obese when their body mass index (BMI), a measurement obtained by dividing a person's weight by the square of the person's height, is over 30 kg/m$^2$, whereby the range of 25 to 30 kg/m$^2$ is defined as being overweight. The definition of obesity, as established by the World Health Organisation, is provided in the table below:

| BMI (kg/m$^2$) | | Classification |
|---|---|---|
| from | Up to | |
| | 18.5 | Underweight |
| 18.5 | 25.0 | Normal weight |
| 25.0 | 30.0 | Overweight |
| 30.0 | 35.0 | Class I obesity |
| 35.0 | 40.0 | Class II obesity |
| 40.0 | 45.0 | Class III obesity |

Having said that, a person skilled in the art will appreciate that the BMI index definition for obesity may vary depending on the race or heritage of the subject. For example, for a subject from East Asian countries, lower BMI values may apply. This is due to negative health consequences due to obesity arising at lower BMI values than the standard BMI values defined for Caucasians, for example. For example, Japan defines obesity as any BMI value greater than 25 kg/m$^2$, while China utilises a BMI value of greater than 28 kg/m$^2$ to define obesity.

It has been shown that deletion of NLRP1 in mice leads to obesity and metabolic syndrome/disorders. Without being bound by theory, it is thought that loss of NLRP1 results in a decreased IL-18 production and lipolysis, as seen in subjects with an IL-18 deficiency. Interleukin-18 (IL-18) has been shown to be activated by Caspase-1 in inflammasome complexes and has been shown to have anti-obesity effects. As used herein, the term "metabolic syndrome" refers to a clustering of at least three of the five following medical conditions (giving rise to a total of 16 possible combinations, all of which are understood to fall under metabolic syndrome): abdominal (central) obesity (see also thin-outside-fat-inside, TOFI individuals), high blood pressure, high blood sugar, high serum triglycerides, and low high-density lipoprotein (HDL) levels. Metabolic syndrome is associated with the risk of developing cardiovascular disease and type 2 diabetes. The syndrome is thought to be caused by an underlying disorder of energy utilization and storage.

As used herein, the term "metabolic disorder" refers to the situation wherein abnormal chemical reactions in the body alter the normal metabolic process. The underlying cause for such a metabolic disorder can be, but is not limited to, underlying genetic mutations or single gene anomalies. Most of these mutations and/or anomalies are inherited in an autosomal recessive fashion. Some of the possible symptoms that can occur as a result of metabolic disorders are, but are not limited to, lethargy, weight loss, jaundice, and seizures. The symptoms expressed are understood to vary depending on the type of metabolic disorder present. The symptoms of metabolic disorders can be sorted into four categories: acute symptoms, late-onset acute symptoms, progressive general symptoms and permanent symptoms.

Examples of metabolic disorders are, but are not limited to, Phenylketonuria (PKU), Malignant PKU, Type 1 tyrosinemia, Type 2 tyrosinemia, Alkaptonuria, Homocystinuria, Hyperhomocysteinuria, Maple Syrup Urine disease, Propionic Acidemia, Multiple Carboxylase deficiency, Methylmalonic Acidemia, Hyperlipidemia and hypercholesterolemia, Fatty Acid Oxidation disorders, Glycogen Storage diseases, Galactosemia, Congenital Disorders of Glycosylation, Purine Overproduction, Lesch-Nyhan syndrome, Gaucher disease Types I and II, Tay-Sachs disease, Fabry disease, Hurler syndrome, Hunter syndrome, Sanfilippo syndrome, Maroteaux-Lamy syndrome, Morquio syndrome, Refsum disease, and Alanine-glyoxylate transaminase defect.

Herein described, but not forming part of the invention, is also a method of treating or preventing a skin tumour in a subject in need thereof. In one example, the method comprises administering a therapeutically effective amount of an inflammasome sensor NLRP 1 mutant into the subject in need thereof.

As used herein, the term "mutation" or "mutated" refers to a natural or artificial modification, or genetic alteration of the genome or part of a nucleic acid sequence of any biological organism, virus or extra-chromosomal genetic element. This mutation can be induced artificially using, but not limited to, chemicals and radiation, but can also occur spontaneously during nucleic acid replication in cell division. Mutations may or may not produce discernible changes in the observable characteristics (phenotype) of an organism. There are various types of mutations known, which can either be small-scale mutations or large-scale mutations. Examples of small-scale mutations are, but are not limited to, substitution mutations, silent mutations, missense mutations, nonsense mutations, frame-shift mutations, insertions, and deletions. Examples of large-scale mutations are, but are not limited to, amplifications, deletions, chromosomal translocations, interstitial deletions, chromosomal inversions and mutations that result in a loss of heterozygosity. Mutations can also be grouped by their effect on the function of the resulting product. These include, but are not limited to, loss-of-function (inactivating) mutations, gain-of-function (activating) mutations, dominant-negative (antimorphic) mutations, lethal mutations and back or reverse mutations. Point mutations, for example, also known as single base modification, are a type of mutation that causes a single nucleotide base substitution, insertion, or deletion of the genetic material, DNA or RNA. The term "frame-shift mutation" indicates the addition or deletion of a base pair, thereby resulting in a shift in the reading frame with which the DNA is read.

For example, silent mutations are mutations in DNA that do not significantly alter the phenotype of the organism in which they occur. Silent mutations can occur in non-coding regions (outside of genes or within introns), or they may occur within exons. When they occur within exons, they either do not result in a change to the amino acid sequence of a protein (also known as a synonymous substitution), or they result in the insertion of an alternative amino acid with similar properties to that of the original amino acid. In either case, there is no significant change in the resulting phenotype. The phrase silent mutation is often used interchangeably with the phrase synonymous mutation. However, synonymous mutations only occur within exons, and are not always silent mutations. Synonymous mutations are mutations that can affect transcription, splicing, mRNA transport, and translation, any of which could alter phenotype, rendering the synonymous mutation non-silent.

In one example, the NLRP1 mutant is caused by one or more of the following non-limiting examples of mutations: substitutions, insertions, deletions, frame-shift mutations, missense mutations, nonsense mutations, duplications, and repeat expansion mutations.

In one example, the NLRP1 mutant has at least one, at least two, at least three, or more mutations. In another example, the NLRP1 mutant has one, two, three, or more mutations. In yet another example, the mutations are located at PYD (pyrin domain) and/or LRR (leucine-rich repeats) domain. In a further example, the mutation located at PYD domain is a missense mutation. In one example, the mutation at the PYD domain results in at least one or two or all amino acid substitution, wherein the amino acid substitution is, but is not limited to, A54T (which is the substitution of an alanine at position 54 to threonine), M77T (which is the substitution of a methionine at position 77 to threonine), and A66V (which is the substitution of an alanine at position 66 to valine). In one example, the mutation at the PYD domain results in one mutation. In another example, the mutation at the PYD domain results in two mutations. In yet another example, the mutation at the PYD domain results in three mutations.

In one example, the amino acid substitution is A54T. In another example, the amino acid substitution is M77T. In a further example, the amino acid substitution is A66V. In one example, the mutation comprises two amino acid substitutions, wherein one amino acid substitution is A54T, and the other amino acid substitution is one of A66V or M77T. In another example, the mutation comprises two amino acid substitutions, wherein one amino acid substitution is A54T, and the other amino acid substitution is M77T. In yet another example, the mutation comprises two amino acid substitutions, wherein one amino acid substitution is A54T, and the other amino acid substitution is A66V. In a further example, the mutation comprises three amino acid substitutions, wherein the amino acid substitutions are A54T, A66V, and M77T.

The term "sequence identity" means that two nucleic acid or amino acid sequences are identical (i.e., on a nucleotide-by-nucleotide or residue-by-residue basis) over the comparison window. The term "percentage of sequence identity" is calculated by comparing two optimally aligned sequences over the window of comparison, determining the number of positions at which the identical nucleic acid base (e.g., A, T, C, G, U, or I) or residue occurs in both sequences to yield the number of matched positions, dividing the number of matched positions by the total number of positions in the comparison window (i.e., the window size), and multiplying the result by 100 to yield the percentage of sequence identity. The terms "substantial identity" as used herein denotes a characteristic of a polynucleotide or amino acid sequence, wherein the polynucleotide or amino acid comprises a sequence that has at least 85 percent sequence identity, preferably at least 90 to 95 percent sequence identity, more usually at least 99 percent sequence identity as compared to a reference sequence over a comparison window of at least 18 nucleotide (6 amino acid) positions, frequently over a window of at least 24-48 nucleotide (8-16 amino acid) positions, wherein the percentage of sequence identity is calculated by comparing the reference sequence to the sequence which may include deletions or additions which total 20 percent or less of the reference sequence over the comparison window. The reference sequence may be a subset of a larger sequence.

Thus, in one example, there is disclosed an isolated polypeptide having at least 70%, at least 75%, at least 80%, at least 85%, at least 90%, at least 91%, at least 92%, at least 93%, at least 94%, at least 95%, at least 96%, at least 97%, at least 98%, at least 99%, at least 99.5%, or 100% sequence identity to the polypeptide of mutant NLRP1, or fragment thereof. In another example, there is disclosed an isolated nucleic acid having at least 70%, at least 75%, at least 80%, at least 85%, at least 90%, at least 91%, at least 92%, at least 93%, at least 94%, at least 95%, at least 96%, at least 97%, at least 98%, at least 99%, at least 99.5%, or 100% sequence identity to the nucleic acid of mutant NLRP1, or fragment thereof.

Also herein described, but not forming part of the invention, is a vector comprising the nucleic acid described herein. The present application also describes a host cell comprising the vector or nucleic acid as disclosed herein. In one example, the host cell is, but is not limited to, a keratinocyte and a fibroblast (such as dermal fibroblast). In another example, there is disclosed a cell culture system comprising the host cell as described herein. In yet another example, the cell culture system has a three dimensional structure, for example, 3D organotypic cultures, cultures in scaffolding and the like

One of the methods disclosed herein is a method of determining the likelihood (or the predisposition) of a subject developing an inflammatory skin disorder and/or an autoimmune skin disorder selected from MSPC (multiple self-healing palmoplantar carcinoma) and FKLC (familial keratosis lichenoides chronica).

In one example of any of the methods disclosed herein, the method comprises detecting NLRP1 mutation in a sample obtained from the subject.

Herein described, but not forming part of the invention, the method comprises administering a therapeutically effective amount of an agent that reduces the effect of inflammasome-dependent cytokines. Examples of such agents are, but are not limited to, a small molecule, an antibody, a polypeptide, and a nucleic acid. In one example, the agent is an antibody. In another example, the antibody is a neutralising antibody.

Also herein described, but not forming part of the invention, is a method of treating a skin inflammation in a subject, comprising detecting NLRP1 mutation in a sample obtained from the subject. In one example, the method comprises determining whether the subject has NLRP1 mutation. In another example, the method comprises, when the subject is shown to have NLRP1 mutation, treating the skin inflammation in the subject by administering any one of the following: an inhibitor capable of inhibiting the activation of inflammasome sensor NLRP1 (Nucleotide-binding domain, leucine-rich repeat containing (NLR) family, pyrin domain containing protein 1 - NLPR1); an agent that prevents the secretion of stress responsive secreted factors, known pro-inflammatory cytokines, keratinocytes differentiation markers, inflammasome-dependent cytokines, and growth factors in the skin; an agent that reduces the effect of inflammasome-dependent cytokines and an inflammasome sensor NLRP1 mutant. In one example, the agents and inhibitors are each to be administered, or are each administered, in a therapeutically effective amount. In another example, the method comprises method of treating a skin inflammation in a subject, comprising detecting NLRP1 mutation in a sample obtained from the subject, determining whether the subject has NLRP1 mutation, and wherein when the subject has NLRP1 mutation, treating the skin inflammation in the subject by administering any one of selected from the group consisting of a therapeutically effective amount of an inhibitor capable of inhibiting the activation of inflammasome sensor NLRP1 (Nucleotide-binding domain, leucine-rich repeat containing (NLR) family, pyrin domain containing protein 1 - NLPR1); a therapeutically effective amount of an agent that prevents the secretion of stress responsive secreted factors, known pro-inflammatory cytokines, keratinocytes differentiation markers, inflammasome-dependent cytokines, and growth factors in the skin; a therapeutically effective amount of an agent that reduces the effect of inflammasome-dependent cytokines; and a therapeutically effective amount of an inflammasome sensor NLRP 1 mutant into the subject in need thereof.

Thus, in one example, the NLRP1 mutation is, but is not limited to, one or more substitutions, insertions, deletions, frameshifts mutations, missense mutations, nonsense mutations, duplications, and repeat expansions.

The NLRP1 mutation is located at PYD (pyrin domain) and/or LRR (leucine-rich repeats) domain. In one example, the mutation is located at the PYD domain. In a further example, the mutation is located at the LRR domain.

In one example, the NLRP1 mutation is detected by increased accumulation of oligomerised NLRP1 in the sample as compared to wild type (that is non-diseased or non-mutant) NLRP1. In another example, the accumulation of oligomerisation is detected by anti-ASC antibody. In yet another example, the method of detecting the NLRP1 mutation further comprises the step of detecting and determining the presence of NLRP3.

In a further example, the method disclosed herein further comprises determining the NLRP1 haplotype of the subject. As used herein, the term "haplotype" refers to a group of genes within an organism that was inherited together from a single parent. The word "haplotype" is a portmanteau of the words "haploid", which describes cells with only one set of chromosomes, and from the word "genotype", which refers to the genetic makeup of an organism. A haplotype can describe a pair of genes inherited together from one parent on one chromosome, or it can describe all of the genes on a chromosome that were inherited together from a single parent. This group of genes was inherited together because of genetic linkage, or the phenomenon by which genes that are close to each other on the same chromosome are often inherited together. The term "haplotype" can also refer to the inheritance of a cluster of single nucleotide polymorphisms (SNPs), which are variations at single positions in the DNA sequence among individuals.

Examination of haplotypes can assist in the identification of patterns of genetic variation that are associated with health and disease states. For instance, if a haplotype is associated with a certain disease, stretches of DNA near the SNP cluster can be analysed in an attempt to identify the gene or genes responsible for causing the disease.

In one example, analysis and identification of the presence or absence of the one or more mutations as disclosed herein is performed utilising, but not limited to, at least one of the following: exome sequencing; PCR, RT-PCR (reverse transcriptase), qPCR (quantitative PCR); Western Blot; gel electrophoresis, such as, but not limited to polyacrylamide gel electrophoresis (PAGE), sodium dodecyl sulfate polyacrylamide gel electrophoresis (SDS-PAGE), Blue-Native PAGE, and 2D-PAGE); enzyme-linked immunosorbent assay (ELISA); immunohistochemistry, such as, but not limited to, histology, immunofluorescence staining; in situ staining, and Disuccinimidyl suberate (DSS) crosslinker protocol. In one example, where in the event that the method disclosed herein is performed by RT-PCR, the primer, or primer pair, is selected from the primers as provided in Table 4.

The methods disclosed herein can be performed on samples obtained from a subject. The samples can be, but are not limited to, bodily fluids (for example, but not limited to, saliva, blood, tears, sweat, urine, seminal fluid, amniotic fluid, and the like), and skin (such as, but not limited to, skin obtained from a skin lesion). In one example, the skin is, but is not limited to epidermis, glabrous skin, plantar skin, epidermal appendages (such as hair follicles), keratinocytes, fibroblast (such as dermal fibroblast), and combinations thereof. In another example, the skin is keratinocytes and/or fibroblast.

Discussed herein are two overlapping Mendelian monogenic skin disorders: multiple self-healing palmoplantar carci- noma (MSPC; OMIM: 616964) and familial keratosis lichenoides chronica (FKLC). These two skin diseases are allelic and caused by distinct gain-of-function mutations in the inflammasome sensor protein, NLR family, pyrin domain containing protein 1 (NLRP1). It is shown that NLRP1 is the most prominently ex- pressed inflammasome sensor in human skin. Through functional analyses of the disease-causing mutations, it was shown that the mechanism of activation for NLRP1 that differs from other inflammasome sensors. Wild-type NLRP1 is kept as an inactive monomer by the combined action of the PYD (pyrin domain) and LRR domain. MSPC and FKLC mutations disrupt these two domains, respectively, leading to constitutive NLRP1 self-oligomerisation and inflammasome activation. Furthermore, spontaneous inflammasome activation and IL-1 secretion is shown to be present in, for example, patients' keratinocytes. Using *ex vivo* organotypic skin models, it was shown that inflammasome- dependent IL-1 cytokines can directly cause skin hyperplasia. These findings expand the clinical diversity of inflammasome disorders to non-fever skin diseases. Without being bound by theory, it is believed that the present application shows genetic evidence connecting inflammasome signalling to inflammatory skin disorders and skin cancer predisposition.

*Pedigrees and Clinical Features of MSPC and FKLC*

A Tunisian kindred (Fig. 2A, MSPC- TN-1) had been introduced that presented with an autosomal dominant skin order, MSPC (OMIM616964). Affected patients developed numerous ulcerative, hyperkeratotic nodular growths on plantar and palmar skin. Clinically, these lesions resemble rapidly growing benign proliferative epithelial skin lesions known as keratoacanthomas (KAs) but display histologic features of well-differentiated squamous cell carcinomas (SCCs) (Fig. 2B-2D). These lesions mostly regressed spontaneously after ~6 months. However, MSPC patients experienced increased susceptibility to malignant SCC (Fig. 2E; Table 1). In addition, 12 out of 15 patients (80%) developed conjunctival lesions characterized histologically by squamous dyskeratotic lobules (Fig. 2H). The putative MSPC locus in this kindred was found to map to chromosome 17p, suggesting a distinct genetic etiology from a similar proliferative skin disease, multiple self-healing squamous epithelioma (MSSE; OMIM132800) caused by mutations in TGFBR1.

Based on these symptoms, two additional MSPC kindreds were ascertained (Fig. 2A, 2F, 2G, 9C, and 9D), including one family that was originally diagnosed with corneal intraepithelial dyskeratosis (MSPC-FR-1; Fig. 2I). In addition to characteristic palmoplantar KAs, a subset of MSPC patients displayed irregular and thickened nails (Fig. 9G) and hyperkeratosis pilaris (Fig. 9H, 9K, 9J; Table 1).

It was further found that a fourth family shared several clinical features with MSPC (FKLC-EG-1) (Fig. 2A, 2L, 9L, 9M, 9Q, and 9R). In addition, the affected children presented with multiple discrete and semi-confluent lichenoid papules on the arms, legs, and lower trunks (Fig. 2J, 2K, 9N, and 9O). Based on these symptoms, the two affected siblings were diagnosed with FKLC. Keratosis lichenoides chronica (KLC), also known as Nekam's disease, is a mostly sporadic chronic skin inflammatory condition with no defined genetic etiology. Further clinical examination revealed that both parents also displayed noticeable, albeit less severe skin phenotypes, including plantar keratosis, follicular hyperkeratosis (Fig. 9L and 9M) and macular amyloidosis (Table 1), suggesting that the skin pathology in this kindred is caused by a semi-dominant allele.

To ascertain the causal mutation(s) in MSPC and FKLC, whole exome sequencing was performed on genomic DNA isolated from MSPC1-TN-1 IV:13 and FKLC-EG-1 V:3 and V:5. After removing annotated polymorphisms, a single heterozygous exonic variant (Chr. 17: 5,487,118 G>A, hgl9) in exon 1 of the NLRP1 gene was identified within the candidate genomic locus (Chr. 17: 1,541,109-12,964,181). Further Sanger sequencing confirmed that this mutation segregated with disease among 16 other MSPC-TN-1 family members (Fig. 2A, top). The resulting amino acid change A54T (Fig. 3A and 3B) affects a highly conserved residue and is, without being bound by theory, thought to be deleterious, according to PolyPhen2 and SIFT algorithms. Furthermore, this missense mutation is found near another, previously identified missense mutation, M77T which had been previously identified in MSPC-FR-1 (Fig. 3A and 3B). One member of MSPC-TN-1, III:5 was reported to be asymptomatic, despite being a carrier of the A54T mutation, suggesting the existence of possible modifier alleles. Sanger sequencing of NLRP1 exon 1 from the MSPC-RO-1 index patient III:3 revealed a third heterozygous mutation, A66V (Fig. 3A and 3B; Table 2), which segregates with disease in additional family members (Fig. 2A). No mutations were found in the TGFBR1 coding sequence in MSPC-RO-1 or other MSPC families (Table 3).

Thus, in one example, the NLRP1 mutation is located at PYD (pyrin domain). In another example, the mutation at the PYD domain comprises at least one or two or all amino acid substitution, wherein the amino acid substitution is, but is not limited to, A54T (which is the substitution of an alanine at position 54 to threonine), M77T (which is the substitution of a methionine at position 77 to threonine), and A66V (which is the substitution of an alanine at position 66 to valine). In one example, the mutation at the PYD domain comprises one mutation. In another example, the mutation at the PYD domain comprises two mutations. In yet another example, the mutation at the PYD domain comprises three mutations. In a further example, the mutation at the PYD domain is a missense mutation.

In one example, the amino acid substitution is A54T. In another example, the amino acid substitution is M77T. In a further example, the amino acid substitution is A66V. In one example, the mutation comprises two amino acid substitutions, wherein one amino acid substitution is A54T, and the other amino acid substitution is one of A66V or M77T. In another example, the mutation comprises two amino acid substitutions, wherein one amino acid substitution is A54T, and the other amino acid substitution is M77T. In yet another example, the mutation comprises two amino acid substitutions, wherein one amino acid substitution is A54T, and the other amino acid substitution is A66V. In a further example, the mutation comprises three amino acid substitutions, wherein the amino acid substitutions are A54T, A66V, and M77T.

Whole exome sequencing of FKLC-EG-1 V:3 and V:5 revealed that both affected children harboured a homozygous deletion within the NLRP1 locus that removed the fifth exon (Fig. 3A and 10A). Subsequent Sanger sequencing revealed that this deletion was present in the heterozygous state in both parents (Fig. 10B, left) and absent in unaffected siblings. The truncated, exon-5-deleted transcript was readily detectable in total mRNAs isolated from the proband V:3 (Fig. 10B, right). At the protein level, this leads to an internal in-frame deletion p.F787_R843del, which removes the first of the six leucine-rich repeats (LRR) and part of the preceding linker region (Fig. 3A and 10A). Taken together, these findings indicate that MSPC and FKLC are allelic disorders caused by distinct germline mutations in the same gene - NLRP1. Without being bound by theory, it is thought that the severe symptoms of the two affected children in FLKC-EG-1 are due to the homozygous inheritance of a semi-dominant NLRP1 mutant allele, which manifests as milder skin defects in the heterozygous parents.

*NLRP1 Is the Predominant Inflammasome Sensor in Human Skin*

The physiological function of NLRP1 remains less understood than that of other inflammasome sensors, such as, for example, NLRP3. Genome-wide association studies have implicated NLRP 1 haplotypes in psoriasis and vitiligo-related autoimmune diseases (OMIM: 606579), suggesting that NLRP1 might play a role in skin-specific immune response. To explore the potential functions of NLRP1 in the skin, the tissue expression profiles of NLRP1 in the Human Protein Atlas RNA-seq database were analysed. It was found that NLRP1 is widely expressed, in contrast to other NLRs such as NLRP3, AIM2, NLRC4, and MEFV, which have more restricted tissue distributions (Fig. 3C, D, and 10C). It was further found that the skin found to have the highest expression level of NLRP1 of all human tissues tested (FKPM (fragments per kilobase of exon per million fragments mapped) = 58.2 $\pm$ 17.4), whereas other known inflammasome sensors including NLRP3, AIM2, NLRC4, and MEFV were not expressed in skin (FKPM < 1) (Fig. 3C, 10C, and 10D).

Next, the transcript levels of NLRP1 and other inflammasome components in the three major cell types in the skin, namely, keratinocytes, melanocytes, and fibroblasts, were examined. NLRP1 was readily detectable by RT-PCR in keratinocytes and fibroblasts, but not in melanocytes. In contrast, none of the other known inflammasome NLRs could be detected in the skin cell types (Fig. 3D, left). Keratinocytes, but not fibroblasts, express all other inflammasome components, including CASP1, ASC (also known as PYCARD), IL-IB, and IL-18 (Fig. 3D, right, lane 3, and 10E). In fact, the levels of IL-1B and IL-18 in cultured primary keratinocytes exceed those in unstimulated peripheral blood mononuclear cells (PBMCs) (Fig. 3D and 10F), suggesting that keratinocytes are poised to initiate inflammasome signalling without prior priming.

Next, the expression of NLRP1 was verified in paraffin preserved primary human skin samples. Using RNAscope *in situ* staining, it was found that NLRP1 mRNA is expressed throughout the epidermis and in dermal fibroblasts in both glabrous skin and plantar skin (Fig. 3E, top, and 10G), in agreement with the RT-PCR results. This was corroborated by immunohistochemical staining using an anti-NLRP1 antibody (Fig. 3E, middle and bottom). In addition, NLRP1 is expressed in epidermal appendages such as hair follicles (Fig. 10H), consistent with the follicular hyperkeratosis seen in MSPC and FKLC patients (Fig. 9H, 9J, 9K, 9M, and 9O; Table 1).

*MSPC and FKLC Mutations Cause Increased Inflammasome Activation*

Many inflammasome-activating NLR proteins share a common domain structure that comprises of an N-terminal PYD followed by a NACHT domain, an evolutionarily conserved NTPase domain, and LRR domain. In general, NLR PYDs are thought to initiate inflammasome assembly, whereas the NACHT and LRR domains regulate self-association and/or ligand binding. Among all PYD-containing NLRs, human NLRP1 uniquely possesses a C-terminal caspase activation and recruitment domain (CARD) preceded by an auto-proteolytic "function-to- find" domain (FIIND; Fig. 3A), both of which contribute to inflammasome activation.

All of the three germline missense mutations in MSPC, A54T, A66V, and M77T are found within the N-terminal PYD (Fig. 3A and 3B). However, this domain is absent in NLRP1 homologs in non-primate mammalian species, with the possible exception of dogs and horses. Murine genomes contain three NLRP1 homologs, Nlrp1a, b, and c, but none encodes a PYD (Fig. 10A, top). In contrast, the amino acid residues (F787- R843) deleted in FKLC-EG-1 are conserved between men and rodents and encompass the first LRR in the LRR domain as well as part of the preceding linker region (Fig. 10A). Without being bound by theory, it is thought that the NLRP1 LRR domain participates in auto-inhibition, as an NLRP1 DLRR mutant has been shown to cause constitutive inflammasome activation *in vitro.* Similarly, the linker region has been implicated in the auto-inhibition of murine Nlrp1a, although its role in human NLRP1 is unclear.

Thus, in one example, the mutation at the PYD domain is a missense mutation. In another example, the mutation at the LRR domain is a deletion. In yet another example, the deletion is an in-frame deletion.

Missense mutations are is a point mutation present in the nucleic acid sequence. This alteration of one single nucleotide results in a codon binding which codes for a different amino acid, thus resulting in a change in the resulting peptide.

As used herein, the term "in-frame deletion" refers to any insertion or deletion in the nucleic acid sequence that is evenly divisible by three, thereby resulting in there being no change in the reading frame of the nucleic acid sequence.

In one example, the mutation is at the LRR domain results in the deletion of amino acid phenylalanine at position 787 to arginine at position 843 (that is F787 to R843 or F787_R843del). In other words, the mutation at the LRR domain results in the truncation of the amino acid sequence between amino acids F787 to R843, thereby retaining the amino acids F878 to R843.

To investigate the functional consequence of the MSPC and FKLC mutations, it was tested whether overexpressed NLRP1 mutants could nucleate oligomeric assemblies of the inflammasome adaptor protein, ASC. When 293T cells stably expressing ASC-GFP (293T-ASC-GFP) were transfected with plasmids encoding NLRP1 MSPC and FKLC mutants, a significant increase in the percentage of cells with ASC-GFP specks, relative to those expressing wild-type NLRP1 (Fig. 4A), were observed. In addition, a higher amount of ASC dimers and oligomers were observed in cells that overexpressed NLRP1 mutants after DSS crosslinking (Fig. 4B, lanes 3,4,5, and 8 versus lane 2). These *in vitro* results suggest that NLRP1 mutants in MSPC and FKLC can cause increased inflammasome assembly as compared to wild-type NLRP1.

Due to the high degree of natural polymorphisms in NLRP1, all minor alleles of NLRP1 with non-synonymous SNPs in the PYD were cloned. None of the 16 SNPs significantly altered the percentage of 293T-ASC-GFP cells with specks (Fig. 4C), supporting that the NLRP1 variants found in MSPC patients are true, rare, pathogenic mutants.

Next, NLRP1 mutants in MSPC were examined to see if these mutants could lead to increased processing of pro-IL-1b by caspase-1 . To this end, wild-type NLRP1, NLRP1 mutants, pro-caspase-1, and pro-IL-1b were overexpressed in HEK293T ASC-GFP cells to reconstitute a functional inflammasome complex. All three MSPC and FKLC mutants led to higher amount of pro-IL-1b cleavage than wild-type NLRP1 (Fig. 4D, lanes 3, 4, and 5 versus lane 2; lane 11 versus lane 10) at a level that was comparable to a known gain-of-function NLRP3 R262W mutant (Fig. 4D, lane 7 versus lane 6). Together, these results demonstrate that all NLRP1 mutants cause hyper-activation of the inflammasome *in vitro.* Furthermore, it was found that all four disease-associated NLRP1 mutants caused significant amounts of mature IL-lb secretion when expressed at physiological levels in immortalized keratinocytes (Fig. 4E, lanes 4-7 versus lane 3). This effect was found to depend on the adaptor protein ASC, as keratinocytes pre-treated with siRNAs against ASC showed a significant decrease in the level of secreted IL-1b following mutant NLRP1 overexpression (Fig. 4F, inset, lane 2 versus lanes 1 and 3).

Although the gain-of-function effect of the FKLC mutant, F787_R843del can be readily rationalized by the auto-inhibitory function of the LRR domain, it is shown that mutations in the PYD, which is widely thought to promote ASC assembly, can lead to a similar effect. To obtain additional evidence to confirm the gain-of-function effects of MSPC NLRP1 mutants, stable THP-1 cell lines that expressed doxycycline-inducible wild-type and mutant NLRP1 (A54T, M77T, or A66V; Fig. 11A and 11B) were generated. THP-1 macrophages expressing NLRP1 mutants experienced increased cell death and secretion of mature IL-lb (Fig. 11C and 11D), which could be blocked by caspase-1 inhibitor, Z-WEHD-FMK (Fig. 11E), or CRISPR/Cas9-mediated deletion of ASC (PYCARD) or caspase-1 (CASP1) (Fig. 11F). Together, these results further demonstrate that the NLRP1 MSPC mutations suffice to cause increased inflammasome activation.

*The Pyrin Domain of NLRP1 Functions as an Atypical, Auto-inhibitory Domain*

As compared to other inflammasome sensor proteins, NLRP1 uniquely consists of both a PYD and CARD (Fig. 3A). Sequence comparison of all PYDs encoded in the human genome revealed that NLRP1 PYD defines its own branch (Fig. 5A). However, all the amino acid residues that are mutated in MSPC, namely A54, A66, and M77 are conserved in other NLRs, including known inflammasome sensors NLRP3, AIM2, and MEFV (Fig. 5B).

To further characterize how A54T, A66V, and M77T lead to inflammasome hyperactivation, the possibility that these mutations directly enhance ASC oligomer assembly was tested. While both NLRP3 PYD and AIM2 PYD domain led to robust ASC speck formation when expressed as mCherry-fusion proteins in 293T ASC-GFP cells (Fig. 5C), neither wild-type NLRP1 PYD, nor the mutant PYDs caused ASC specks in the same setting (Fig.5C and 12A). Hence, it is considered unlikely NLRP 1 PYD is directly enhance ASC speck formation.

Based on these results, and without being bound by theory, it is thought that NLRP1 PYD plays a fundamentally different role from other PYDs, and that it maintains NLRP1 in an auto-inhibited, inactive state, instead of directly engaging in ASC oligomer formation. Thus, the ability of a series of N-terminally truncated NLRP1 mutants to initiate ASC-speck formation in 293T ASC- GFP cells (Fig. 5D, 5E, and 12B) were assayed. Similar to the MSPC mutant M77T, all N-terminal truncation mutants showed an ~3- to 6-fold increase in the percentage of cells with ASC-GFP specks (Fig. 5D and 5E) and led to increased IL-lb processing (Fig. 12D, lanes 3-9 versus lane 2). Notably, simply removing the PYD (amino acids 93 to 1474; Fig. 5D) caused increased ASC-GFP speck formation and increased IL-1b processing, despite very low expression levels of this mutant (Fig. 12D, lane 4). In addition, all NLRP1 truncation mutants lacking the PYD led to increased secretion of endogenous IL-lb in immortalized keratinocytes (Fig. 5F). In fact, the C-terminal auto-proteolytic fragment, (amino acids 1213 to 1474) was sufficient to nucleate ASC-GFP specks (Fig. 5G) and strongly activate inflammasome signalling (Fig. 5D to 5F).

Conversely, all C-terminal truncation mutants lacking the CARD failed to increase the number of ASC specks in 293T ASC-GFP cells (Fig. 5F and 12C), increase IL-lb processing (Fig. 12D, lanes 10 and 11 versus lanes 2 and 3, and 12E, lanes 8-11 versus lane 7), or activate IL-1b secretion in immortalised human keratinocytes, even in the presence of the M77T mutation (Fig. 5F). Taken together, these results demonstrate that NLRP1 PYD is an auto-inhibitory domain, while the C-terminal auto-proteolytic fragment encompassing the CARD is responsible for activating the inflammasome.

*NLRP1 Mutations in MSPC Disrupt PYD Domain Folding*

Functional characterization of NLRP1 PYD suggests that the three missense MSPC mutations likely cause inflammasome hyper-activation by disrupting PYD-dependent auto-inhibition. It had been previously shown that NLRP1 PYD forms a bundle of five a helices, in contrast to the canonical death domain superfamily fold consisting of six helices (Fig. 5H). Interestingly, MSPC mutations A54T, A66V, and M77T all affect residues that are located along the central helices (a4 and a5) within the hydrophobic core (Fig. 5H). Mutations of these residues are thus expected to destabilize the PYD structure. To test this, recombinant wild-type and mutant NLRP1 PYDs were purified. With the exception of M77T, all recombinant PYDs were soluble and isolated to >99% purity (Fig. 12F, inset) and eluted as a single monomer from sizeexclusion chromatography (Fig. 12F). Then the folding state of all recombinant NLRP1 PYDs were queried using in-solution 2D [$^{15}$N,$^{1}$H]-HSQC NMR. While wild-type NLRP1 PYD and the three PYD SNP variants S55L, E63K, and Q64L displayed well- dispersed chemical shift spectra that are characteristic of natively folded proteins (Fig. 5I, left, and 12G), both MSPC mutants, A54T and A66V, were found to populate the "random coil" region (Fig. 5I, middle and right), indicating that the A54T and A66V mutants are misfolded/unfolded in solution. These findings were corroborated by circular dichroism analysis of recombinant wild-type and mutant NLRP1 PYDs (Fig.12H). As further evidence for the requirement of these conserved residues to maintain the overall PYD architecture, it was found that "transplanting" the MSPC mutations to NLRP3 (Fig. 5H) abrogates PYD-dependent inflammasome activation (Fig. 12I and 12J).

*Mechanistic Basis for NLRP1 PYD- and LRR-Mediated Auto-inhibition*

Elucidating the structural basis for NLRP1 activation has been traditionally hampered by a lack of a bona fide ligand for NLRP1. It was reasoned that the activating mutants identified from MSPC and FKLC kindred offers a unique opportunity to probe the mechanistic basis for NLRP1 inflammasome activation.

To address this question, blue native PAGE (BN- PAGE) was used to directly visualize the native conformations of wild- type and patient-derived mutant NLRP1 proteins. Overexpressed wild-type NLRP1 migrated as a prominent band at a molecular size of ~150 kDa under native conditions (Fig. 6A, top and middle), which is similar in size to native rabbit IgG (Fig. 13A) and denatured, uncleaved NLRP1 on SDS-PAGE (Fig. 6A, bottom). Hence the majority of wild-type NLRP1 molecules are likely monomeric. In contrast, the abundance of this 150 kDa, monomeric species was drastically reduced for the MSPC and FKLC mutants. Instead, these mutants predominantly migrated as high molecular weight oligomers (Fig. 6A, lanes 2 to 5). These results suggest that the PYD and LRR domain are independently required to maintain NLRP1 as a monomer and prevent spontaneous self-oligomerisation.

Consistent with this model, the DPYD mutant (amino acids 93 to 1474) was found exclusively as a high molecular weight oligomer (Fig. 6B, lane 4, top), despite its low expression level (Fig. 6B, lane 4, middle). In contrast, deletion of the CARD from the NLRP1 M77T mutant drastically reduced oligomer formation (Fig. 6B, lane 3 versus lane 2). The C-terminal auto-proteolytic fragment (amino acids 1213 to 1474; molecular weight, ~30 kDa), on the other hand, exists exclusively as oligomers of ~1000 kDa (Fig. 6B, lane 5). Together with previous functional experiments (Fig. 5A to 5F), it is surmised that this fragment contains all the critical structural elements required for NLRP1 oligomerisation and inflammasome activation. Thus, blocking the generation of this fragment via auto-proteolytic cleavage is thought to inhibit inflammasome activation by NLRP1 mutants. Indeed, when the cleavage site mutation F1212A was introduced into MSPC and FKLC mutants (Fig. 6C, bottom), it severely diminished NLRP1 oligomerisation and caused the reappearance of the monomeric, 150 kDa band (Fig. 6C, lanes 4, 6, and 8 versus lanes 3, 5, and 7). Functionally, the F1212A mutation abrogated the ability of the MSPC and FKLC mutants to nucleate ASC-GFP specks in 293T cells (Fig. 6D) and to activate caspase-1-dependent IL-lb secretion in keratinocytes (Fig. 6E), confirming that auto-proteolytic cleavage is a prerequisite for NLRP1 oligomerisation and downstream inflammasome activation.

The role of the C-terminal cleavage fragment in NLRP1 MSPC mutants was further characterised using 2D-PAGE. NLRP1-expressing 293T lysates were fractionated by BN-PAGE, eluted from excised gel slices, and further separated by reducing SDS-PAGE. Full-length wildtype NLRP1 and its C-terminal fragment were both predominantly found in a lower molecular weight fraction corresponding to -150 kDa, similarly to native, tetrameric GAPDH (Fig. 13B, fraction 5, bottom). In contrast, NLRP1 M77T mutants displayed increased levels of the C-terminal fragment in high-molecular weight fractions (Fig. 13B, middle, fractions 1-4), suggesting that the increased oligomerisation of the NLRP1 mutants can indeed be attributed to the C-terminal auto-proteolytic fragment.

Taken together, these findings lead us to the model for NLRP1 function as shown in Fig. 6F. While wild-type NLRP1 exists as an inactive monomer in the absence of a ligand, binding to a putative ligand rapidly causes NLRP1 to self-oligomerise in a manner that is dependent on auto-proteolytic cleavage within the FIIND domain. When the PYD or the LRR domain is mutated as in MSPC and FKLC respectively, this self-inhibitory mechanism is lost. This triggers constitutive self-oligomerisation and spontaneous inflammasome activation. In addition, unlike other NLR PYDs, recombinant NLRP1 PYD does not initiate ASC filament formation in vitro (Fig. 13C), and a direct interaction between NLRP1 PYD and CARD (Fig. 13D) was not detected.

*NLRP1 Activation Causes Epidermal Inflammation and Hyperplasia via Paracrine IL-1 Signalling*

As primary human epidermal keratinocytes are the major skin cell type expressing all components of the NLRP1 inflammasome (Fig. 3), NLRP1 activation within keratinocytes is thought to contribute directly to MSPC and FKLC pathology. NLRP1 overexpression in immortalized keratinocytes (Fig. 7A and 14B) led to perinuclear ASC specks exclusively in cells expressing NLRP1 mutants (Fig. 14A) and a >100-fold increase in the levels of inflammasome-dependent cytokines, IL-1a, IL-lb, and IL-18 released into the culture media (Fig. 7B). Coincubation with a specific caspase 1 inhibitor, Z-WEVD-FMK abrogated this increase (Fig. 7B). In addition, keratinocytes expressing NLRP1 mutants demonstrated >5-fold increase in cell death relative to wild-type NLRP1, as measured by lactate dehydrogenase release (Fig. 14C).

Next, next-generation RNA sequencing was used to characterize the consequences of MSPC NLRP1 mutants on keratinocyte gene expression (Fig. 7C). Unsupervised clustering of differentially regulated transcripts clearly distinguished the mutant NLRP1 expressing keratinocytes from the controls, despite a very modest level of overexpression (1.2-fold by FKPM; Fig. 14D). Gene set enrichment analysis (GSEA) revealed a remarkable enrichment of IL-1/NFkB signalling pathway genes among the up-regulated transcripts (Fig. 7C, right), suggesting that IL-1/NFkB activation is a likely driver for the skin pathology seen in MSPC and FKLC.

A closer examination of the up-regulated transcripts revealed a significant overlap with a previous dataset on recombinant IL-1a-inducible genes in primary keratinocytes (Fig. 7D, inset). These include stress-responsive secreted factors, known pro-inflammatory cytokines, as well as keratinocyte differentiation markers (Fig. 7D, 14E, and 14F). These results suggest that the consequences of NLRP1 inflammasome activation extend well beyond the immediate pyroptotic cell death and IL-1 release from the initiating cells. The secreted IL-1 cytokines likely trigger the release of other inflammatory cytokines from surrounding cells, creating a paracrine, pro-inflammatory milieu. Consistent with this model, treatment with recombinant IL-1 and TNFa, as well as conditioned media from a MSPC primary keratinocyte culture led to increased transcript levels of keratinocyte stress markers S100A9 and PI3 (Fig. 14G) as well as IL-6, IL-8, and FGF7 (also known as keratinocyte growth factor, KGF) from dermal fibroblasts (Fig. 14H). Given that these cytokines and growth factors have roles in skin inflammatory and proliferative diseases, it as further thought that the recurrent, focal hyperkeratotic growth seen in both MSPC and FKLC patients is a direct result of IL-1-driven pro-inflammatory and pro-proliferative paracrine signalling. Indeed, skin organotypic cultures treated with IL-1a, IL-1b, and IL-18 showed a significant increase in epidermal thickness (Fig. 7E and 7F, left) and proliferative, Ki67-positive cells in the basal layer (Fig. 7E and 7F, right) and an expansion of supra- basal, keratin-10, and involucrin-positive differentiated keratinocytes (Fig. 7E). This was consistent with the up-regulation of keratinocyte differentiation markers shown in RNA-seq experiment (Fig. 6D and S6E) and the highly keratinized nature of the MSPC and FKLC lesions (Fig. IB-1D, 1F, 1L, and S1F). Furthermore, IL-1-treated *ex vivo* epidermis significantly up-regulated stress-markers S100A8/9 and S100A7/psoriasin (Fig. 141). This data provides further evidence that paracrine IL-1/NFkB signalling is sufficient to cause epidermal hyperplasia.

*In Vivo Evidence for Keratinocyte Inflammasome Activation in MSPC and FKLC Patients*

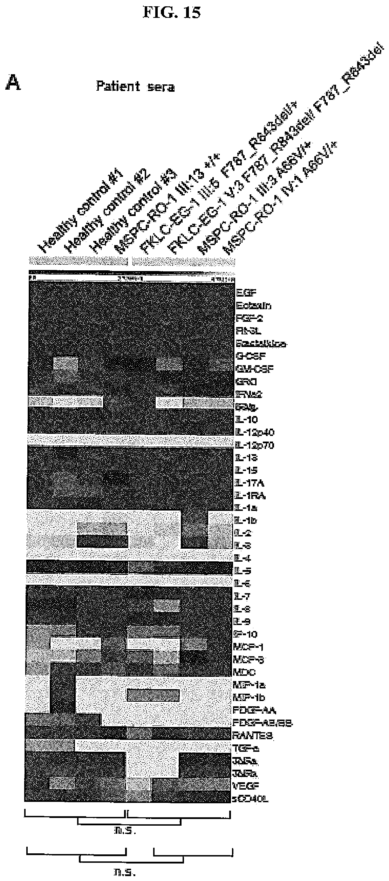

To study the endogenous NLRP1 inflammasome in patient cells, skin punch biopsies were obtained from two MSPC probands, MSPC-TN-1 IV:2 (NLRP1A54T/+) and MSPC-RO-1 III:3 (NLRP1A66V/+), and one FKLC proband FKLC-EG-1 V:3 (NLRP1F787_R843del/F787_R843del) and her mildly affected father FKLC-EG-1 III:5 (NLRP1F787_R843del/+). Five independent keratinocyte cultures were successfully isolated and designated NLRP1A54T/+ lesional, NLRP1A54T/+ non-lesional, NLRP1A66V/+ non- lesional, NLRP1F787_R843del/F787_R843del lesional, and NLRP1 NLRP1F787_R843del/+. The Luminex platform was used to compare the cytokine and chemokine profiles of patients' primary keratinocytes to those derived from unrelated healthy donors. The bona fide inflammasome-dependent cytokine IL-1b was the top cytokine up-regulated in keratinocyte cultures derived from MSPC and FKLC probands, followed by TNFa, IL-1RA, IL-1a, TGFa, and GM-CSF (Fig. 8A; p < 0.05, one-tailed t test). The significant increase in IL-lb, IL-18, and IL-1$\alpha$ secretion was confirmed using quantitative ELISA (Fig. 8B). These results demonstrate that MSPC and FKLC patients' keratinocytes are prone to spontaneous inflammasome activation and cytokine release in culture. It was also possible to observe endogenous ASC oligomers by western blot in DSS-crosslinked keratinocyte lysates from MSPC and FKLC patients, but not in keratinocytes derived from healthy donors (Fig. 8C, lanes 1 and 2 versus lanes 3-6). An increase in IL-lb levels in the sera (Fig. 8D and 15A) from MSPC and FKLC patients was not observed.

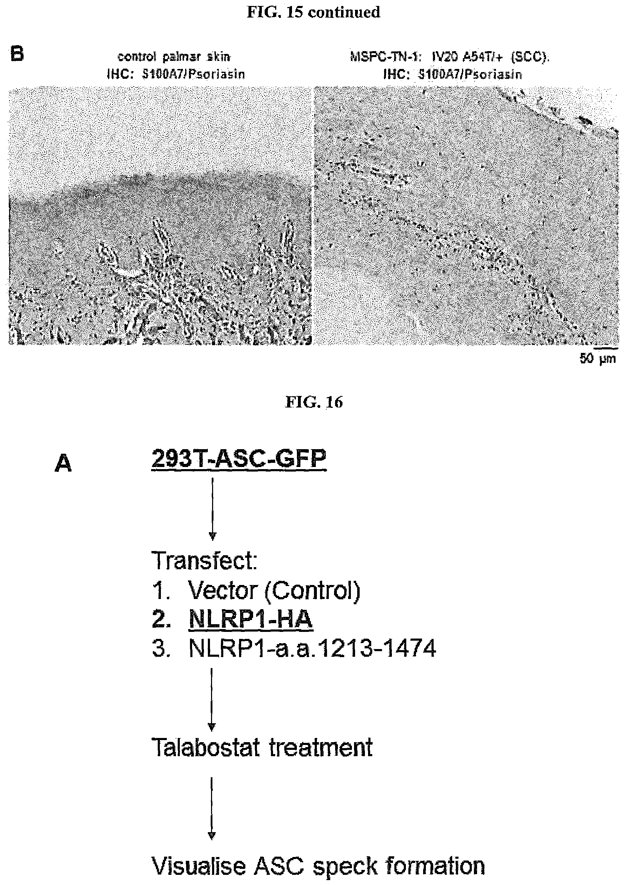

Interestingly, keratinocytes derived from the father of the FKLC-EG-1 proband also displayed ASC oligomer formation, despite the fact that there was no significant induction of IL-lb and IL-18 secretion. It was reasoned that the F787_R843del mutation caused only mild inflammasome activation in the heterozygous state, consistent with his subclinical symptoms. In this regard, it is worth noting that an activating gain-of-function mutation in the same region of murine Nlrpla displayed no phenotypes in the heterozygous state, but has been shown to cause severe auto-inflammation when bred to homozygosity. Finally, the up-regulation of inflammatory markers S100A8/9 and S100A7/psoriasin was validated in a primary squamo-proliferative skin lesion biopsy from the MPSC proband MSPC-TN-1 IV:20 (NLRP1A54T/+) (Fig. 8E and 15B), further demonstrating that MSPC lesions experience a chronic, unresolved inflammatory response as a result of NLRP1 inflammasome activation.

In summary, the genetic etiology of two skin disorders, MSPC and FKLC, has been resolved based on the information provided above. It is shown that MSPC patients experience recurrent keratoacanthomas (KA) in palmoplantar skin, as well as in conjunctival and corneal epithelia, and are highly susceptible to malignant squamous cell carcinoma. FKLC shares multiple clinical symptoms with MSPC but displays more severe symptoms such as generalized lichenoid papular lesions on the limbs and the trunk.

*Consequences of NLRP1 Gain- and Loss-of-Function Variants*

The results demonstrate that the two diseases are allelic and both result from germline mutations in the inflammasome sensor, NLRP1. While all MSPC cases are caused by heterozygous missense mutations in the PYD of NLRP1, FKLC arises from bi-allelic inheritance of an internal deletion within the highly conserved NLRP1 LRR domain. Functionally, all MSPC and FKLC mutations in NLRP1 are gain of function and lead to increased inflammasome activation.

Although the function of endogenous NLRP1 in immune defence is still unclear, it is of note that NLRP1 homologs in other mammals such as elephant, hedgehog, and dolphin have acquired a large number of null mutations, insomuch that NLRP1 can be considered to be a pseudo gene in these mammalian species. Importantly, at least five healthy individuals have been found to carry homozygous splice site mutations in NLRP1. The identification of these possible NLRP1-null healthy individuals suggests that the absence of NLRP1 is likely much less detrimental than gain-of-function mutations.

*Model of Pathogenesis for MSPC and FKLC*

Without being bound by theory, it is thought that a chronic, unresolved keratinocytedriven inflammation underlies the pathology in MSPC and FKLC (Fig. 8F). The characteristic recurrent focal lesions in both diseases are thought to originate from the aberrant activation of NLRP1 inflammasome in select keratinocytes. The ensuing rapid, localized release of IL-1 cytokines kick starts a paracrine signalling network involving neighbouring keratinocyte and fibroblasts, leading to the secretion of secondary inflammatory cytokines and growth factors, such as TNFa and KGF. This aberrant "wound-healing"-like response then leads to epidermal hyperplasia and keratoacanthoma formation. As the lesion grows and disrupts the skin barrier, infiltrating immune cells might act to control and restrict the lesion, leading to regression as is observed in MSPC patients. However, the continuous unresolved inflammation over many years is thought to facilitate the acquisition of additional oncogenic mutations and promote malignant transformation toward SCC development (Fig. 8F).

*A Mechanism of PYD-Mediated Inflammasome Regulation*

The performed biochemical analyses revealed unique biochemical properties of NLRP1. NLRP1 PYD unexpectedly functions as an auto-inhibitory domain, unlike the PYDs of other known inflammasome sensors. Thus, despite having both a PYD and a CARD, it is thought that NLRP1 should be functionally classified as an NOD-, LRR- and CARD-containing (NLRC) protein, rather than an NOD-, LRR-, and pyrin domain containing (NLRP) protein. NLRP1 PYD functions non-redundantly with the LRR domain to maintain NLRP1 in an inactive monomeric form. When either domain is mutated, as in the case of MSPC and FKLC, this auto-inhibitory mechanism is lost. This results in an increased propensity for NLRP1 to oligomerise. It was further demonstrated that the monomer-to-oligomer transition is an obligatory step for NLRP1 inflammasome activation and requires the auto- proteolytic cleavage of the FIIND domain. These findings define a mechanism for inflammasome regulation and provide a conceptual framework for future structural studies.

*Inflammasome in Skin Cancer Susceptibility and Skin Inflammatory Diseases*

Murine studies have implicated inflammasome components in epithelial hyperplasia. The data shown herein establishes a link between aberrant inflammasome activation and inherited inflammatory and proliferative skin disorders. Thus, inflammasome modulation can be and is used clinically to ameliorate chronic skin inflammatory diseases and decrease the risk for epithelial skin tumour.

NLRP1 is highly polymorphic in the general human population. It is thought that each NLRP1 haplotype is associated with a different propensity for activation, with the MSPC and FKLC mutants representing the extreme end of this spectrum. This line of thought concurs with previous genome-wide association studies (GWAS) linking NLRP1 SNPs to generalized vitiligo, Addison's disease, and congenital toxoplasmosis. Moreover, the phenotypes of FKLC overlap significantly with several common dermatologic diseases, such as keratosis pilaris and lichen planus, for which mechanistic insights are currently lacking.

The invention illustratively described herein may suitably be practiced in the absence of any element or elements, limitation or limitations, not specifically disclosed herein, Thus, for example, the terms "comprising", "including", "containing", etc. shall be read expansively and without limitation. Additionally, the terms and expressions employed herein have been used as terms of description and not of limitation, and there is no intention in the use of such terms and expressions of excluding any equivalents of the features shown and described or portions thereof, but it is recognized that various modifications are possible within the scope of the invention claimed. Thus, it should be understood that although the present invention has been specifically disclosed by preferred embodiments and optional features, modification and variation of the inventions embodied therein herein disclosed may be resorted to by those skilled in the art, and that such modifications and variations are considered to be within the scope of this invention.

As used in this application, the singular form "a," "an," and "the" include plural references unless the context clearly dictates otherwise. For example, the term "a genetic marker" includes a plurality of genetic markers, including mixtures and combinations thereof.

As used herein, the term "about", in the context of concentrations of components of the formulations, typically means +/- 5% of the stated value, more typically +/- 4% of the stated value, more typically +/- 3% of the stated value, more typically, +/- 2% of the stated value, even more typically +/- 1 % of the stated value, and even more typically +/- 0.5% of the stated value.

Throughout this disclosure, certain embodiments may be disclosed in a range format. It should be understood that the description in range format is merely for convenience and brevity and should not be construed as an inflexible limitation on the scope of the disclosed ranges. Accordingly, the description of a range should be considered to have specifically disclosed all the possible sub-ranges as well as individual numerical values within that range. For example, description of a range such as from 1 to 6 should be considered to have specifically disclosed sub-ranges such as from 1 to 3, from 1 to 4, from 1 to 5, from 2 to 4, from 2 to 6, from 3 to 6 etc., as well as individual numbers within that range, for example, 1, 2, 3, 4, 5, and 6. This applies regardless of the breadth of the range.

Certain embodiments may also be described broadly and generically herein. Each of the narrower species and sub-generic groupings falling within the generic disclosure also form part of the disclosure. This includes the generic description of the embodiments with a proviso or negative limitation removing any subject matter from the genus, regardless of whether or not the excised material is specifically recited herein.

The invention has been described broadly and generically herein. Each of the narrower species and sub-generic groupings falling within the generic disclosure also form part of the invention. This includes the generic description of the invention with a proviso or negative limitation removing any subject matter from the genus, regardless of whether or not the excised material is specifically recited herein.

Other embodiments are within the following claims and non- limiting examples. In addition, where features or aspects of the invention are described in terms of Markush groups, those skilled in the art will recognize that the invention is also thereby described in terms of any individual member or subgroup of members of the Markush group.

## EXPERIMENTAL SECTION

*Experimental model and subject details*

*MSPC and FKLC Subjects*

MSPC and FKLC families were identified and diagnosed by clinical geneticists and/or dermatologists. All genomic DNA samples were isolated from saliva using Oragene DNA collection kit (OG-500, DNAGenotek). Informed consent was obtained from all individual family members in accordance with local ethical review board requirements in the following institutions: Comité de Protection des Personnes Sud-Ouest et Outre-Mer II and Comité de Protection des Personnes Sud-Ouest et Outre-Mer II, Toulouse, France; National Institute for Infectious Diseases "Matei Bals"; "Carol Davila" University of Medicine and Pharmacy, Bucharest, Romania; Farhat Hached University Hospital, Sousse, Tunisia; Faculty of Medicine, Alexandria University, Egypt; Institute of Medical Biology, A* STAR, Singapore.

*Cell Culture*

All 293T and derivative cell lines were cultured in complete high-glucose DMEM media (Life Technologies) supplemented with 10% FBS. THP-1 cells were cultured in RPMI-1640 media supplemented with 10% FBS. THP-1 differentiation was induced by incubation with 400 ng/ml phorbol myristoyl acetate for 48 hours. Primary keratinocytes and fibroblasts from healthy human skin were obtained from de-identified surplus surgical waste with fully informed consent and obtained with full ethical clearance through the IMB Skin Cell Bank. Primary keratinocytes from MSPC and FKLC patients were isolated from fresh skin punch biopsies and grown on mouse 3T3 feeder cells. Immortalized N/TERT-1 keratinocytes were cultured in KSFM media (Life Technologies) supplemented with 300 mM $CaCl_2$.

*Method details*

*Exome Sequencing of MSPC Families*

For MSPC exome sequencing, 1 mg of purified genomic DNA was subjected to exome capture using Illumina TruSeq Exome Enrichment Kit (Illumina). Illumina HiSeq2500 high output mode was used for sequencing as 100 bp (basepair) paired-end runs at the UCLA Clinical Genomics Centre. Sequence reads were aligned to the human reference genome (Human GRCh37/hgl9 build) using Novoalign (v2.07). PCR duplicates were identified by Picard (v1.42) and GATK (Genome Analysis Toolkit) (v1.1) was used to re-align insertions and deletions (INDELs), recalibrate the quality scores, call, filter, recalibrate, and evaluate the variants. SNVs and INDELs across the sequenced protein-coding regions and flanking junctions were annotated using Variant Annotator X (VAX), a customized Ensembl Variant Effect Predictor (Yourshaw et al., 2014). An average coverage of ~85X was achieved across the exome.

*Exome Sequencing of FKLC-EG-1*

Following informed consent, genomic DNAs from two affected siblings were subjected to whole-exome capture using in-solution hybridization with the SureSelect All Exon 50 Mb Version 4.0 (Agilent) followed by massively parallel sequencing (Illumina HiSeq2000) with 100 bp paired-end reads. The resulting variant calls were filtered with the BCFtools utility (samtools.github.io/bcftools/), filtered for a minimum coverage (calls with fewer than four reads filtered) and hard filtered for quality (variants with quality <20 filtered from further analysis). This high-quality call set was then annotated with respect to the genes, and for consequences on protein sequence and/or splicing with the ANNOVAR tool (www.openbioinformatics.org/annovar/). Further annotation was done through further rounds of ANNOVAR annotation against dbSNP135 (www.ncbi.nlm.nih.gov/snp), population frequency estimates from the 1000 Genomes project (www.1000genomes.org/) and the National Institutes of Health Heart, Lung and Blood Institute Grand Opportunity Exome Sequencing Project (esp.gs.washington.edu/drupal/), and ~2000 control exomes that have been processed through the same bioinformatics analysis pipeline.

*RT-PCR, In Situ Staining, and Immunohistochemistry*

Human tissue RNA array was purchased from Clontech. cDNA synthesis was performed with iScript cDNA synthesis kit according to the manufacturer's instructions (Clontech) with 1 $\mu$g of purified RNA in 20 $\mu$l. RT-PCR was performed using 1 $\mu$l of 10x diluted cDNA using HotStart Taq polymerase (QIAGEN). Q-PCR was performed in triplicate wells using 1 $\mu$l of 10x diluted cDNA using SYBR Green Master Mix (ThermoFisher). The primers are listed in Table 4.
Control skin sections for immunohistochemistry were obtained from de-identified surplus surgical waste with informed consent. RNAscope (Advanced Cell Diagnostics) staining was performed using 'Brown Kit' using manufacturer-supplied controls (dapB and POLII) and a custom-synthesized probe against human NLRP1. Standard immunohistochemistry protocols were followed for NLRP1 staining using a rabbit polyclonal NLRP1 antibody (Adipogen, AL176). For antigen retrieval, slides were heated in sodium citrate buffer (10 mM sodium citrate, 0.05% Tween 20 [pH 6.0]) at 95°C for 20 minutes. Primary antibodies were diluted in antibody dilution buffer (10% normal goat serum, 1% BSA in PBS) overnight at 4°C. Signals were visualized with the Dako EnVision rabbit-HRP kit (Agilent).

*Plasmid Construction*

To construct plasmids used for transient transfections, PCR-amplified NLRP1 and NLRP3 cDNA were cloned into pCS2+ vector using standard restriction cloning with ClaI and XhoI sites. Doxycycline inducible NLRP1 and NLRP3 expression plasmids were constructed by InFusion cloning of the respective cDNA fragments into an AgeI-EcoRI linearized pTRIPZ backbone (Clontech). ASC-GFP lentiviral construct was assembled by Infusion Cloning (Clontech) in pCDH-puro (Systems Bio).

*Transient Transfection and Stable Lentiviral Overexpression*

All lentiviruses were produced in 293T cells by co-transfection of second generation helper plasmids, concentrated using Lenti-X (Clontech) and kept as frozen stock until use. 293T transient transfection experiments were performed using Lipofectamine 2000 (Life Technologies), while all transfection experiments in immortalized keratinocytes were carried out using FugeneHD (Promega) using a '3:1' ratio according to the supplied protocol. Pooled siRNAs against human PYCARD (ASC) were purchased from ThermoFisher and transfected with Lipofectamine RNAiMax (Life Technologies).

*Disuccinimidyl Suberate (DSS) Crosslinking*

All harvested cell pellets were kept at -80°C till use. For DSS crosslinking, cell pellets from a confluent well in 6-well plate were suspended in 200 $\mu$l of 1 mM DSS in PBS for 15 minutes at 37°C with constant mixing. Crosslinked pellets were centrifuged at 20,000 RCF for 5 minutes. Protein complexes were solubilized in 1x Laemmli SDS-PAGE buffer for 10 minutes at 95°C and centrifuged again at 20,000 RCF for 5 minutes. The supernatant was used for western blot analysis.

*SDS-PAGE, Western Blotting, and ELISA*

Protein lysate was quantified using the Bradford assay and 20 mg was used per well for SDS-PAGE. Proteins were transferred using TransBlot (Bio-rad) using the 'mixed molecular weight' setting (25V, 7 minutes). Western blotting was carried out with overnight incubation of primary antibodies diluted in 1xTBS with 1% Tween-20 and 3% non-fat milk at 4°C. All ELISA experiments were performed strictly according to the manufacturers' recommended protocols.

*Immunofluorescence*

293T-ASC-GFP cells were seeded at ~70% confluence on poly-lysine coated coverslips. N/TERT immortalized keratinocytes were seeded on uncoated coverslips. All cells were grown in growth media and allowed to adhere overnight. Coverslips were fixed with 4% paraformaldehyde in 1xPBS and permeablized with 0.5% Triton-X in 1x PBS. All primary antibodies were diluted in PBS with 1% BSA and 0.05% Triton-X. Primary antibody incubation was performed at 4°C overnight with gentle mixing. Secondary antibody incubation was performed for 1 to 2 hours at room temperature. Coverslips were mounted with DAPI-containing Prolong Gold mounting media (ThermoFisher).

*Blue-Native PAGE and 2D-PAGE*

Blue-Native PAGE was performed using the Novex® NativePAGE Bis-Tris gel system (ThermoFisher) according to the manufacturers' instructions with minor modifications. Briefly, 293Ts cells were transfected with various NLRP1 expression plasmids at a ratio of 1 μg plasmid per well in a standard 6-well plate using Lipofectamine 2000 reagent (ThermoFisher). Cells were harvested 48 hours post-transfection and lysed in Sample Prep buffer containing 1% digitonin, clarified by centrifugation at 20,000 RCF for 10 minutes and supplemented with Coomassie G-250 to a final concentration of 0.25%. 10 μl of protein lysate per well was using for BN-PAGE. Electrophoresis was performed with the 'dark blue' cathode buffer for ~1 hr at 150 V constant voltage, followed by the 'light blue' buffer at 250V for ~1.5 hours. The proteins were transferred onto a PVDF membrane using a Trans-Blot Turbo system at 25 V for 10 minutes and detected by standard western blot procedures without the recommended acetic acid fixing step. For 2D PAGE, the entire BN-PAGE gel lane was excised and cut into 8 slices. Each slice was eluted in an elution buffer containing 50 mM Tris-HCl, 150 mM NaCl and 0.1% SDS overnight at room temperature with constant mixing. The supernatant was concentrated the next day using an Amicon Ultra 0.5 mL filter (MWCO = 30 kDa) (Merck Millipore) to 40 ml and supplemented with 10 ml 1x Laemmli buffer.

*Luminex Multiplex Cytokine/Chemokine Array*

Luminex multiplex cytokine and chemokine array was performed according the manufacturer's protocol, using the human cytokine/ chemokine panel I (HCYTMAG-60K-PX41 Merck Millipore). Cytokine levels from different samples were analysed using 'hierarchical clustering' in Multiple Experiment Viewer (www.tm4.org).

*Recombinant Protein Purification and NMR Structural Analysis*

Recombinant GB1-PYD WT and its mutants were expressed in E. coli BL21. All recombinant proteins were purified from the soluble fraction to homogeneity by Superdex 75 gel-filtration chromatography (GE healthcare) with an elution buffer containing 50 mM $Na_2HPO_4$, 50 mM NaCl, 20 mM DTT at pH 6.5. For NMR studies, U-$^{15}$N-labeled proteins were produced by using standard M9 minimal media prepared with $^{15}NH_4Cl$. The NMR samples were prepared with protein concentrations between 100 and 200 mM in buffer containing 95%/5% $H_2O/D_2O$, 50 mM $Na_2HPO_4$, 50 mM NaCl, 20 mM DTT, at pH 6.5. Due to the low solubility of the cleaved protein, the recombinant PYDs were maintained as GB1 fusion proteins for the NMR experiments. 2D [$^{15}$N,$^1$H]-HSQC spectra were recorded at 25°C in Bruker 600 and 700 MHz spectrometers equipped with room-temperature and cryogenic triple-resonance probes.

*RNA-Seq*

Total RNA samples were processed using the Illumina TruSeq stranded mRNA kit. Prepared libraries were quantified using KAPA qPCR and Agilent Bioanalyzer, and sequenced on the Illumina HiSeq-2000. The reads were processed and mapped using TopHat and CuffLink in the Galaxy suite. Differential gene expression was obtained using CuffDiff.

*Phylogenetic Analysis of PYDs*

Amino acid sequences of PYDs in the human genomes were downloaded from Prosite and aligned using the Muscle algorithm. Sequence similarity was computed using ClustalW2-Phylogency using the Neighbour-joining clustering method and visualized using Tree-Of-Life (itol.embl.de/).

*3D Skin Organotypic Co-cultures*

Briefly, keeping all solutions on ice, each gel was formed by using 8 parts collagen (rat tail collagen type I (BD biosciences) suspended in acetic acid) mixed with 1 part 10x DMEM and I part FBS containing $1 \times 10^5$ normal human fibroblasts (NHF). The collagen-NHF mix was then loaded into a cell culture insert (BD Biosciences) and allowed to solidify at 37°C before adding NHF media to the culture. After three days $1 \times 10^6$ normal human primary keratinocytes (NHK) were seeded on-top of the collagen gel, in NHK medium. The next day the cultures were raised to the air-medium interface. After airlift, the gels were then treated either with a mixture of cytokines, IL-1α (R & D Systems), IL-1b (R & D Systems), IL-18 (MBL) to give a final concentration of 10 ng/ml each, or left untreated as a control. The additives were then replaced with medium change every 2-3 days. Organotypic co-cultures were harvested after 7 days and embedded in OCT for cryo-sectioning.

*Immunofluorescence Staining of Organotypic Cultures*

Frozen sections of 3D organotypic cultures, were exposed to 1:1 methanol:acetone fixation before incubation with 10% goat serum for 20 minutes, to block non-specific binding. Primary antibodies were then added overnight at 4°C. Primary antibodies include rabbit ki67 (ab15580, Abcam, 1:50), mouse keratin 10 (clone DEC10, Leica, 1:50), involucrin (clone SY5, Abeam, 1:100), S100A7 (clone MAC 387, Dako, 1:50) and S100A9/8 (Psoriasin, NovousBio, 1:50). Secondary antibodies (AlexaFluor 594 goat anti-rabbit or AlexaFluor 488 goat antimouse) were then added for 20 minutes at room temperature. Nuclei were counter stained with DAPI. Coverslips were mounted onto glass slides using Hydromount mounting media (National Diagnostics) before microscopic visualization.

*Circular Dichroism Spectroscopy of NLRP1 PYD Domain*

Circular dichroism (CD) measurements were performed on a Chirascan qCD series spectropolarimeter. Samples were diluted in 50 mM phosphate buffer, 50 mM NaCl, 20 mM DTT (pH 7.4) to a final protein concentration of 0.1 mg/mL. The protein concentration was determined by measuring absorbance at 280 nm. Native CD spectra were recorded over the wavelength range of 200-260 nm in a cuvette with a path length of 0.1 cm at the temperature of 293K. Samples were pre-equilibrated for 15 minutes prior to running the experiments. The 'blank' signal obtained for the buffer alone was subtracted from the individual protein spectra. Thermal denaturation CD profiles were acquired by monitoring the ellipticity at 222 nm in the range from 293K to 365K using a water bath controlled by a Peltier device. The signal was recorded at 0.1K intervals and 1.5 s of response time. The CD signal was converted to mean residue molar ellipticity as follows:

$$[\theta] = 10^6 \theta / [C] N l$$

where [C] is the protein concentration, N is the protein residue number and 1 is the path-length (in cm). Calculated mean residue molar ellipticity is given in deg cm$^2$ dmol$^{-1}$. The CD thermal unfolding data were analysed using least-squares minimization for the fitting two-state equilibrium unfolding model.

*Fluorescence Anisotropy ofASC Filamentation*

A fluorescence polarization-based assay was used to measure the reaction kinetics of ASC filament formation. Dylight Fluor 488 was conjugated irreversibly to a single cysteine residue of ASC in an overnight reaction and under denaturing conditions. Chromophore- labeled ASC-PYD was further purified by dialysis and size exclusion chromatography to remove unreacted free dye. ASC filament reconstitution was initiated by rapid mixing to physiological pH conditions. Filament formation is accompanied by a change in the rotational correlation time of the fluorophore covalently attached to monomeric ASC-PYD, which results in changed fluorescence polarization. The fluorescence polarization time course was measured on a Synergy H1 Hybrid microplate reader (Biotek). Data were acquired in 10 second intervals for a total time of 120 minutes.

*Quantification and statistical analysis*

A custom Image! workflow was used for the percentage calculation of ASC-GFP speck-containing cells. Briefly, the total cell number per image was counted in ImageJ using binary intensity thresholding of the DAPI images, a 'watershed' filter followed by the automatic 'particle count' algorithm, with a minimum cut-off radius of 50 pixels. ASC-GFP specks were counted in the same way using the GFP images, except no watershed filter was applied and the minimum radius was set at 10 pixels. For each sample, three fields were chosen at random and at least 100 cells were scored. All bar graphs were calculated based on three independent replicates, except experiments involving primary patient keratinocytes and sera (Fig. 8 and 15), where error bars were calculated from three technical replicates.
The following experiments were performed with the experimented blinded to the sample identity: Luminex cytokine/chemokine array (Fig. 8A), all image analysis for 293T-ASC-GFP assays, ELISA for primary keratinocytes and patient sera (Fig. 8B to 8D). Other experiments were not performed in a blinded fashion.

The p values from all pairwise comparisons were calculated using two-tailed Student's t test, except the Luminex array data, where a one-tailed t test was used (Fig. 8A). Statistical tests were performed in GraphPad Prism 6 or Microsoft Excel using the TTEST function. Bar graphs were plotted as mean $\pm$ SDM and statistical significance was denoted as follows: *, $p < 0.05$; **, $p < 0.01$; ***, $p < 0.001$; ****, $p < 0.0001$.

*Data and software availability*

*Data Resources*

The accession number for the RNA-seq data reported in this paper is GEO: GSE85791.

# TABLES

## Table 1 – clinical symptoms of MSPC and FKLC patients

| Pedigree Number | Mode of inheri-tance | NLRP1 Mutation | | Ge nde r | Age of On-set | No. of palmo-plantar lesions | Affected Epithelial Tissues | | | | Other symptoms | | | Second-ary tumours | Evolutio n Period | Treat-ment |
|---|---|---|---|---|---|---|---|---|---|---|---|---|---|---|---|---|
| | | | | | | | Plant ar | Pal-mar | Con-junc-tival | Others | Hyper-keratosis pilaris | Subungual lesions/Nail thickening | Others | | | |
| MSPC-TN-1 III:10 | AD | p. Ala54 Thr | Heter ozygo us | M | 25 | 10 | + | + | - | - | - | - | | Head and neck | Months to years | Surgery/ Acitretin |
| MSPC-TN-1 III:11 | AD | p. Ala54 Thr | Heter ozygo us | M | 20 | 10 | + | + | + | - | - | + | | Lung | Weeks to months | Acitretin |
| MSPC-TN-1 III:15 | AD | p. Ala54 Thr | Heter ozygo us | F | 10 | 10 | + | + | + | - | - | - | | - | Weeks | - |
| MSPC-TN-1 IV:2 | AD | p. Ala54 Thr | Heter ozygo us | M | 5 | 15 | + | + /- | + | Lower lip | - | - | | Lip SCC. Bilateral heel SCC | Month to years | Surgery/ Acitretin |
| MSPC-TN-1 IV:5 | AD | p. Ala54 Thr | Heter ozygo us | M | 15 | 10 | + | + | + | Base of nose | - | + | | Nose | Months to years | Surgery/ Acitretin |
| MSPC-TN-1 IV:8 | AD | p. Ala54 Thr | Heter ozygo us | F | 3 | 10 | + | + /- | - | - | - | - | | - | Months | - |
| MSPC-TN-1 IV:9 | AD | p. Ala54 Thr | Heter ozygo us | M | 7 | 10 | + | + | + | - | - | - | | - | Weeks | Acitretin |
| MSPC-TN-1 IV:11 | AD | p. Ala54 Thr | Heter ozygo us | M | 3 | 15 | + | + | - | - | - | - | | - | Weeks | Acitretin |
| MSPC-TN-1 IV:12 | AD | p. Ala54 Thr | Heter ozygo us | F | 1 | 15 | + | + | + | - | - | - | | - | Weeks | Surgery/Ac itretin |
| MSPC-TN-1 IV:13 | AD | p. Ala54 Thr | Heter ozygo us | M | 5 | 15 | + | + | + (bi-lateral) | - | + | - | | - | Weeks | Acitretin |
| MSPC-TN-1 IV:14 | AD | p. Ala54 Thr | Heter ozygo us | M | 5 | 10 | + | + | + | - | - | - | | - | Months to years | Acitretin |

EP 3 515 470 B1

| Pedigree Number | Mode of inheri-tance | NLRP1 Mutation | | Gender | Age of On-set | No. of palmo-plantar lesions | Affected Epithelial Tissues | | | | Other symptoms | | | Second-ary tumours | Evolution Period | Treat-ment |
|---|---|---|---|---|---|---|---|---|---|---|---|---|---|---|---|---|
| | | | | | | | Plantar | Pal-mar | Con-junc-tival | Others | Hyper-keratosis pilaris | Subungual lesions/Nail thickening | Others | | | |
| MSPC-TN-1 IV:15 | AD | p. Ala54 Thr | Heter ozygo us | M | 5 | 10 | + | + | + | - | - | - | | - | Weeks to years | Acitretin |
| MSPC-TN-1 IV:16 | AD | p. Ala54 Thr | Heter ozygo us | M | 5 | 15 | + | + | + | - | - | - | | - | Weeks | Acitretin |
| MSPC-TN-1 IV:20 | AD | p. Ala54 Thr | Heter ozygo us | M | 4 | 10 | + | + | + | - | + | + | | - | Years | Surgery/Ac itretin |
| MSPC-TN-1 V:4 | AD | p. Ala54 Thr | Heter ozygo us | M | 15 | 10 | + | + | + | - | - | - | | - | Months | Acitretin |
| MSPC-FR-1 II:2 | AD | p.Met 77Thr | Heter ozygo us | F | 10 | 20 | + | + | + | Cornea | - | + | Maxillary decalcifi-cation, alveolysis and tooth loss , loss of conscious-ness associated with local anesthetic | - | Years | Acitretin |
| MSPC-FR-1 III:2 | AD | p.Met 77Thr | Heter ozygo us | M | 2 | 15 | + | + | + | Cornea, Larynx | + | + | Bone Decalcifi-cation, alveolysis and tooth loss | - | Years | Acitretin (stopped because Acitretin-associated dry eye syndrome has caused keratoprost hesis expulsion) |
| MSPC-RO-1 I:1 | AD | p.Ala 66Val | Heter ozygo | M | 4 | >15 | + | + | - | - | - | - | | - | Months to years | Surgery |

| Pedigree Number | Mode of inheri-tance | NLRP1 Mutation | | Gender | Age of On-set | No. of palmo-plantar lesions | Affected Epithelial Tissues | | | | Other symptoms | | | Second-ary tumours | Evolution Period | Treat-ment |
|---|---|---|---|---|---|---|---|---|---|---|---|---|---|---|---|---|
| | | | | | | | Plantar | Pal-mar | Con-junc-tival | Others | Hyper-keratosis pilaris | Subungual lesions/Nail thickening | Others | | | |
| | | | us | | | | | | | | | | | | | |
| MSPC-RO-1 II:1 | AD | p.Ala 66Val | Heter ozygo us | M | 7 | 15 | + | + | - | - | - | - | | - | Months to years | Surgery |
| MSPC-RO-1 II:2 | AD | p.Ala 66Val | Heter ozygo us | M | 5 | 15 | + | + | - | - | - | - | | - | Weeks to years | Surgery |
| MSPC-RO-1 II:5 | AD | p.Ala 66Val | Heter ozygo us | F | 4 | 10 | + | + | - | lower lip | - | - | | - | Months to years | Surgery |
| MSPC-RO-1 III:2 | AD | p.Ala 66Val | Heter ozygo us | M | 18 | - | - | - | - | - | - | - | palmar hyperkera-tosis | - | | |
| MSPC-RO1- III:3 | AD | p.Ala 66Val | Heter ozygo us | M | 5 | 15 | + | + | - | lower lip, right forear m | - | - | | - | Weeks to years | Surgery |
| MSPC-RO1- IV:1 | AD | p.Ala 66Val | Heter ozygo us | F | - | - | - | - | - | - | - | - | Eczema | - | - | - |
| NEKAM-EG-1 III-5 | | p. F787 R84 3del | Heter ozygo us | M | - | - | - | - | - | - | + | - | keratosis pilaris on the trunk and on soles. | - | - | - |
| NEKAM-EG-1 IV:1 | | p. F787 R84 3del | Heter ozygo us | F | - | - | - | - | - | - | + | - | macular amyloidosis | - | - | - |
| NEKAM-EG-1 V:3 | | p. F787 R84 3del | Homo zygou s | F | 8m | Not assess ed | + | + | + | Middle ear (choles teatom a), conjun ctiva | + | + | corneal ulcers & vascularisati on, corneal limbal stem cell deficiency, | Conjunct-tival squamou s cell carcinom a | Months | Acitretin, surgery for eye and ear lesions |

| Pedigree Number | Mode of inheri-tance | NLRP1 Mutation | | Ge nde r | Age of On-set | No. of palmo-plantar lesions | Affected Epithelial Tissues | | | | Other symptoms | | | Second-ary tumours | Evolutio n Period | Treat-ment |
|---|---|---|---|---|---|---|---|---|---|---|---|---|---|---|---|---|
| | | | | | | | Plant ar | Pal-mar | Con-junc-tival | Others | Hyper-keratosis pilaris | Subungual lesions/Nail thickening | Others | | | |
| | | | | | | | | | | | | | cholestea-toma | | | |
| NEKAM-EG-1 V:5 (decease d) | | p-F787_R84 3del | Homo zygou s | M | 10m | - | - | - | - | - | + | - | lichenoid papules and plaques on arms, legs and the trunk | - | - | - |
| NEKAK-EG 1: III:4 (decease d) | | Patie nt DNA not availa ble | - | M | - | - | - | - | - | - | + | - | keratosis pilaris | - | - | - |

**Table 2 - NLRP1 exonic variants for MSPC-RO-1 III:3**

| NLRP1 exonic variants of MSPC-RO-1 proband III:3 | | | |
|---|---|---|---|
| **NLRP1 exon** | **Nucleotide Variant** | **Amino Acid Change** | **ExAC Allele Frequency** |
| 1 | **17: 5481087 T/C** | **A66V** | **0** |
| 2 | None | None | - |
| 3 | 17:5485367 A / T (rs12150220) | L155H homozygous | 0.3749 |
| 4 | 17:5462654 G / A (rs2001363) | None | 0.04865 |
| | 17:5461847 G / A (rs61753136) | None | 0.05036 |
| | 17:5461671 G / C (rs52795654) | T782S | 0.05253 |
| 5 | None | None | - |
| 6 | 17:5445243 G / A (rs11657747) | T878M | 0.044 |
| 7 | None | None | - |
| 8 | 17:5440248 T / C (rs11657249) | None | 0.04298 |
| | 17:5440221 T / C (rs7215868) | None | 0.04278 |
| | 17:5440197 T / C (rs7215856) | None | 0.07946 |
| 9 | 17:5437285 G / A (rs34733791) | T995I | 0.04762 |
| 10 | None | None | - |
| 11 | 17:5436263 C / T (rs2301582) | V1059M | 0.3248 |
| 12 | 17:5433841 A / G (rs56872041) | None | 0.04797 |
| 13 | 17:5425077 T / C (rs11651270) | M1184V homozygous | 0.4549 |
| | 17:5424991 G / A (rs11653580) | None | 0.04849 |
| | 17:5424906 C / G (rs11653832) | V1241L | 0.04863 |
| 14 | None | None | - |
| 15 | None | None | - |
| 16 | None | None | - |
| 17 | None | None | - |
| * Positions are in reference to GRCh37/hg19 | | | |

**Table 3 - Sanger sequencing results for TGFBR1 exons for MSPC-RO-1 patients**

| TGFBR1 exon | III:3 Affected, NLRP1 A66V/+ | II:5 Affected, NLRP1 A66V/+ | Unrelated control, Unaffected, NLRP1 +/+ |
|---|---|---|---|
| 1 | WT* | WT | WT |
| 2 | WT | WT | WT |
| 3 | WT | WT | WT |
| 4 | WT | WT | WT |
| 5 | WT | WT | WT |
| 6 | WT | WT | WT |
| 7 | WT | WT | c.1255+24G>A |
| 8 | WT | WT | c.1386+94_1386+90delTCTTT |
| 9 | WT | WT | c.*69A>G |
| * Sequence verified using TA-cloning. One out of four clones harboured an in-frame deletion in the polymorphic alanine repeat from A18 to A26, resulting in the removal of 1 alanine. | | | |

**Table 4 - Genotyping and RT-PCR primers**

| Primer pair | Sequence | Related Figure/ Table |
|---|---|---|
| FKLC-EG-1 Genotyping primer pair | AGACCCTCTTGTTATCCTGGGAA; GTGTCTTTTATCACTCCTCCTCACTG. | Fig. 10B |
| RT-PCR primers for NLRP 1 exon 5 cDNA | ACAGACATGGAGCTCTTAGTGT; GGGTCTGGTTGGCTCTCAG | Fig. 10B |
| RT-PCR primers for NLRP 1 | CCGCCTGGCCTGTTACTT; CCTGGCTTGGAGACTCATGG | Fig. 3D, Fig. 10D,10F |
| RT-PCR primers for NLRP3 | CATGCTGCCTGTTCTCATGG; CCCCAACCACAATCTCCGAATG | Fig. 3D, Fig. 10D,10F |
| RT-PCR primers for MEFV | CGCCCTGGAACACAAGAAGA; GCTGCTCCTCCCCTGATTTT | Fig. 3D, Fig. 10D,10F |
| RT-PCR primers for AIM2 | AACTTTGGGATCAGCCTCCTG; TGAAGCCGTCCAGAAGTGTC | Fig. 3D, Fig. 10D,10F |
| RT-PCR primers for NLRC4 | AAAAGACTTTGTCCATTCAAGTCCT; CCCTGCTGACTGAGAGAAC | Fig. 3D, Fig. 10D,10F |
| RT-PCR primers for CASP1 | CTGCCTGAGGAGCTGGAAAG; CAGTTACCTGGCAGGGACG | Fig. 3D, Fig. 10D,10F |
| RT-PCR primers for ASC | TCTACCTGGAGACCTACGGC; TCCAGAGCCCTGGTGC | Fig. 3D, Fig. 10D,10F |
| RT-PCR primers for IL1B | TGGAAGGAGCACTTCATCTGT; TCTTCAGCCAATCTTCATTGCTC | Fig. 3D, Fig. 10D,10F |
| RT-PCR primers for IL18 | ATCGCTTCCTCTCGCAACAA; GTCCGGGGTGCATTATCTCT | Fig. 3D, Fig. 10D,10F |

(continued)

| Primer pair | Sequence | Related Figure/ Table |
|---|---|---|
| RT-PCR primers for GAPDH | CGACAGTCAGCCGCATCTT; CCCCATGGTGTCTGAGCG | Fig. 3D, Fig. 10D,10F |
| RT-PCR primers KRT14 | CCTCCTCCCAGTTCTCCTCT; ATGACCTTGGTGCGGATTT | Fig. 3D, Fig. 10D,10F |
| Primer pair to amplify TGFBR1 Exon-1 | CGGCGGCGGGGAGGCGG; CAGGAGCGAGCCAGAGGCC | Table 3 |
| Primer pair to amplify TGFBR1 Exon-2 | TGCTACATTTCCTTGGGCTTCC; GTCACTTCTTGCCTCTAAACGG | Table 3 |
| Primer pair to amplify TGFBR1 Exon-3 | CCTAGTGGGAGAAGACTGATT; TCACATTCTAGCAAGTTGGCT | Table 3 |
| Primer pair to amplify TGFBR1 Exon-4 | CCCCAGTGAGATAAATTCC; TGCTATCAAGAGTCAAGAAAA | Table 3 |
| Primer pair to amplify TGFBR1 Exon-5 | GGGGCTTACTCTGAGGAACTA; GGTAGAGATGCGGTTTTGTCA | Table 3 |
| Primer pair to amplify TGFBR1 Exon-6 | GTGGGCTGAAATGCTTTGATA; CTTCTTACCTGTTGGCAATCT | Table 3 |
| Primer pair to amplify TGFBR1 Exon-7 | GGCAGTAAGGGGATGATTTTC; TTTGTTTTCTCTGGCACTCGG | Table 3 |
| Primer pair to amplify TGFBR1 Exon-8 | TATCAACTCAGGGAAGTGGCT; GCCCTTTCAATGTGCTTACAA | Table 3 |
| Primer pair to amplify TGFBR1 Exon-9 | CACCAGTACCCTATTGATGG; CTAACCAGGAGTAAATCACTT | Table 3 |
| Primer pair for NLRP 1 exon 1 | GACAGCACTGTTCTCTGCCT; CAGGGAGGGCTCAGTGGT | Table 2 |
| Primer pair for NLRP1 Exon 2-3 | TGAGAGAGGAGACCAGCTCAG; GGGGGAATGAAAAGAAAAAT | Table 2 |
| Primer pair for NLRP1 Exon 3 | GCCCCTCTACTTCAACATGG; AAACAGTGCCTTGGACCAGT | Table 2 |
| Primer pair for NLRP1 Exon 4 Fragment 1 | TCCCTCCCTTACTTAGAAATCAGA; AGCCCCTGCAGTATGACTA | Table 2 |
| Primer pair for NLRP1 Exon 4 Fragment 2 | CAAAGACCTCACCCCAGAAG; TGTGGTGGTCTTGGAAGTCA | Table 2 |
| Primer pair for NLRP1 Exon 4 Fragment 3 | GCCTTTAGGTTGGTCAAATCA; GTGGCGGCTGAATTTAATG | Table 2 |
| Primer pair for NLRP1 Exon 4 Fragment 4 | CTGGAGTCCCTCCACTGCT; TAGGAGGAGAGGTGGCAGAC | Table 2 |

(continued)

| Primer pair | Sequence | Related Figure/ Table |
|---|---|---|
| Primer pair for NLRP1 Exon 5 | GGACAAAGCCCAACTGTGTT; CAGCTTCTGCCTTGGATCTC | Table 2 |
| Primer pair for NLRP1 Exon 6 | CCCCAAGTACCATGTGCATT; GAGCTTCCTCCTGAGCCTCT | Table 2 |
| Primer pair for NLRP1 Exon 7 | GTAGGTGCTGGCATCCTGAG; CCAGAGAATTCAGCGACTTG | Table 2 |
| Primer pair for NLRP1 Exon 8 Fragment 1 | ATTGGAGACTGCCTCACAGC; GCTGTGGTGTCTGGGTTTCT | Table 2 |
| Primer pair for NLRP1 Exon 8 Fragment 2 | TGTACCTCCTCCTCAGAGC; TTCTCATGCCTCAGCCTCTT | Table 2 |
| Primer pair for NLRP1 Exon 9 | AGAGATGAGGTGGGGAGGTC; TAACGGAAATCCAGCCAGTT | Table 2 |
| Primer pair for NLRP1 Exon 10 Fragment 1 | GCAGCCTAAACCTTCTGAGC; AGGGAGTGTGTCTGCAGCTT | Table 2 |
| Primer pair for NLRP1 Exon 10 Fragment 2 | GCTCACTGCAGCCTAAACCT; AAGCTGCAGACACACTCCCT | Table 2 |
| Primer pair for NLRP1 Exon 10 Fragment 3 | CGCCTCCTGAGTAGAACTGG; GTGGGCAGGTTGAGAGAGAA | Table 2 |
| Primer pair for NLRP1 Exon 10 Fragment 4 | CCGCCTCCTGAGTAGAACTG; TGGGCAGGTTGAGAGAGAAC | Table 2 |
| Primer pair for NLRP1 Exon 11 | GAGCAGAAGCTGCAGACACA; TGAAAAGCAGACACCCAGTG | Table 2 |
| Primer pair for NLRP1 Exon 12 | AGGGATCCACAGTCAAGAGC; GAGGCTGGGAGATGTGTTTC | Table 2 |
| Primer pair for NLRP1 Exon 13 | GAGCCGAGATTGTACCATTG; GACGAATTGTTTGCCCACTT | Table 2 |
| Primer pair for NLRP1 Exon 14 | CGGGCTGTCTCTGTAGCAGT; TCCCTCCTCCGAACTTTCTT | Table 2 |
| Primer pair for NLRP1 Exon 15 | CCCTGACTTACCCGTGAGGT; CTCCTGACCTTGTGATCTGC | Table 2 |
| Primer pair for NLRP1 Exon 16 | GCATAAGGAAGGGATTCACC; GATAGGACAAGGGGGTATGG | Table 2 |
| Primer pair for NLRP1 Exon 17 | GACACAGGTGCATTTCTGGA; TTAGTGCTGGAAGGCAAACC | Table 2 |

[00171] Table 5 – Key resources table

| REAGENT or RESOURCE | SOURCE | IDENTIFIER |
|---|---|---|
| **Antibodies** | | |
| Rabbit polyclonal anti-GFP antibody | Abcam | RRID: AB_303395, Cat#: ab290 |
| Rabbit polyclonal anti-HA tag antibody | Santa Cruz Biotechnology | RRID: AB_631618, Cat#: sc-805 |
| Mouse monoclonal anti-V5 tag antibody | Abcam | RRID: AB_471093, Cat# ab27671 |
| Rabbit polyclonal anti-NLRP1 antibody | Adipogen | RRID: AB_2490439, Cat#: AL176 |
| Sheep polyclonal anti-NLRP1 antibody | R&D Systems | RRID: AB_10891878, Cat#: AF6788 |
| Rabbit polyclonal anti-ASC antibody | Adipogen | RRID: AB_2490440, Cat #: AG-25B-0006 |
| Rabbit polyclonal anti-Ki67 | Abcam | RRID: AB_443209, Cat#: ab15580 |
| Mouse monoclonal anti-Keratin 10 | Leica | Clone DE-K10 |
| Mouse monoclonal anti-GAPDH antibody | Santa Cruz Biotechnology | RRID: AB_627679, Cat #: sc-32233 |
| Mouse monoclonal anti-Involucrin antibody | Abcam, ab68 | RRID: AB_305656, |
| Mouse anti-S100A7 antibody | Dako | Cat #: M074701, Clone MAC 387 |
| Rabbit polyclonal anti-S100A9/8 antibody | Novus Biologicals | RRID: AB_2184110, Cat #IMG-6007A |
| **Chemicals, Peptides, and Recombinant Proteins** | | |
| Caspase-1/ICE Inhibitor Z-YVHD-FMK | R&D systems | FMK002 |
| Doxycycline | Sigma-Aldrich | Cat# D9891, CAS # 24390-14-5 |
| Nigericin | Sigma-Aldrich | Cat# N7143, CAS # 28643-80-3 |
| Recombinant human IL-1β | R&D Systems | 201-LB-025 |
| Recombinant human IL-1α | R&D Systems | 200-LA-002 |
| Recombinant human IL-18 | R&D Systems | 9124-IL-010 |
| **Critical Commercial Assays** | | |
| Human IL-1β ELISA Quantikine ELISA kit | R&D Systems | DLB50 |
| Human IL-1α ELISA Duoset ELISA kit | R&D Systems | DY201 |
| Human IL-18 ELISA kit | MBL International | 7620 |
| RNAScope 2.5 Brown Kit | Advanced Cell Diagnostics | 322370 |
| Novex NativePAGE Bis-Tris gel system | ThermoFisher | BN2008, BN2007, BN1004BOX |
| FirstChoice Human Total RNA Survey Panel | Applied Biosystems/Ambion | AM6000 |
| TruSeq Exome Enrichment Kit (used for whole exome sequencing for MSPC-TN-1) | Illumina | FC-150-1091 |
| Illumina HiSeq 2500 (used for whole exome sequencing for MSPC-TN-1) | Illumina | HiSeq 2500 |
| Sciipt cDNA synthesis kit | Biorad | 1708891 |
| Dako EnVision rabbit-HRP Kit | Dako | K401111 |
| Lenti-X concentrator | Clontech | 631232 |
| FugeneHD | Promega | E2312 |

| | | |
|---|---|---|
| FugeneHD | Promega | E2312 |
| Lipofectamine RNAiMax | ThermoFisher | 13778030 |
| QuickChange II Site-directed Mutagenesis | Agilent | 200522 |
| InFusion HD Cloning Kit | Takara/Clontech | 638910 |
| Human cytokine/chemokine panel I | HCYTMAG-60K-PX41 | Merck Millipore |
| Illumina TruSeq stranded mRNA kit | Illumina | RS-122-2101 |
| Oragene DNA collection kit | DNAGenotek | OG500 |
| ExoSAP-IT | Affymetrix | 78201 |
| BigDye Terminator v3.1 Cycle Sequencing Kit | Applied Biosystems | 4337454 |

| REAGENT or RESOURCE | SOURCE | IDENTIFIER |
|---|---|---|
| Amicon Ultra 0.5ml filter (MWCO = 3GKda) | Merck Millpore | UFC503024 |
| **Deposited Data** | | |
| PolyA RNA-seq | This paper | GEO: GSE85791 |
| **Experimental Models: Cell Lines** | | |
| HEK293T | Lab stock | RRID: CVCL_0063 |
| Primary neonatal human keratinocytes | CELLnTEC | Cat# HPEKp |
| N/TERT-1 Immortalized human keratinocytes | Rheinwald lab | N/A |
| 3T3 mouse fibroblasts | Lab stock | N/A |
| THP-1 | ATCC | RRID: CVCL_0006 |
| THP1-Cas9 | Baker et al., 2015; S. Masters' lab | N/A |
| THP1-Cas9 ASC-/- | Baker et al., 2015; S. Masters' lab | N/A |
| **Recombinant DNA** | | |
| pCI-Caspase 1 | Addgene | #41552 |
| pCDH-ASC-GFP | This paper | N/A |
| pCS2-NLRP1-HA wild type, missense, and deletion mutants | This paper | N/A |
| pTRIPZ-NLRP1-HA wild type, A55T, M77T and A66V | This paper | N/A |
| pCS2-NLRP1-PYD-mCherry wild type, A55T, M77T and A66V | This paper | N/A |
| pCS2-NLRP3 | This paper | N/A |
| pCS2-NLRP3-PYD-mCherry | This paper | N/A |
| pCS2-AIM2-PYD-mCherry | This paper | N/A |
| pET20-GB1-NLRP1-PYD | Hiller et al., 2003 | N/A |
| pET20-GB1-NLRP1-PYD(A54T) | This paper | N/A |
| pET20-GB1-NLRP1-PYD(S55L) | This paper | N/A |
| pET20-GB1-NLRP1-PYD(E63K) | This paper | N/A |
| pET20-GB1-NLRP1-PYD(Q64H) | This paper | N/A |
| pET20-GB1-NLRP1-PYD(A66V) | This paper | N/A |
| pET20-GB1-NLRP1-PYD(M77T) | This paper | N/A |
| pET20-NLRP1-CARD | This paper | N/A |
| **Sequence-Based Reagents** | | |
| Human PYCARD siGENOME siRNA | ThermoFisher | M-004378-01-0005 |
| Primers used in this study, see Table S4 | This paper | N/A |
| **Software and Algorithms** | | |
| Picard (v1.42) | https://broadinstitute.github.io/picard/ | N/A |
| Novoalign (v2.07) | http://www.novocraft.com/products/novoalign/ | N/A |
| GATK (Genome Analysis Toolki) (v1.1) | https://software.broadinstitute.org/gatk/ | N/A |
| OutLink | http://cole-tapnell-lab.github.io/cufflinks/ | N/A |
| RVIS | http://genic-intolerance.org/ | N/A |
| BEDTools | https://launchpad.net/ubuntu/+source/bedtools | N/A |
| ANNOVAR | http://annovar.openbioinformatics.org/en/latest/ | N/A |
| SAMtools | http://samtools.sourceforge.net/ | N/A |
| iTOL: Interactive Tree of Life | http://itol.embl.de | N/A |
| Muscle | http://www.ebi.ac.uk/Tools/msa/muscle/ | N/A |
| ClustalW2 phylogeny | http://www.ebi.ac.uk/Tools/phylogeny/clustalw2_phylogeny/ | N/A |
| Multiple Experiment Viewer | http://www.tm4.org | N/A |

**Claims**

1. A method of determining the likelihood or predisposition of a subject in developing MSPC (multiple self-healing palmoplantar carcinoma) or FKLC (familial keratosis lichenoides chronica), comprising detecting NLR family, pyrin domain containing protein 1 (NLRP1) mutation in a sample obtained from the subject, wherein the NLRP1 mutation is located at PYD (pyrin domain) and/or LRR (leucine-rich repeats) domain.

2. A method of determining whether a skin inflammation in a subject is MSPC (multiple self-healing palmoplantar carcinoma) or FKLC (familial keratosis lichenoides chronica), comprising detecting NLRP1 mutation in a sample obtained from the subject, wherein the NLRP1 mutation is located at PYD (pyrin domain) and/or LRR (leucine-rich repeats) domain.

3. The method of claim 1 or 2, wherein NLRP1 mutation at the PYD domain results in at least one or two or all amino acid substitution selected from the group consisting of A54T, M77T, and A66V.

4. The method of claim 1 or 2, wherein NLRP1 mutation at the LRR domain results in the deletion of amino acid phenylalanine at position 787 to arginine at position 843 .

5. The method of any one of claims 1 to 4, wherein NLRP1 mutation is detected by an increased accumulation of oligomerized NLRP1 or increase of ASC (Apoptosisassociated speck-like protein, also known as PYCARD) oligomers formation in the sample obtained from the subject as compared to wild type (non-diseased or non-mutant) NLRP1.

6. The method of any one of claims 1 to 5, wherein the sample of the subject is selected from the group consisting of bodily fluid, and skin.

7. The method of any one of claims 1 to 5, wherein the sample of the subject is skin selected from the group consisting of epidermis, glabrous skin, plantar skin, epidermal appendages, keratinocytes and fibroblast.

**Patentansprüche**

1. Verfahren zur Bestimmung der Wahrscheinlichkeit oder Veranlagung eines Probanden, MSPC (multiples selbstheilendes palmoplantares Karzinom) oder FKLC (familiäre Keratosis lichenoides chronica) zu entwickeln, umfassend ein Erkennen einer NLR-Family-Pyrin-Domain-Containing-Protein-1-Mutation (NLRP1-Mutation) in einer Probe, die von dem Probanden erhalten wird, wobei die NLRP1-Mutation sich an der PYD (Pyrin-Domäne) und/oder der LRR-Domäne (Lycin-Rich-Repeats-Domäne) befindet.

2. Verfahren zur Bestimmung, ob eine Hautentzündung in einem Probanden MSPC (multiples selbstheilendes palmoplantares Karzinom) oder FKLC (familiäre Keratosis lichenoides chronica) ist, umfassend ein Erkennen einer NLRP1-Mutation in einer Probe, die von dem Probanden erhalten wird, wobei die NLRP1-Mutation sich an der PYD (Pyrin-Domäne) und/oder der LRR-Domäne (Lycin-Rich-Repeats-Domäne) befindet.

3. Verfahren nach Anspruch 1 oder 2, wobei die NLRP1-Mutation an der PYD-Domäne zu mindestens einer oder zwei oder allen Aminosäuresubstitutionen führt, die aus der Gruppe bestehend aus A54T, M77T und A66V ausgewählt sind.

4. Verfahren nach Anspruch 1 oder 2, wobei die NLRP1-Mutation an der LRR-Domäne zu der Deletion der Aminosäure Phenylalanin an Position 787 zu Arginin an Position 843 führt.

5. Verfahren nach einem der Ansprüche 1 bis 4, wobei die NLRP1-Mutation durch eine erhöhte Akkumulation von oligomerisiertem NLRP1 oder eine Erhöhung einer Bildung von Oligomeren von ASC (Apoptose-assoziiertes Speck-ähnliches Protein, auch als PYCARD bekannt) in der Probe, die von dem Probanden erhalten wird, im Vergleich zum Wildtyp-NLRP1 (nicht erkrankt oder nicht mutiert) erkannt wird.

6. Verfahren nach einem der Ansprüche 1 bis 5, wobei die Probe des Probanden aus der Gruppe bestehend aus Körperflüssigkeit und Haut ausgewählt ist.

7. Verfahren nach einem der Ansprüche 1 bis 5, wobei die Probe des Probanden Haut ist, die aus der Gruppe bestehend aus Epidermis, unbehaarter Haut, Plantarhaut, epidermalen Anhängseln, Keratinozyten und einem Fibroblast ausgewählt ist.

**Revendications**

1. Méthode pour déterminer la susceptibilité ou la prédisposition d'un sujet à développer un MSPC (carcinome palmoplantaire auto-involutif multiple) ou une FKLC (kératose lichénoïde chronique familiale), comprenant la détection d'une mutation de la protéine 1 contenant un domaine de pyrine de la famille LNR (NLRP1) dans un échantillon obtenu auprès du sujet, dans laquelle la mutation de NLRP1 est située au niveau des domaines PYD (domaine de pyrine) et/ou LRR (répétitions riches en leucine).

2. Méthode pour déterminer si une inflammation cutanée chez un sujet est un MSPC (carcinome palmoplantaire auto-involutif multiple) ou une FKLC (kératose lichénoïde chronique familiale), comprenant la détection d'une mutation de NLRP1 dans un échantillon obtenu auprès du sujet, dans laquelle la mutation de LNRP1 est située au niveau des domaines PYD (domaine de pyrine) et/ou LRR (répétitions riches en leucine).

3. Méthode selon la revendication 1 ou 2, dans laquelle la mutation de NLRP1 au niveau du domaine PYD a pour résultat la substitution d'au moins un ou de deux ou de la totalité des acides aminés choisis dans le groupe constitué par A54T, M77T et A66V.

4. Méthode selon la revendication 1 ou 2, dans laquelle la mutation de NLRP1 au niveau du domaine LRR a pour résultat une délétion de l'acide aminé phénylalanine à la position 787 jusqu'à l'arginine à la position 843.

5. Méthode selon l'une quelconque des revendications 1 à 4, dans laquelle la mutation de NLRP1 est détectée par une accumulation accrue de NLRP1 oligomérisée ou une augmentation de la formation d'oligomères d'ASC (protéine de type speck associée à l'apoptose, également appelée PYCARD) dans l'échantillon obtenu auprès du sujet, en comparaison avec la NLRP1 de type sauvage (non malade ou non mutante).

6. Méthode selon l'une quelconque des revendications 1 à 5, dans laquelle l'échantillon du sujet est choisi dans le groupe constitué par un fluide corporel et la peau.

7. Méthode selon l'une quelconque des revendications 1 à 5, dans laquelle l'échantillon du sujet est une peau choisie dans le groupe constitué par un épiderme, une peau glabre, une peau plantaire, des appendices épidermiques, des kératinocytes, et un fibroblaste.

**FIG. 1**

# FIG. 2

FIG. 2 continued

## FIG. 3

**FIG.3 continued**

**C**

**D** Primary human cells

**FIG.3 continued**

**FIG. 4**

**A**

**B**

**FIG. 4 continued**

FIG. 4 continued

## FIG. 4 continued

**FIG. 5**

A

NLRP10
MEFV      NLRP6
NLRP3          ASC
NLRP12              PYDC1
NLRP1
                        NLRP8
NLRP9
NLRP4                   NLRP13
NLRP14                  AIM2
NLRP11                  MNDA
NLRP5
PYDC2        NLRP7   IFI16
    NLRP2

B

```
NLRP1  MEVA  YLVAQYGEQRAWDLALHTWEQMG
NLRP3  VDLA  LMIDFNGE KAWAMAVWIFAA N
AIM2   IQVA  LMIQNAGAVSAVMKTIRIFQK N
MEFV   VKMA  LLVTYYGE YAVQLTLQVLRA N
       :.:   ::    *        ::
```

☐ MSPC mutants
■ SNP variants (1000 Genome)

C

| | DAPI | ASC-GFP | mCherry |
|---|---|---|---|
| NLRP1 PYD-WT mCherry | | | |
| NLRP3 PYD mCherry | | | |
| AIM2 PYD mCherry | | | |

20 µm

FIG. 5 continued

## FIG. 5 continued

**G**

**H**

## FIG. 5 continued

## FIG. 6

## FIG. 6 continued

**FIG. 7**

**A**

**B**

# FIG. 7 continued

**C** Unsupervised hierarchical clustering

DOX

Top enriched GSEA gene sets
1. SESTO_RESPONSE_TO_UV
2. RASHI_RESPONSE_TO_IONIZING_RADIATION
3. PID_NFKAPPAB_CANONICAL_PATHWAY
4. DUTTA_APOPTOSIS_VIA_NFKB
5. REACTOME_IL1_SIGNALING
6. BIOCARTA_RELA_PATHWAY

Top enriched GO categories
1. WOUND_HEALING
2. INFLAMMATORY_RESPONSE

**D**

**FIG. 7 continued**

**FIG. 8**

**A**

**Luminex: patient-derived primary keratinocytes**

**B**

**ELISA: patient-derived primary keratinocytes**

**FIG. 8 continued**

C **Patient-derived primary keratinocytes**

D **Patient sera**

**FIG. 8 continued**

E

control palmar skin
IHC: S100A8/9

MSPC-TN-1: IV20 A54T/+ (SCC).
IHC: S100A8/9

50 µm

F

## FIG. 9

FIG. 9 continued

FIG. 10

## FIG. 10 continued

**D** Human tissue cDNA array

**E** Primary keratinocytes (ENCODE)

**F**

**G** Palmar skin:

RNAscope: dapB (negative control)

RNAscope NLRP1(brown)

50 µm

## FIG. 10 continued

**H**

Abdominal skin:
RNAscope:
dapB (negative control)

RNAscope
NLRP1(brown)

50 μm

## FIG. 11

**A**

Stably transduced THP1

┌ Pyroptotic cell death

└ IL-1β release

PMA
differentiation

+/-
DOX

LPS
stimulation

**B**

DOX: vector, WT, A54T, M77T, A66V, NLRP3 WT, NLRP3 R262W (- - - - - - -) and vector, WT, A54T, M77T, A66V, NLRP3 WT, NLRP3 R262W (+ + + + + + +)

HA

150

25

1  2  3  4  5  6  7    8  9  10  11  12  13  14

## FIG. 11 continued

## FIG. 12

## FIG. 12 continued

FIG. 12 continued

## FIG. 12 continued

**FIG. 12 continued**

**FIG. 13**

## FIG. 13 continued

**B**

293T NLRP1 WT    293T NLRP1 M77T

1D: Blue Native PAGE 3-12%

Expected NLRP1 monomer size: 150 kDa    NLRP1 oligomers
Expected GAPDH tetramer size: 148 kDa

**C**

ASC pH=3.7
ASC pH=7.0
ASC+ NLRP1-PYD #1 pH=7.0
ASC+ NLRP1-PYD #2 pH=7.0
ASC+ NLRP1-PYD #3 pH=7.0

FIG. 13 continued

**FIG. 14**

A     Immortalized keratinocytes

B

## FIG. 14 continued

C

D

E  RNAseq validation differentiation markers

F  RNAseq validation inflammatory markers

FIG. 14 continued

G

H

**FIG. 14 continued**

50 µm

# FIG. 15

**A**     Patient sera

FIG. 15 continued

**B**

control palmar skin
IHC: S100A7/Psoriasin

MSPC-TN-1: IV20 A54T/+ (SCC).
IHC: S100A7/Psoriasin

50 µm

FIG. 16

**A** <u>293T-ASC-GFP</u>

Transfect:
1. Vector (Control)
2. <u>NLRP1-HA</u>
3. NLRP1-a.a.1213-1474

Talabostat treatment

Visualise ASC speck formation

## FIG. 16 continued

**B**

293T-ASC-GFP

**FIG. 17**

**A**    \*Sitagliptin:
- Specific DPP4 inhibitor
- Not DPP8 or 9

| | IC50/uM | |
|---|---|---|
| | Talabostat | Sitagliptin |
| DPP4 | 0.002 | 0.018 |
| DPP8 | 0.0015 | 48 |
| DPP9 | 0.00076 | 100 |

FIG. 17 continued

**293T-ASC-GFP**

B

NLRP1

|  | vector | WT | M77T |
|---|---|---|---|

| | | | | | | | | | |
|---|---|---|---|---|---|---|---|---|---|
| Mock | + | - | - | + | - | - | + | - | - |
| Talabostat | - | + | - | - | + | - | - | + | - |
| *Sitaglipitin | - | - | + | - | - | + | - | - | + |

HA

GFP

GAPDH

1 mM
DSS
Crosslinked

GFP

kDa
−150

−25

−50

−37

−250
−150
−100
−75

−50

1 2 3 4 5 6 7 8 9

FIG. 18

A

293T overexpressing
• NLRP1-WT-HA
or
• GoF NLRP1 mutants

⬇

Talabostat treatment

⬇

Blue-Native PAGE to visualize oligomerization

FIG. 18 continued

FIG. 19

## FIG. 19 continued

**B**

**C**

## 10x concentrated media

Longer exposure:

**FIG. 20**

A

Immortalized keratinocytes

NLRP1 a.a.1213-1474

[Talabostat]/µM  -  0.1  0.3  -

Cell lysate — ASC

n.s. control

DSS crosslinked — ASC

B

Untreated | Talabostat (2 µM) | NLRP1 a.a. 1213-1474 transfected

"Ballooning" is characteristic of pyroptosis

## FIG. 21

| | DAPI | NLRP1 | ASC | Merged |
|---|---|---|---|---|

Control

2uM
Talabostat
(24 hrs)

## FIG. 22

Day 0:     Day 1:     Day 2:     Day 3:     Day 4:     Day 5:

Seed
N/TERT
keratinocytes

Treat with siRNA:
- No siRNA (control)
- siNLRP3 (control)
- siNLRP1
- siASC
- siCASP1
- siGSDMD

- siELF3 (additional control)

Treat with
Talabostat

Harvest cells,
Collect media

**FIG. 23**

N/TERT-immortalized keratinocytes

**FIG. 24**

N/TERT-immortalized keratinocytes

**EP 3 515 470 B1**

**Patent documents cited in the description**

- WO 2007106790 A2

- WO 2015084875 A1